# EUROPEAN PATENT APPLICATION

(11) **EP 0 059 226 A1**
(43) Date of publication of application: **08.09.1982**
(21) Application number: 81100525.5
(22) Date of filing: 24.01.1981
(51) Int. Cl.: C07C 111/00, C07C 121/78, C07C 143/68, C07C 147/12, C07C 149/42, A01N 51/00

(54) **Substituted N-nitroaniline compounds and inorganic and organic salts thereof useful for improving crop yields**

(71) Applicant: AMERICAN CYANAMID COMPANY, Stamford Connecticut 06904-0060 (US)
(72) Inventor: O'Neal, Thomas Denny, Princeton New Jersey 08540 (US); Bhalla, Prithvi Raj, Hightstown New Jersey 08520 (US); Cross, Barrington, Rocky Hill New Jersey 08553 (US)
(74) Representative: Wächtershäuser, Günter, Prof. Dr.

(57) **Abstract**

Di- tri- and tetrasubstituted N-nitroaniline compounds and inorganic and organic salts thereof which are effective for Increasing axillary branching, improving canopy and enhancing flowering of herbaceous omamental plants and graminaceous crops, leguminous crops, cotton and sunflowers. The salts are also effective as yield enhancing agents and lodging inhibitors for the crops.

## Description

The invention is the use of plant regulating substituted N-nitroanilines represented by formulae (I) and (Ia) below: 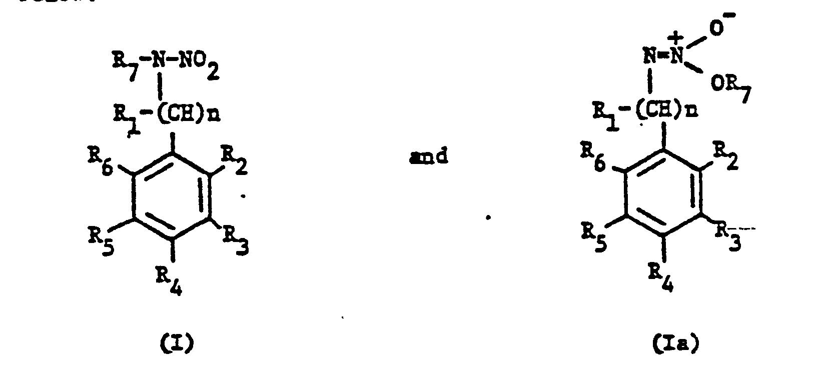 wherein n is an integer of 0 or 1; R₁ is hydrogen, C₁-C₃ alkyl, Cₗ-C₃ haloalkyl, CN, halogen, NO₂, SO₂R₈, COR₉, phenyl or substitued phenyl and the substituents are CH₃, CF₃, CH₃O, halogen, N0₂ or OCH₂-CO₂C₂H₅; R₂ and R₆ are hydrogen, halogen, C₁-C₄ alkyl, C₁-C₄ haloalkyl, C₁-C₄ haloalkoxy, SO₂R₈, SR₈ or COR₁₀; R3 is hydrogen, C₁-C₄ alkyl, C₁-C₄ alkoxy, NH₂, NH-COCH₃, halogen, NO₂, SO₂R₈, CN or CO₂CH₃; R₄ is hydrogen, halogen, C₁-C₂ alkyl, SO₂R₈, SR₈, OCF₃, COR₉, CN, NH-COCH₃, SO₂NR₁₀R₁₁, 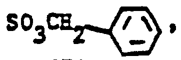 SO₃CH₂, phenoxy or substituted phenoxy and the substituents are CH₃, CF₃, CH₃O, halogen, NO₂ or OCH₂CO₂C₂H₅; R₅ is hydrogen, C₁-C₃ alkyl or halogen; R₇ is hydrogen, C₁-C₁₂ alkyl optionally substituted with halogen or CN, C₃-C₄ alkenyl, C₃-C₄ alkynyl, the methyl esters of lover alkanoic acids or the moiety 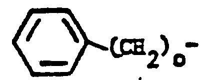 wherein o is an integer of 0, 1,2 or 3, and the moiety may optionally be substituted with halogen, C₁-C₂ alkyl, C₁-C₂ alkox^{y}, (Cₗ-C₃ alkyl)3 SiCH₂ - or N02; R₈ is C₂-C₆ alkyl, phenyl or benzl; R₉ is hydrogen, hydroxyl, C₁-C₃ alkyl, C₁-C_{3 alkox}y or phenyl; R₁₀ and R₁₁ each are hydrogen, C₁-C₄ alkyl or phenyl; with the proviso that not less than two and not more than four of R₂ to R₆ are to be substituted with groups other than hydrogen in any one of the compounds represented by formulae (I) and (Ia) and mixtures thereof; and with the proviso that when R₃, R₅ and R₇ are all hydrogen and n is an integer of 0 and the compounds are of formula I then R₂ cannot be halogen, Cₗ-C₄ alkyl, SO₂ CH₃, C₁-C₃ haloalkyl or C₁-C₃ haloalkoxy; R₄ cannot be halogen or methyl; and R₆ cannot be halogen, CF₃ or C₁-C₄ alkyl; and when R₇ is hydrogen, the above compounds of formulae (I) and (Ia) may form inorganic or organic salts, the salt being represented by formula (II) below: 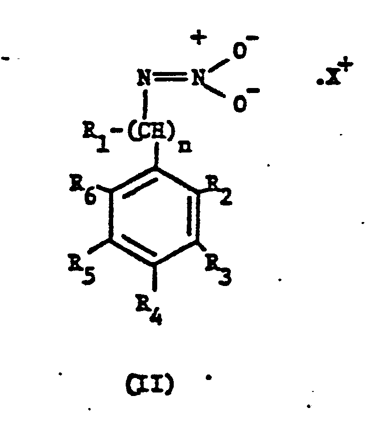 when X is Cu, n is an organic radical represented by formula IIIa 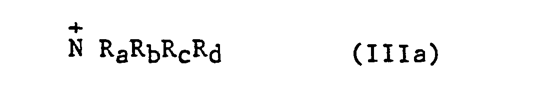 wherein Rₐ is hydrogen, C₁-C₃₀ alkyl straight chain or branched [optionally mono - or disubstituted with HO-, CH₃O₂C-, CH₃S-, C₃-C₁₈ alkenylaminoalkylene (C₁-C₃), H₂N-, (CH₃)₂N-, 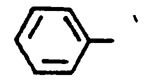 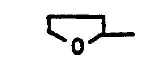 J, C₃-C₆ alkenyl, C₃-C₆ alkynyl, C₃-C₆ cycloalkyl, 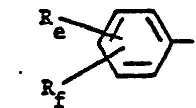 wherein Rₑ and R_{f} each may be H, CH₃ or n-C₁₆H₃₃SO₂-; R_{b} is hydrogen, C₁-C₃₀ alkyl straight chain or branched [optionally mono- or disubstituted with HO- or 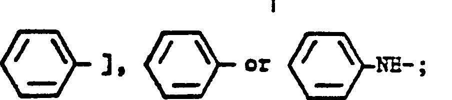 R_{c} is hydrogen, C₁-C₃₀ alkyl straight chain or branched [optionally mono- or disubstituted with HO- or 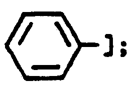 R_{d} is hydrogen, C₁-C₃₀ alkyl straight chain or branched [optionally substituted with HO-, 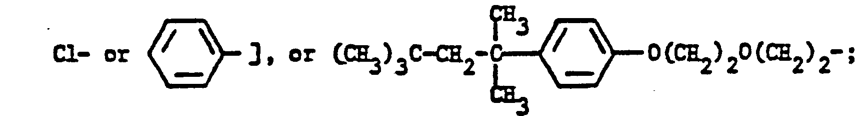 and when Rₐ and R_{b} are taken together with the nitrogen they are attached to they repreaent a moiety of 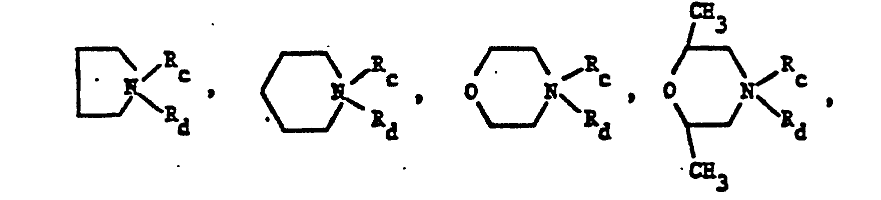 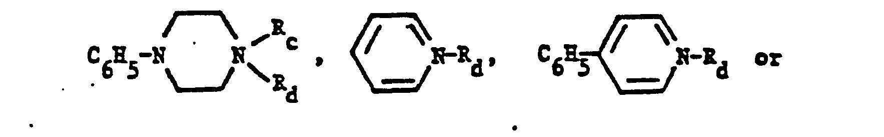 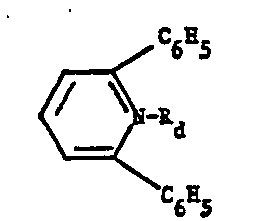 wherein R_{c} and B_{d} are defined as above; organic cation X⁺ may also be imidazolium, pyridinium, quinolinium, dithiolium, tetrazolium or be represented by formula IIIb 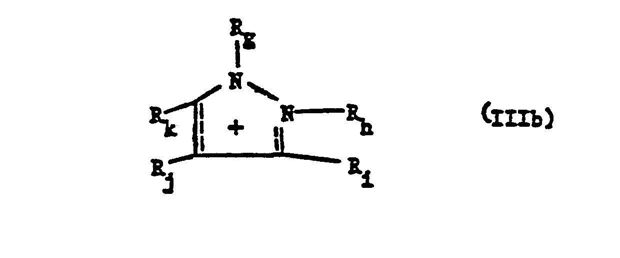 wherein R_{g} and Rₕ each are C₁-C₃ alkyl; Rⱼ is hydrogen, halogen, C₁-C₃ alkyl or C₁-C₃ alkozy; Rᵢ and Rₖ each are hydrogen C₁-C₆ alkyl straight chain or branched, C₃-C₆ cycloalkyl, 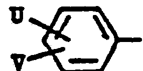 or 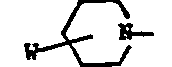 ; wherein U and V each are hydrogen, halogen, C₁-C₃ alkyl, C₁-C₃ alkoxy, CN or CF₃; W is hydrogen or C₁-C₃ alkyl; and each - symbol represents a single or double bond, wherein if both are single bonds then the cations are pyrazolidinium cations represented by formula (IIIc) 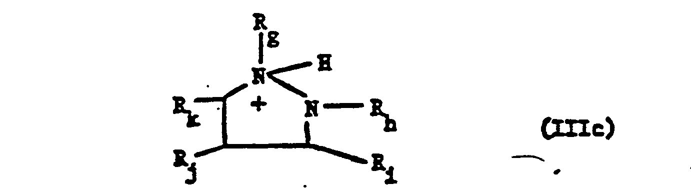 wherein R_{g} to Rₖ are as defined above; and when one of the = symbols represents a double bond then the cations are pyrazolinium cations represented by formula (IIId) 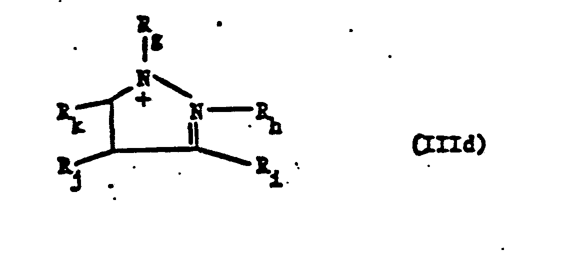 wherein R_{g} to Rₖ are as defined above; and when both = symbols represent double bonds, then the cations are pyrazolium cations represented by formula (IIIe) 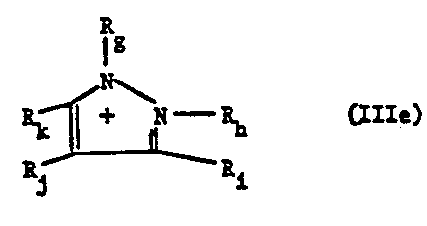 wherein R_{g} to Rₖ are as defined above. X may also be represented by Formulae IIIf and IIIg 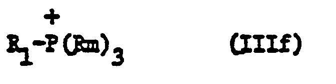 wherein R₁ is C₁-C₆ alkyl, 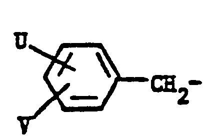 or 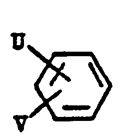 wherein U and V are as hereinabove defined; Rₘ is C₁-C₆ alkyl or 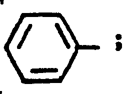 SRₐR_{b}R_{c} (IIIg); and I RₐR_{b} (IIIh); and with the proviso that when n is an integer of 0; R₃ and R₄ are both hydrogen; R₂ is halogen, C₁-C₄ alkyl, SO₂CH₃, C₁-C₃ haloalkyl or C₁-C₃ haloalkoxy; R₄ is halogen or methyl; R₆ is halogen, CF₃ or C₁-C₄ alkyl, and the salts are of formula II; then X⁺ cannot be alkali metals, ammonium, H₃NRₐ H₂NRₐR_{b}, HNRₐR_{b}R_{c}and NRₐ R_{b}R_{c}R_{d}, wherein the R groups: Rₐ to R_{d} each are selected from C₁-C₁₂ alkyl, C₂-C₄ hydroxyalkyl, aryl, substituted aryl wherein the substituents are selected from halogen and C₁-C₆ alkyl, aralkyl C₁-C₃, and substituted benzyl wherein the substituents are selected from halogen and C₁-C₆ alkyl, nor can X⁺ be a heterocyclic ring which may be formed when two of the Rₐ to R_{d} groups are taken together with the nitrogen to which they are attached to.

A group of compounds represented by formula IV when n is 0, of particular interest for use in the invention, are graphically illustrated and described as follows: 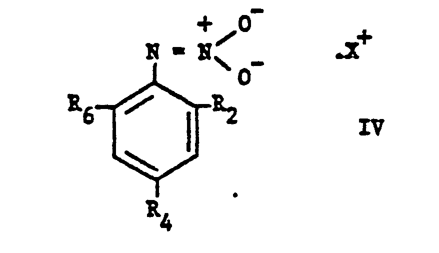 wherein R₂ is Br, Cl, F, I, C₁-C₄ alkyl, OCHF₂, CF₃, SR₈, SO₂R₈, or COR₉; R₄ is Br, Cl.F, I, CH₃, OCF₃, NHCO-CH₃, CN, SR₈, SO₂R₈, COR₉, phenoxy or substituted phenoxy wherein the substituents are selected from-the group described above; R₆ is Br, Cl, F, I or C₁-C₄ alkyl; R₈ and R₉ are as hereinabove defined; andn cation x⁺ is as defined and restricted by the above proviso.

A second group of compounds represented by formula (IV) above and of interest are those wherein R₂, R₄ and R₆ are each bromine; X⁺ is R₈NH₃, wherein Rₐ is C₁₈H₃₅, n-C₁₈H_{37'} n-C₂₀H₄₁, 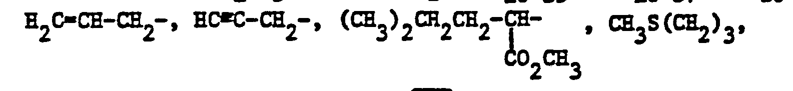 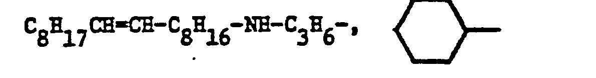 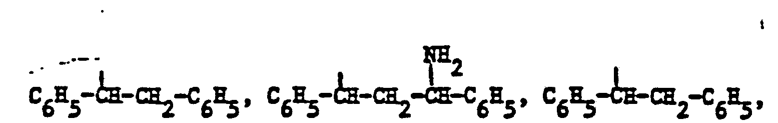 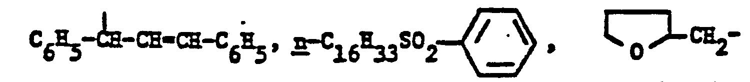 or (CH₃)₂N-.

A third group of compounds of interest and represented by formula IV above are those wherein R₂, R₄ and R₆ each are bromine ⁺ and X is n-C₁₆H₃₃ N(CH₃)₃, 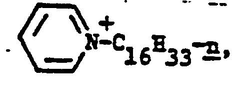 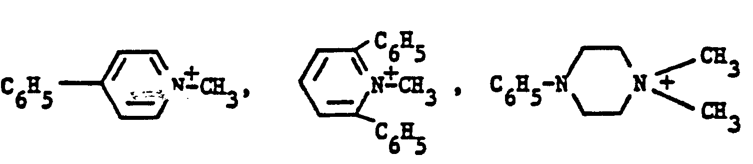 or

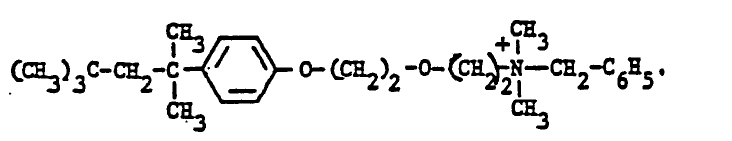

Also of interest are the compounds:

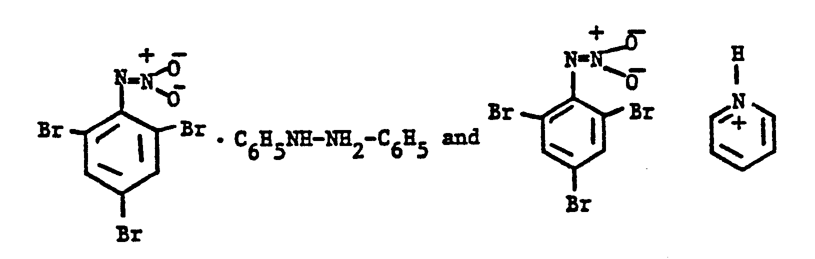

Another, important group of plant regulating compounds represented by formula II above, are those wherein the cation X⁺ is pyrazolium, pyrazolinium or pyrazolidinium moieties as represented by structural formula IV and discussed and described in detail as follows: 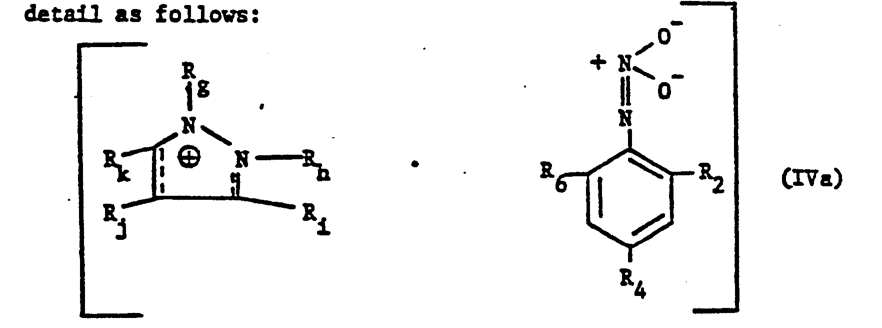 comprising a cation of formula (IIIb) 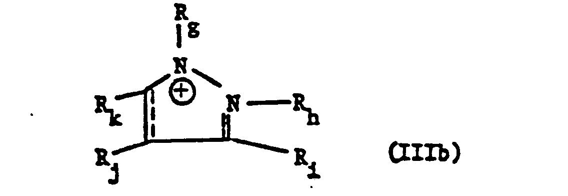 wherein R_{g} and Rₕ each are methyl or ethyl; Rⱼ is hydrogen, bromine, chlorine. fluorine, methyl or methoxy; Rⱼ and Rₖ each are hydrogen, cyclohexy 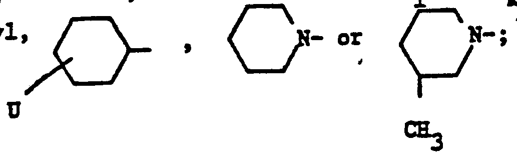 U is hydrogen, CF₃, methyl, methoxy, chlorine, bromine or fluorine; and a phenyl- nitramine anion of formula (IV) 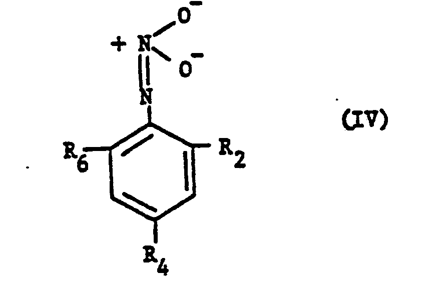 wherein R₂,R₄ and R₆ each are bromine, chlorine, iodine, methyl or trifluoromethyl; and― represents a single or double bond.

A preferred group of salts useful in the invention are represented by formula (IVa) above are those wherein each of the drogen; R₁ and Rₖ each are cyclohexyl, 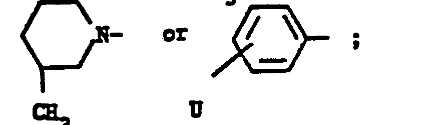 U is hydrogen, methyl, methoxy, chlorine, fluorine or trifluoromethyl.

The most preferred compounds useful in the invention are represented by formula (IVa) are those, wherein R₂ R₄, R₆ each are trifluoromethyl, bromine or iodine; Rₖ and each are phenyl.

Of special interest are formula IV salts represented by the structures shown below:

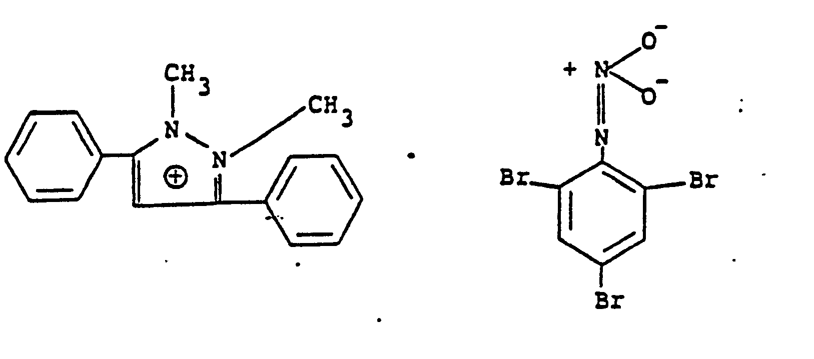

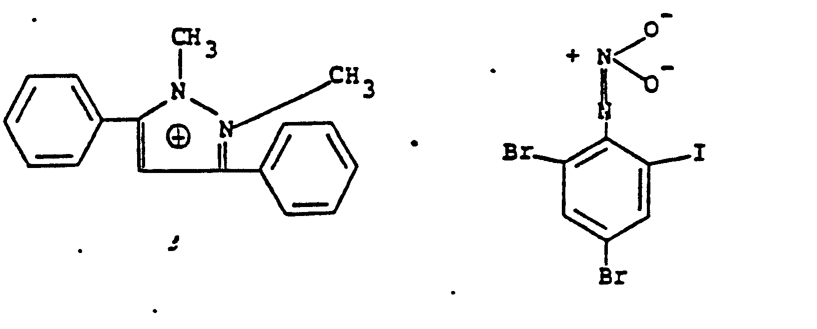

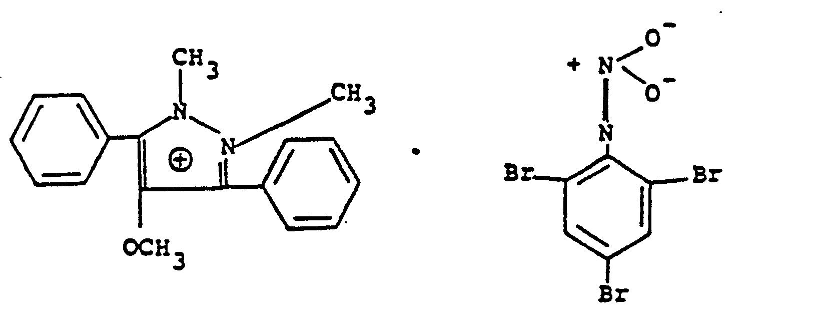

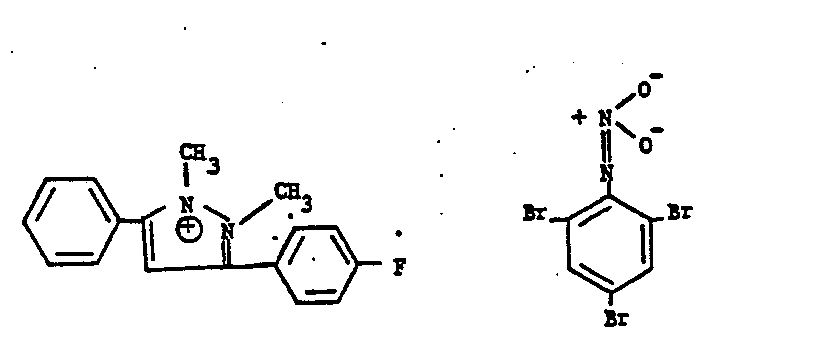

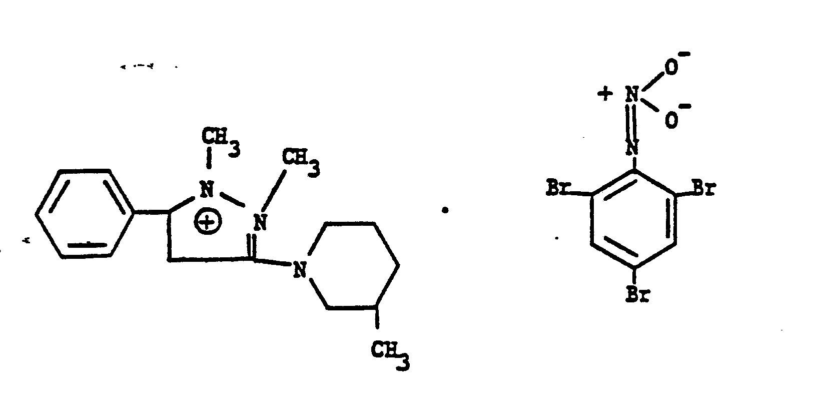

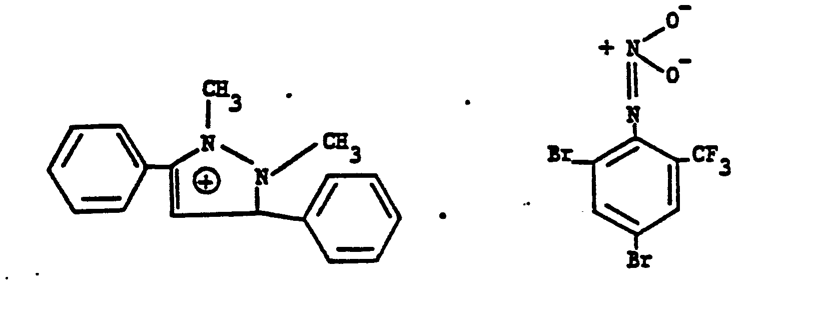

Other comgouads of formula II useful in the invention are compounds wherein the anion has the structure(V) 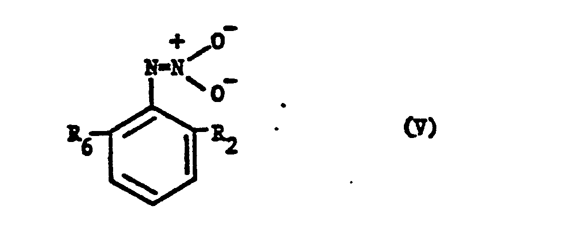 and R₂ and R₆ each are halogen, C₁-C₄ alkyl or C₁-C₃ haloalkyl; cation X⁺ is as hereinabove defined. Among the compounds described above, preferred are those wherein R₂ and R₆ each are bromine, chlorine, iodine or CF₃; and cation X⁺ is C₁₈H₃₅N H₃ or is the pyrazolium cation of formula 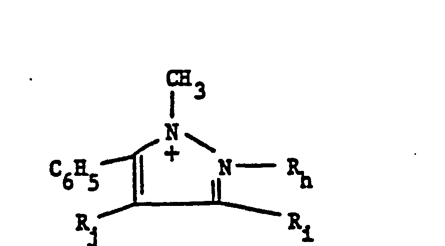 wherein Rₕ is Cₗ-C₂ alkyl; R₁ is phenyl; Rⱼ is hydrogen, halogen or methyl.

Also useful in the invention are compounds formula II salts wherein the anion is represented by structure (VI) 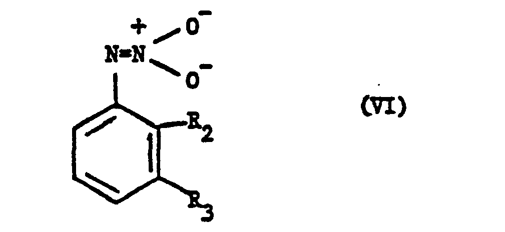 R₂ and R₃ each are halogen or C₁-C₄ alkyl; cation X⁺ is as hereinabove defined. Of particular interest are those salts wherein R₂ + + and R₃ each are bromine or chlorine; cation X⁺ is C₁₈H₃₅NH₃ or the pyrazolium cation of formula 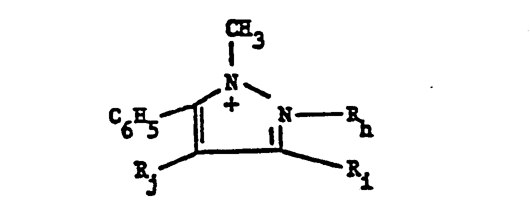 wherein Rₕ is C₁-C₂ alkyl; R₁ is phenyl; Rⱼ is hydrogen, halogen or methyl.

Among the trisubstituted salts of formula II useful in the invention the following are of interest: 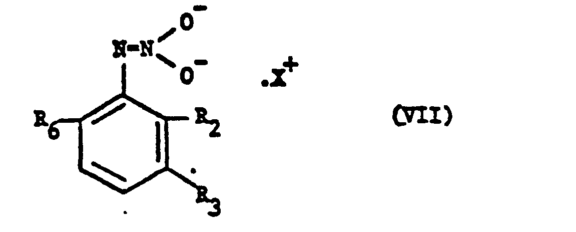 wherein R₂, R₃ and R₆ each are halogen, alkyl C₁-C₄ or haloalkyl C₁-C₄; cation X⁺ is as hereinabove defined; and especially those wherein R₂, R₃ and R₆ each are Br, Cl or I and when R₂ and R₃ are Cl the R₆ is Br, or if one is CF₃, the other two have to be + Br or I; and cation X⁺ is C₁₈H₃₅NH₃ or is the moiety 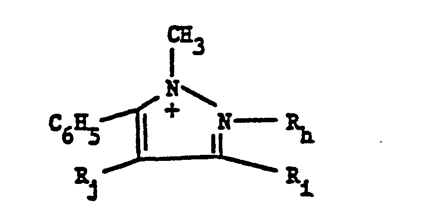 Rₕ is C₁-C₂ alkyl; R₁ is phenyl;Rⱼ is hydrogen, halogen or methyl.

Further compounds useful in the invention are of the structure: 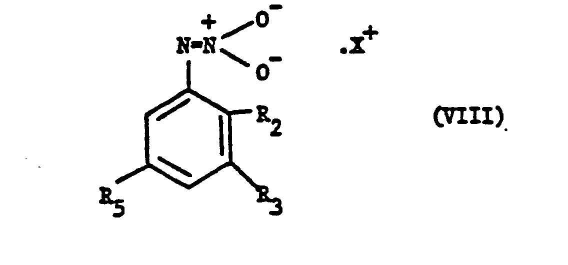 wherein R₂, R₃ and R₅ each are Br, Cl, I,F or C₁-C₄ alkyl; the cation especially those wherein X⁺is as hereinabove defined, and is preferably R₂, R₃ and R₅ all are Br.or I, and cation X⁺ is C₁₈H₃₅NH₃ or a pyrazolium cation of formula: 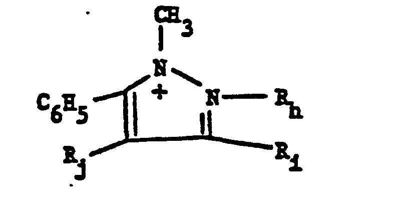 wherein Rₕ is C₁-C₂ alkyl; R₁ is phenyl; Rⱼ is hydrogen, halogen or methyl.

A further trisubstituted salt useful in the invention is the structure: 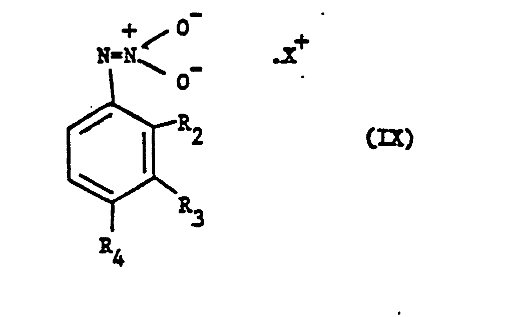 wherein R₂, R₃ and R₄ are Br, Cl, I or C₁-C₂ alkyl; cation X⁺ is as hereinabove defined, preferentially C₁₈H₃₅NH₃ or the moiety 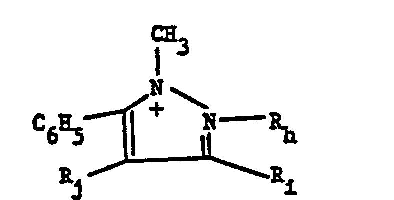 wherein Rₕ is C₁-C₂ alkyl; Rᵢ is phenyl; Rⱼ is hydrogen, halogen or methyl.

Among the tetrasubstituted salts of formula II useful in the invention are of the formula: 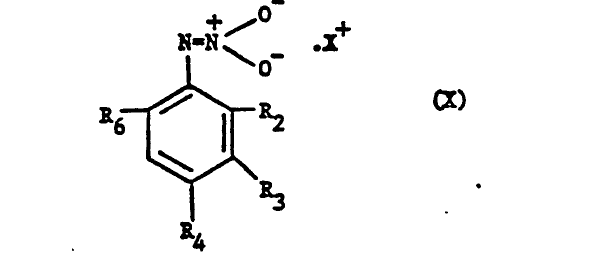 wherein R₂ and R₆ each are C₁-C₄ alkyl, Br, Cl, F or CF₃; R₃ is CH₃, CH₃O_{'} CO₂CH₃, SO₂CH₃, NH₂, NHCOCh₃, CN, NO₂, Cl or F; R₄ is C₁-C₂ alkyl, Br, Cl or F; cation X⁺ is as hereinabove defined. Of particular interest are those compounds wherein substituents R₂ to R₆* are in the combinations given below:

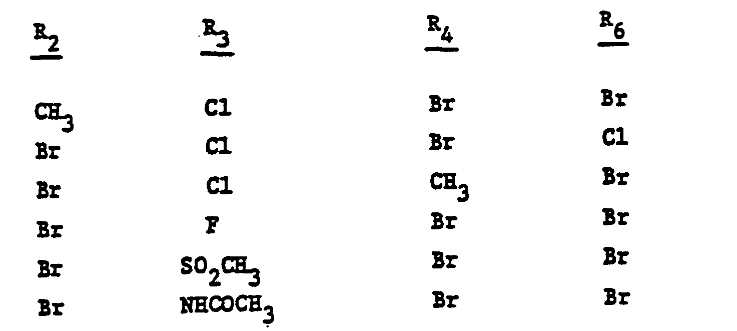

and cation X^{t} is C₁₈H₃₅NH₃ or a pyrazolium cation of formula 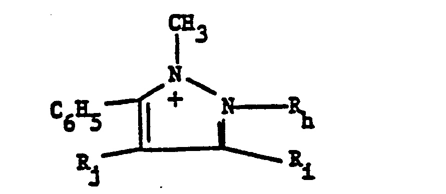 wherein Rₕ is C₁-C₂ alkyl; R₁ is phenyl; Rⱼ is hydrogen, halogen or methyl.

Among the formula II salts wherein n is 1, the following are useful in the invention: 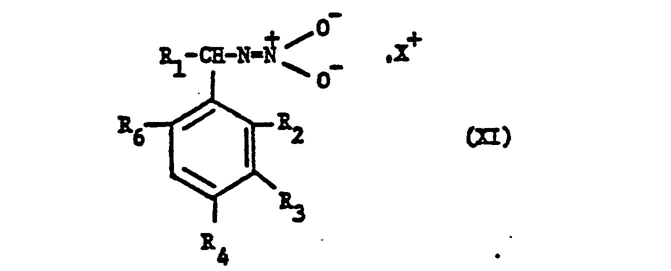 wherein R₁ is hydrogen, C₁-C₃ alkyl, C₁-C₃ haloalkyl, CN, halogen, NO₂, SO₂R₈, COR₉, phenyl or substituted phenyl and the substituents are CH₃, CF3, Ch₃O, NO₂, Br, Cl, F or OCH₂CO₂C₂H₅; R₂, R₃ and R₆ each are hydrogen, C₁-C₄ alkyl, Br. Cl. F or CF₃;R₄ is hydrogen, C₁-C₂ alkyl, Br, Cl or F; cation X⁺ is as hereinabove defined; but is preferably C₁₈H₃₅NH₃ or a pyrazolium cation of formula 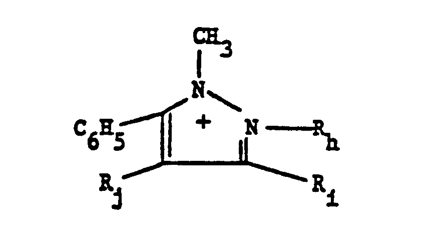 wherein Rₕ is Cₗ-_{C2} alkyl; R₁ is phenyl; Rⱼ is hydrogen, halogen or methyl; R₈ and R₉ are as hereinabove defined.

Among the compounds represented by formula I wherein n is ₀ and R₇ is other than hydrogen, the following are of useful in the invention: 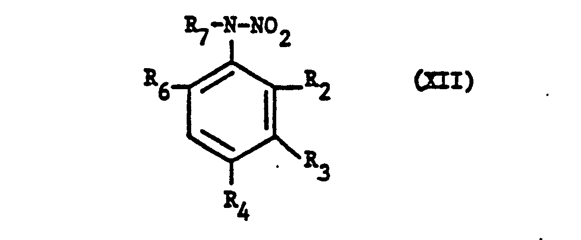 wherein R₂ and R₆ each are Cl, Br, I, NO₂; R₃ is hydrogen or C₁-C₂ alkyl; R₄ is hydrogen, halogen or Cₗ₋C₃ alkyl; R₇ is C₁-C₁₂ alkyl optionally substituted with halogen or CN, C₃₋C₄ alkenyl, C₃₋C₄ alkynyl, (C₁-C₃ alkyl)₃SiCH₂-, the methyl esters of lower alkanoic acids or the moiety 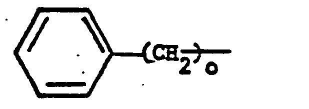 wherein o is an integer of 0,1,2 or 3 and the moiety may optionally be substituted with halogen,C₁-C₂ alkyl, C₁-C₂ alkoxy or nitro.

Among the compounds of formula Ia wherein n is 0 and R₇ is other than hydrogen the following are useful in the invention: 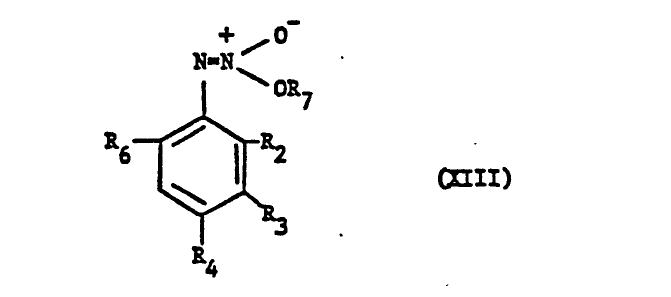 wherein R₂ and R₆ each are Cl, Br, I or NO₂; R₃ is hydrogen or C₁-C₂ alkyl; R₄ is hydrogen, halogen or Cₗ-C₃ alkyl; R₇ is C₁-C₁₂ alkyl optionally substituted with halogen or CN, C₃-C₄ alkanyl, C₃₋C₄ alkynyl, (C₁-C₃ alkyl)₃SiCH₂-, the methyl esters of lower alkanoic acids or the moiety 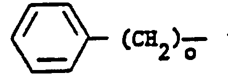 wherein o is an integer of 0,1,2 or 3 and the moiety may optionally be substituted with halogen, C₁-C₂ alkyl, C₁-C₂ alkoxy or NO₂.

Among the compounds of the invention the following compounds represented by formulae (I) and (Ia) are novel: 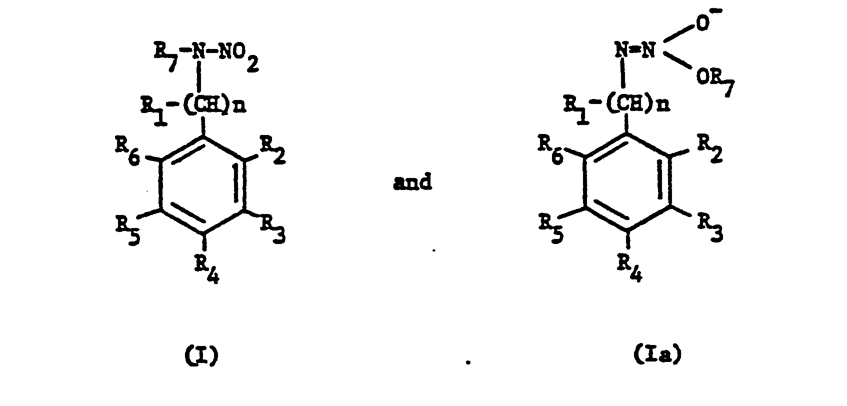 wherein n is an integer of 0 or 1; R₁ is hydrogen, C₁-C₃ alkyl, C₁-C₃ haloalkyl, CN, halogen, NO₂, SO₂R₈, CORg, phenyl or substituted phenyl and the substituents are CH₃,CF₃, CH₃O, halogen, N0₂ or OCH₂CO₂C₂H₅;B₂ and R₆ are hydrogen, halogen, C₁-C₄ alkyl, C₁-C₄ haloalkyl, C₁-C₄ haloalkcoxy, SO₂R₈, SR₈ or COR₁₀;R₃ is hydrogen, C₁-C₄ alkyl, C₁-C₄ alkoxy, NH₂, NHCOCH₃, halogen, NO₂; SO₂R₈, CN or CO₂CH₃; R₄ is hydrogen, halogen, C₁-C₂ alkyl, SO₂R₈, CORg, CN, NHCOCH₃, SO₂NR₁R₁₁, 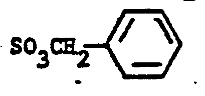 , phenoxy or substituted phenoxy and the substituents are CH₃, CF₃, OCF₃. CH₃O, halogen, NO₂ or OCH₂CO₂C₂H₅;R₅ is hydrogen, C₁-C₃ alkyl or halogen; R₇ is hydrogen, Cₗ-C₁₂ alkyl optionally substituted with halogen or CN, C₃₋C₄ alkenyl, C₃₋C₄ alkynyl, the methyl esters of lower alkanoic acide or the moiety 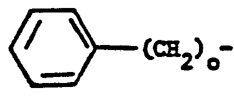 wherein o is an integer of 0,1,2 or 3, and the moiety may optionally be substituted with halogen, C₁-C₂ alkyl, C₁-C₂ alkoxy, (C₁-C₃ alkyl)₃SiCH₂ or NO_{2;} R₈ is C₁₋C₆ alkyl, phenyl or benzyl; R₉ is hydrogen, hydroxyl, C₁-C₃ alkyl, C₁-C₃ alkoxy or phenyl; R₁₀ and R₁₁ each are hydrogen, Cₗ-C₄ alkyl or phenyl; with the proviso that not less than two and not more than four of R₂ to R₆ are to be substituted with groups other than hydrogen in any one of the compounds of formulae I and Ia and mixtures thereof; and with the additional provisos that when R₁, R₅ and R₇ are hydrogen and n is 0 then:
a if only R₂ and R₃ are substituted, they cannot be both CH₃ or Cl or combinations thereof;
b if only R₂ and R₆ are substituted they cannot be both CH₃, C₂H₅, C₃H₇, Br or Cl or combinations thereof;
c if R₃ is N0₂ then R₂, R₄ and R₆ cannot be all Br; and with the further proviso that when R₃, R₅ and R₇ are all hydrogen and n is 0 then: R₂ cannot be halogen, C₁-C₄ alkyl, SO₂CH₃, C₁-C₃ haloalkyl or C₁-C₃ haloalkoxy; R₄ cannot be halogen or methyl; and R₆ cannot be halogen, CF₃ or C₁-C₄ alkyl.

Another group of novel compounds represented by formula Ib and of interest are illustrated and described as follows: 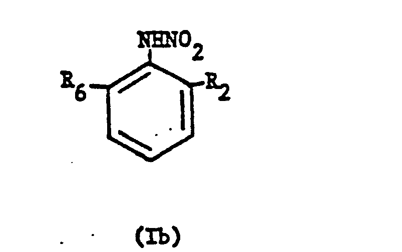 wherein R₂ and R₆ each are Br, Cl, F, I, C₁-C₄ alkyl, C₁-C₄ haloalkyl, C₁-C₄ haloalkoxy, SR₈, SO₂R₈ or COR₁₀; R₈ is C₁-C₆ alkyl, phenyl or benzyl; R₁₀ is hydrogen, C₁-C₄ alkyl or phenyl; with the proviso that R₂ and R₆ cannot be both CH₃, C₂H₅, C₃H₇ Br or Cl or combinations thereof.

The following group of novel compounds of formula (Ic) and of interest are graphically illustrated and described as follows: 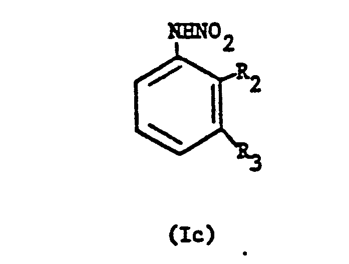 wherein R₂ is Br, Cl, F, I, C₁-C₄ alkyl, C₁-C₄ haloalkyl, Cₗ₋C₄ haloalkoxy, SR₈, SO₂R₈ or COR₁₀; R₃ is Br, Cl, F, I, C₁-C₄ alkyl, C₁-C₄ alkoxy, NH₂, NHCOH₃, NO₂, SO₂R₈, ^{C}N or CO₂CH₃; R₈ is C₁-C₆ alkyl, phenyl or benzyl; R₁₀ is hydrogen, C₁-C₄ alkyl or phenyl; with the proviso that R₂ and R₃ cannot be both CH₃ or Cl or combinations thereof.

Another group of novel compounds of formula (_{Id}) and of interest are graphically illustrated and described as follows: 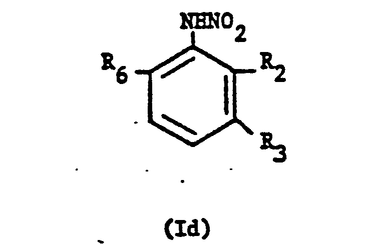 wherein R₂ and R₆ are Br, Cl, F, I, C₁-C₄ alkyl, C₁-C₄ haloalkyl, C₁-C₄ haloalkoxy, SO₂R₈, SR₈ or COR₁₀; R₃ is Br, Cl, F, I, C₁-C₄ alkyl, C₁-C₄ alkoxy, Hh₂, NHCOCH₃, NO₂, SO₂R₈, CN or CO₂CH₃; R₈ is C₁-C₆ alkyl, phenyl or benzyl, R₁₀ is hydrogen, C₁-C₄ alkyl or phenyl.

A group of novel compounds represented by formula Ie and useful in the invention are graphically illustrated and described as follows: 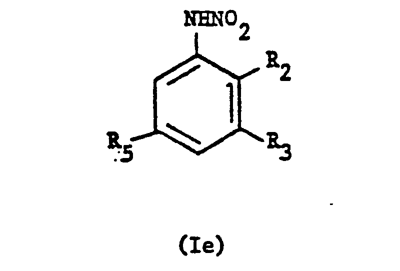 Wherein R₂ is Br, C1, F, I, C₁-C₄ alkyl, C₁-C₄ haloalkyl, C₁-C₄ haloalkoxy, SR₈, SO₂R₈ or COR₁₀; R₃ is Br, C1, F, I, Cₗ₋C₄ alkyl, C₁-C₄ alkoxy, NH₂, NHCOCH₃, NO₂, SO₂R₈, CN or CO₂CH₃; R₅ is Br, Cl, F, I or C₁-C₃ alkyl; R₈ is -C₁- C₆ alkyl, phenyl or benzyl; R₁₀ is hydrogen, C₁-C₄ alkyl or phenyl.

Still another group of novel compounds represented by formula If and of interest are graphically illustrated and described as follows: 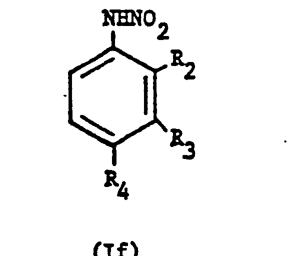 wherein R₂ is Br,Cl, F, I, C₁-C₄ alkyl, C₁-C₄ haloalkyl, C₁-C₄ haloalkoxy, SO₂R₈, SR₈ or COR₁₀; R₃ is Br, C1, F, I, C₁-C₄ alkyl, C₁-C₄ alkoxy, NH₂, NHCOCH₃, NO₂, SO₂R₈, CN.or CO₂CH₃; R₄ is Br, Cl, F, I, C₁-C₂ alkyl, SO₂R₈, COR₉, CN, NHCOCH₃, SO₂NR₁₀R₁₁' 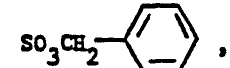 phenoxy or substituted phenoxy and the sub- wtituents are CH₃,CF₃, CH₃O, Br, C1, F, I, AO₂ or OCH₂CO₂C₂H₅; R₈ is C₁-C₆ alkyl, phenyl or benzyl; R₋₉ is hydrogen, hydroxyl, C₁-C₃ alkyl, C₁-C₃ alkoxy or phenyl; R₁₀ and R₁₁ each are hydro-Sen, C₁-C₄ alkyl or phenyl.

A group of novel compounds represented by formula _{I}g and useful in the invention are graphically illustrated and described as follows: 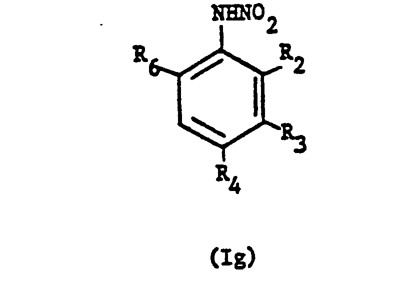 vrherein R₂ and R₆ are Br, Cl, F, I, C₁-C₄ alkyl, C₁-C₄ haloalkyl, C₁₋C₄ haloalkoxy, SO₂R₈, SR₈ or C_{O}R₁₀; R₃ is C₁-C₄ alkyl, C₁-C₄ alkoxy, NH₂, NHCOCH₃, Br, Cl, F, I, NO₂, SO₂R₈, CN or CO₂CH₃; R₄ is Br, Cl, F, I, C₁-C₂alkyl, SO₂R₈, COR₉, CN, NHCOCH₃, SO₂HR₁₀R₁₁' 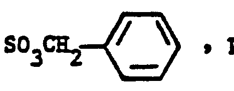 phenoxy or substituted phenoxy and the substituents are CH₃, CF₃, Ca₃O, Br, Cl, F, I, NO₂ or OCH₂CO₂ C₂H₅; R₈ is C₁-C₆ alkyl, phenyl or benzyl; R₉ is hydrogen, hydroxyl, Cₗ-C₃ alkyl, Cₗ-C₃ alkoxy or phenyl; R₁₀ and R₁₁ are hydrogen, C₁-C₄ alkyl or phenyl; with the proviso that when R₃ is NO₂, then R₂, R₄ and R₆ cannot be all Br.

A group of novel compounds represented by formula Ih and of interest are graphically illustrated and described as follows: 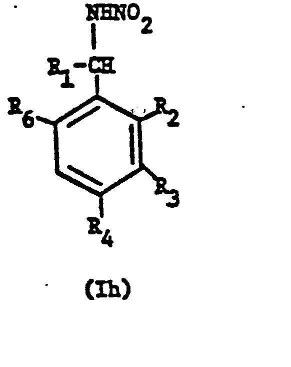 wherein R₁ is hydrogen, C₁-C₃ alkyl, Cₗ-C₃ haloalkyl, CN, Br, Cl, F, _{I}, NO₂, SO₂R₈, COR₉, phenyl or substituted phenyl and the sub- stitilents are CH₃, CF₃, CH₃0, N0₂ or OCH₂CO₂C₂H₅; R₂ and R₆ are hydrogen, Br, Cl, F, I, C₁-C₄ alkyl, C₁-C₄ haloalkyl, ^{C}1^{-C}₄ haloalkosy, SO₂R₈, SR₈ or COR₁₀; R₃ is hydrogen, C₁-C₄ alkyl, C₁-C₄ alkoxy, NH₂, NHCOCH₃, Br, Cl, F, I, NO₂, SO₂R₈' CN or CO₂CH₃; R₄ is hydrogen, Br, Cl, F, I, C₁-C₂ alkyl, SO₇R₈, COR₉, CN, NHCOCH₃, SO₂NR₁₀R11, 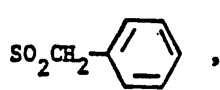 phenoxy or substituted phenoxy and the substituents are CH₃, CF₃, CH₃O, Br, Cl, F, I, NO₂ or OCH₂CO₂C₂H₅; R₈ is C₁-C₆ alkyl, phenyl or benzyl; R₉ is hydrogen, hydroxyl, C₁-C₃ alkyl, Cₗ-C₃ alkoxy or phenyl; R₁₀ and R₁₁ are hydrogen, C₁-C₄ alkyl or phenyl; with the proviso that not less than two of R₂ to R₆ are to be substituted with groups other than hydrogen.

Still another group of novel compounds of formula Iᵢ useful in the invention are graphically illustrated and described as follows: 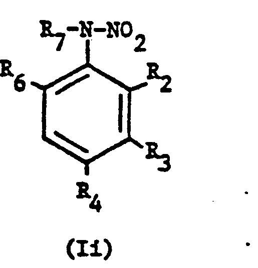 wherein R₂ and R₃ are hydrogen, Br, Cl, F, I, C₁-C₄ alkyl, C₁-C₄ haloalkyls C₁-C₄ haloalkoxy, SO₂R₈, SR_{8'} or COR₁₀; R₃ is hydrogen, C₁-C₄ alkyl, C₁-C₄ alkoxy, NH₂, NHCOCH₃, Br, Cl, F, I, NO₂, SO₂R₈, CN or CO₂M₃ R, is hydrogen, Br, Cl, F, I, C₁-C₂ alkyl, SO₂R₈, COR₉, CN, NHCOCH₃, SO₂NR₁₀R₁₁ 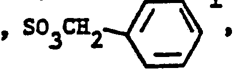 phenoxy or sub- stituted phenoxy and the substituents are CH₃, CH₃' CH₃"' Br, Cl, F,I, NO₂ or OCH₂CO₂C₂H₅; R₇ is C₁-C₁₂ alkyl optionally substituted with halogen or CN, C3-C4 alkenyl, C₃-C₄ alkynyl, the methyl esters of lower alkanoic acids or the moiety 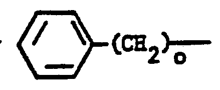 wherein o is an integer of 0,1,2 or 3, and the moiety may optionally be substituted with Br, Cl, F, I, C₁-C₂ alkyl, C₁-C₂ alkoxy, (C₁-C₃ alkyl)₃SiCH₂-' or NO₂; R₈ is C₁-C₆ alkyl, phenyl or benzyl; R₉ is hydrogen, hydroxyl, C₁-C₃ alkyl, C₁-C₃ alkox^{y} or phenyl; R₁₀ and R₁₁ are hydrogen, C₁-C₄ alkyl or phenyl; with the provisos that when; a R₂, R₄ and R₆ are Br then R₇ cannot be CH₃; b and when R₂ and R₆ are both Br then R₇ cannot be Cₗ₋C₄ alkyl, and o cannot be an integer of 1,2 or 3; c and when R₂ and R₆ are both CH₃ then o cannot be 3.

The novel group of compounds represented by formula ij and of particular interest are graphically illustrated and described as follows: 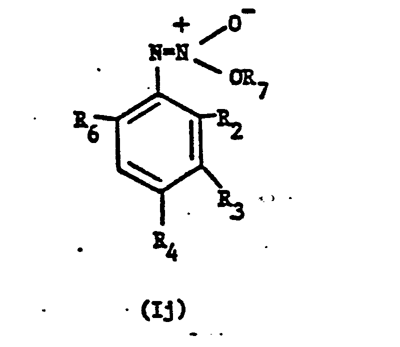 wherein R₂ and R₆ are hydrogen, Br, Cl, F, I, C₁-C₄ alkyl, C₁-C₄ haloalkyl, C₁-C₄ haloalkoxy, SO₂R₈, SR₈ or COR₁₀; R₃ is hydrogen, C₁-C₄ alkyl, C₁-C₄ alkoxy, NH₂ ,NHCOCH₃, Br, Cl, F, I, NO₂, SO₂R₈, CN or COCH₃; R₄ is hydrogen, Br, Cl, F, I, Cₗ₋C₂ alkyl, SO₂R₈, COR₉, CN, NRCOCH₃, SO₂NR₁₀R₁₁, 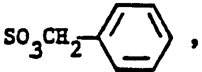 phenoxy or substituted phenoxy and the substituents are CH_{3'} CF_{3'} CH₃O, Br, Cl, ^{F,} I, NO₂ or OCH₂CO₂C₂H₅; R₇ is C₁-C₁₂ alkyl optionally substituted with halogen or CN, C₂-C₄ alkenyl, C₂-C₄ alkynyl, the methyl esters of lover alkanoic acids or the moiety 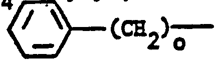 wherein o is an integer of 0,1,2 or 3, and the moiety may optionally be substituted with Br, Cl, F, I, C₁-C₂ alkyl, C₁-C₂ alkoxy, (C₁-C₃ alkyl)₃SiCH₂- pr NO₂; R₈ is C₁-C₆ alkyl, phenyl or benzyl; R₉ is hydrogen, hydroxyl, C₁-C₃ alkyl, C₁-C₃ alkoxy or phenyl; R₁₀ and R₁₁ are hydrogen, C₁-C₄ alkyl or phenyl.

The hereinabove identified compounds of formulae I and Ia may also form a group of novel inorganic and organic salts represented by formula II and useful in the invention, graphically illustrated and described as follows: 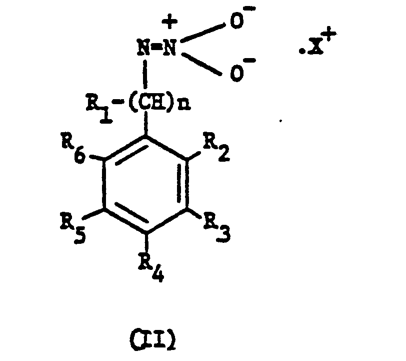 wherein n is an integer of 0 or 1; R₁ is hydrogen, C₁-C₃ alkyl, C₁-C₃ haloalkyl, CN, Br, Cl, F, I, NO₂, SO₂R₈, COR₉, phenyl or substituted phenyl and the substituents are CH₃, CF₃, CH₃O, Br, Cl, F, I, NO₂ or OCH₂CO₂C₂H₅; R₂ and R₆ are hydrogen, halogen, C₁-C₄ alkyl, _{Cl}-_{C4} haloalkyl, Cₗ-C₄ haloalkoxy, SO₂R₈, SR₈ or COR₁₀; R₃ is hydrogen, C₁-C₄ alkyl, C₁-C₄ alkoxy, NH₂, NHCOCH₃, Br, Cl, F, I, NO₂, SO₂R₈, CN or CO₂CH₃; R₄ is hydrogen, Br, Cl, F, I, C₁-C₂ alkyl,SO₂R₈, COR₉, CN, HSCOCH₃, SO₂HR₁₀R₁₁, OCF₃, 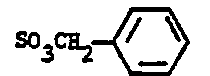 , phenoxy or substituted phenoxy and the substituents are CH₃, CF₃, CH₃O, Br, Cl, F, I, NO₂ or OCH₂CO₂C₂H₅; R₅ is hydrogen, C₁-C₃ alkyl, Br, Cl, F, or I; X⁺ is Cu or organic;

when the cation X⁺ is organic, X⁺ is represented by formula IIIa 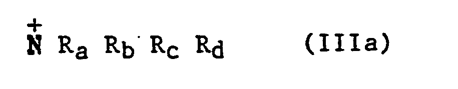 wherein Rₐ, R_{b}, R_{c}, and R_{d} are as defined above; organic cation X⁺ may also be imidazolium, pyridinium, quinolinium, dithiolium, tetrazolium or be represented by formula IIIb 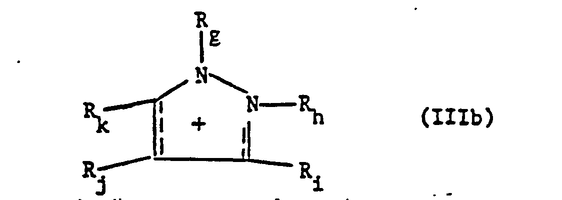 wherein Rg, Rₕ, Ri, Rj and Rₖ are is defined above.

A group of novel salts represented by formulae II, IV and IVa in the disclosure and of special interest are graphically.illustrated and described as follows: 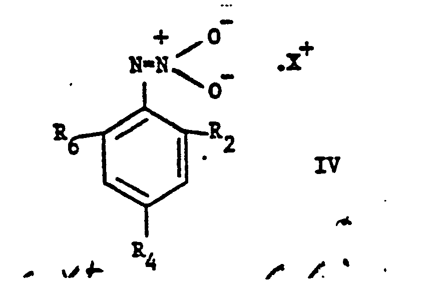 ^{R}2, ^{R}4, and X⁺ are as defined above; and _{X}⁺ may also be represented by formulae IIIf and and IIIg 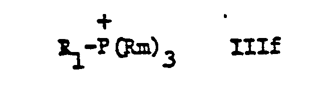 wherein R₁ is C₁-C₆ alkyl, 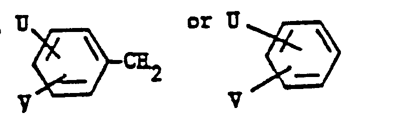 wherein U and V are as hereinabove defined; Rₘ is C₁-C₆ alkyl or 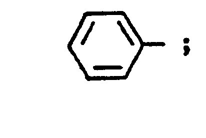 + S RₐR_{b}R_{c} (IIIg) and I RₐR_{b} (IIIh) , wherein Rₐ,R_{b} and R_{c} are as hereinabove defined; with the provisos that when R₂, R₄ and R₆ each are Br, Cl or at least one of them is CH₃, cation X⁺ cannot be Ba²⁺ ;and when R₂, R₄ and R₆ are Dr , Cl or mixtures thereof , X ⁺ cannot be Na; and when R₂, R₄ and R₆ each are Br, X⁺ cannot be K⁺ , Ag⁺, HH₄⁺ or anilino; and with the proviso that when R₂ is halogen, C₁-C₄ alkyl, SO₂CH₃, C₂-C₃ haloalkyl or C₁-C₃ haloalkory; R₄ is halogen, or methyl; R₆ is halogen, CF₃ or C₁-C₄ alkyl, and the salts are of formula IV above; then X⁺ cannot be alkali metals, ammonium, H₃N⁺Rₐ, E₂N⁺ RₐR_{b}, HN⁺RₐR_{b}R_{c} and NRₐR_{b}R_{c}R_{d}, wherein the R groups: Rₐ to R_{d} each are selected from C₁-C₁₂ alkyl, C₂-C₄ hydroxyalkyl, aryl, substituted aryl wherein the substituents are selected from halogen and C₁-C₆ alkyl, aralkyl C₁-C_{3'} and substituted beazyl wherein the substituents are selected from halogen and C₁-C₆ alkyl, nor can X⁺ be a heterocyclic ring which may be formed when two of the R₈ to R_{d} groups are taken together with the nitrogen to which they are attached to.

Another group of novel salts represented by formula V are of interest and are graphically illustrated and described as follows: 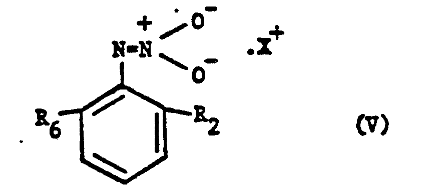 wherein R₂, R₆ and X⁺ are as defined above.

The following group of novel salts of formula (VI) and of interest are graphically illustrated and described as follows: 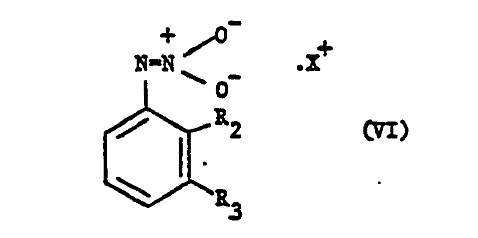 wherein R₂ is Br, Cl, F, I, C₁-C₄ alkyl, C₁-C₄ haloalkyl, C₁-C₄ haloalkczy, SR₈, SO₂R₈ or COR₁₀; R₃ is Br, Cl, F, I, C₁-C₄ alkyl, C₁-C₄ alkoxy . NH₂, NHCOCH₃, NO₂, SO₂R8, CN or CO₂CH₃; R₈ is C₁-C_{6,} alkyl, phenyl or benzyl; R₁₀ is hydrogen, C₁-C₄ alkyl or phenyl; X⁺ is as defined above.

A group of novel salts represented by formula VII and useful in the invention are graphically illustrated and described as follows: 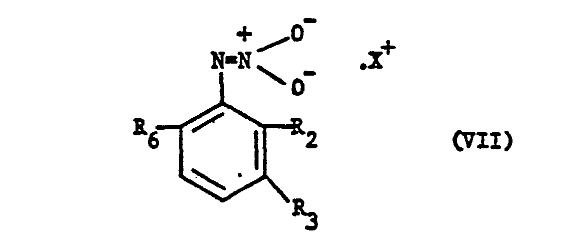 wherein R₂ and R₆ each are Br, Cl, F, I, C₁-C₄ alkyl, C₁-C₄ haloalkyl, C₃-C₄ haloalkoxy, SR₈, SO₂R₈ or COR₁₀; R₃ is Br, Cl, F, I, C₁-C₄ alkyl, C₁-C₄ alkoxy, NH₂, NHCOCH₃, NO₂, SO₂R₈, CN or CO₂CH₃; R₈ is C₁-C₆ alkyl, phenyl or benzyl; B₁₀ is hydrogen, Cₗ-C₄ alkyl or phenyl; X⁺ : as defined above.

A group of novel salts represented by formula VIII and useful in the invention are graphically illustrated and described as follows: 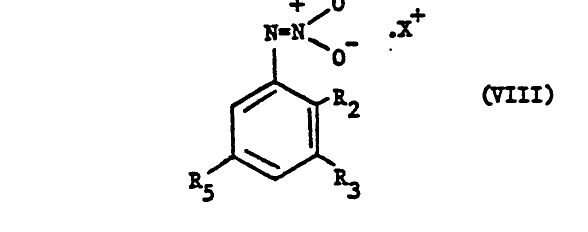 wherein R₂ is Br, Cl, F, I, C₁-C₄ alkyl, C₁-C₄ haloalkyl, C₁-C₄ haloalkoxy, SR₈, SO₂R₈ or COR₁₀; R₃ is Br, Cl, F, I, C₁-C₄ alkyl, C₁-C₄ alkoxy, NH₂, SHCOCH₃, NO₂, SO₂R₈, CN or CO₂CH₃; R₅ is Br, Cl, F, I or C₁-C₃ alkyl; R₈ is C₁-C₆ alkyl, phenyl or benzyl; R₁₀ is hydrogen, C₁-C₄ alkyl or phenyl; X⁺ 1 as defined above.

Another group of novel salts represented by formula IX and useful in the invention are graphically illustrated and described as follows: 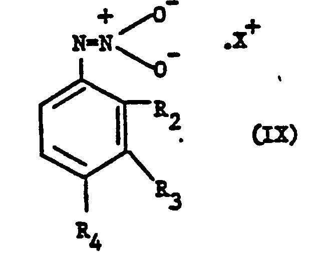 wherein R₂ is Br, Cl, F, I, C₁-C₄ alkyl, C₁-C₄ haloalkyl, C₁-C₄ haloalkaxy, SR₈ SO₂R₈ pr COR₁₀; R₃ is Br, Cl, F, I, C₁-C₄ alkyl, C₁-C₄ alkaxy, NH₂, NHCOCH₃, NO₂,SO₂R₈, CN or CO₂CH₃; R₄ is Br, Cl, F, I, C₁- C₂ alkyl, SO₂NR10R₁₁, NHCOCH₃, CN, SR₈, SO₂R₈, 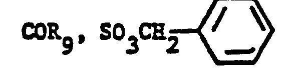 , phenoxy or substituted phenoxy and the substituents are CH₃, CF₃, OCH₃, Br, Cl, F, I, NO₂ or OCH₂CO₂C₂H₅; R₈ is _{Cl-C6} alkyl, phenyl or benzyl; R₉ is hydrogen, hydroxyl, C₁-C₃ alkyl, C₁-C₃ alkoxy or phenyl; R₁₀ and R₁₁ each are hydrogen, C₁-C₄ alkyl or phenyl; X⁺ is as defined above.

Another group of novel salts represented by formula (X) and of interest are graphically illustrated and described as follows: 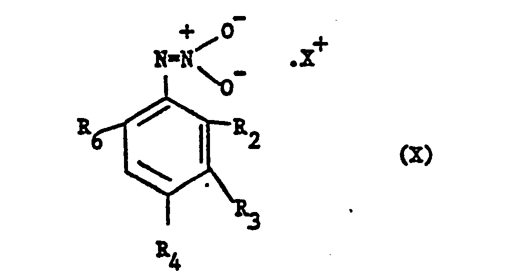 wherein R₂ and R₆ are Br, Cl, F, I, C₁-C₄ alkyl, C₁-C₄ haloalkyl, C₁-C₄ haloalkoxy, SO₂R₈, SR₈ or COR₁₀; R₃ is C₁-C₄ alkyl, C₁-C₄ alkoxy, NH₂, NHCOCH₃, Br, Cl, F, I, NO₂, SO₂R₈, CN or CO₂CH₃; R4 is Br, Cl, F, I, C₁-C₂ alkyl, SO₂R₈, COR₉, CN, NHCOCH₃, SO₂NR₁₀R₁₁, 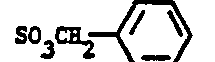 phenoxy or substituted phenoxy and the substituents are CH₃, CF₃, CH₃O, Br, Cl, F, I, NO₂ or OCH₂CO₂C₂H₅; R₈ is C₁-C₆ alkyl, phenyl or benzyl; R₉ is hydrogen, hydroxyl, C₁-C₃ alkyl, Cₗ-C₃ alkoxy or phenyl; R₁₀ and R₁₁ are hydrogen, C₁-C₄ alkyl or phenyl; X⁺ is as detined above.

Still another group of novel salts represented by formula XI and of interest are graphically illustrated and described as follows: 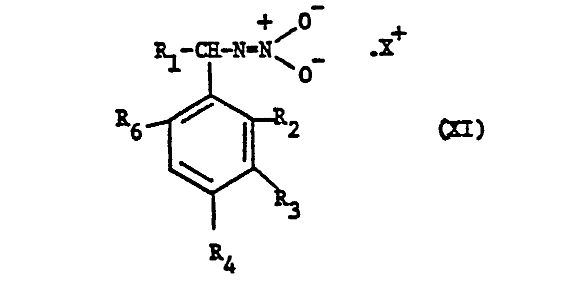 wherein R₁ is hydrogen, Cₗ-C₃ alkyl, C₁-C₃ haloalkyl, CN, Br, Cl, F, I, NO₂, SO₂R₈, COR₉, phenyl or substituted phenyl and the substituents are CH₃, CF₃, CH₃O, N0₂ or OCH₂CO₂C₂H₅; R₂ and R₆ are hydrogen, Br, Cl, F, I, C₁-C₄ alkyl, C₁-C₄ haloalkyl, C₁-C₄ heloalkoxy, SO₂R₈, SR₈, COR₁₀; R₃ is hydrogen, C₁-C₄ alkyl, C₁-C₄ alkoxy, NH₂, NHCOCH₃, Br, Cl, F, I, NO₂, SO₂R₈, ^{CN} or CO₂CH₃ ; ^{R}₄ is hydrogen, Br, Cl, F, I, C₁-C₂ alkyl, SO₂R₈, COR₉, CN, NHCOCH₃, SO₂NR₁₀R₁₁, 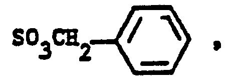 phenoxy or substituted phenoxy and the substituents are CH₃, CF₃, CH₃O, Br, Cl, F, I, NO₂ or OCH₂CO₂C₂H₅; R₈ is C₁-C₆ alkyl, phenyl or benzyl; R₉ is hydrogen, hydroxyl, C₁-C₃ alkyl, C₁-C₃ alkaxy or phenyl; R₁₀ and R₁₁ are hydrogen, Cl-C4 alkyl or phenyl; X⁺ is as detined above.

The invention.relates to methods for treating graminaceous crops, leguminous crops, cotton, sunflowers and herbaceous ornamental plants to increase axillary branching, improve the canopy and enhance flowering thereof. Surprisingly, application of the above-said salts to the foliage of the above-identified crops has the further advantage that said salts produce a dwarfing effect in said plants while increasing the stem stiffness thereof. They also enhance yield therefrom.

Dwarfing and/or stiffening of the stems of cotton, legumes, graminaceous crops and sunflowers is most advantageous to the farmer since lodging of these crops usually results in reduced yields of the affected crops.

Lodging, in the present application, refers to the deflection of the plant from the vertical, varying in degree from only a slight deflection to complete deflection (i.e. plants prone) caused by, in most cases, the action of wind and/or-rain on the plants. This deflection is such that when the causal agent (wind, rain) is no longer present the deflection is neither immediately nor completely overcome. Moreover, where extensive or severe lodging has occurred, the crop may be difficult to harvest and the yield markedly reduced.

In practice we have found that application to the foliage of seedling plants of from about 0.06 to 2.0 kg/ha and preferably about 0.125 to 0.50 kg/ha is sufficient to achieve the several desirable and advantageous biological responses in plants described above.

Inasmuch as the salts or compounds of formula (I) of the present invention have only limited solubility in water, they are generally formulated for foliar application as wettable powders, flowable dispersions or emulsion concentrates, which are usually dispersed in water or in other, inexpensive, liquid diluents for application to the foliage of said plants as a liquid spray.

A typical wettable powder can be prepared by grinding together approximately 46X by weight of a finely divided carrier such as attapulgite, 50% by weight of the pyrazolium, pyrazolinium or pyrazolidine salt of 2,4,6-trisubstituted-N-nitroaniline, 3X by weight of the sodium salt of condensed naphthalene sulfonic acids and 1% by weight of sodium N-methyl-N-oleoyltaurate.

A typical flowable dispersion can be prepared by admixing about 42X by weight of the N-nitroaniline salt with about 3% by weight of the sodium salt of condensed naphthalene sulfonic acids, 2% by weight of finely divided bentonite and 53% by weight of water.

Emulsion concentrates may be prepared by dissolving 15% to 70X by weight of the formula (I) compound in 85X to 30% of a solvent such as N-methylpyrrolidone, lower alcohols, methylisobutylketone, 2-methoxy ethanol, propylene glycol; diethylene glycol, diethylene glycol monomethyl ether, formamide, methylformamide, and the like, and mixtures thereof. Advantageously, surfactants such as polyoxyethylated vegetable oil or an alkyl phenoxy polyoxyethylene ethanol are also incorporated in amounts of 1% to 5% by weight of said concentrate.

In accordance with this invention, the substituted N-nitroaailines (phenylnitramines) can be prepared from the corresponding substituted anilines by a variety of conventional procedures. For illustrative purposes, one such procedure is hereinbelow graphically illustrated and described as follows: 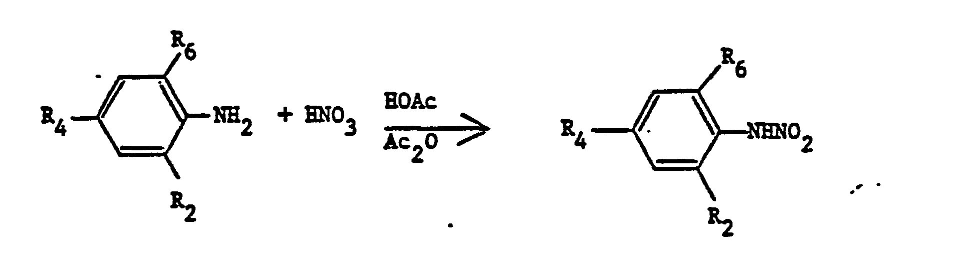 wherein R_{2'} R₄ and R₆ are as defined above. The above synthesis is conveniently carried out in a solvent, such as acetic acid, preferably in the presence of acetic anhydride. The product may be precipitated from the reaction mixture by the addition of ice water. Purification may be effected by conventional procedures such as recrystallization, chromatography, and the like.

The nitroanilines, wherein R₂ and/or R₆ are haloalkyl or hydrosyalkyl and R₄ is halogen, can be prepared by a two-step synthesis involving Step 1, the reaction of the appropriate haloalkyl aniline or haloalkoxyaniline with halogen (e.g. chlorine or bromine) in the presence of an acid acceptor such as sodium acetate, potassium acetate, or the like, in the presence of an inert organic solvent (e.g. tert-butanol, CCl₄, ethylene dichloride, chlorobenzene, or the like). The reaction is generally conducted at a temperature in the range of from 25°C to 75°C, and preferably at a temperature of about 45°C to 55°C. The reaction yields the corresponding 2,4,6-trisubstituted aniline, which may then be converted, in Step 2, to the corresponding 2,4,6-trisubstituted N-nitroaniline by reaction with nitric acid in the presence of a solvent, such as acetic acid, and acetic anhydride. Steps 1 and 2 can be graphically illustrated as follows: 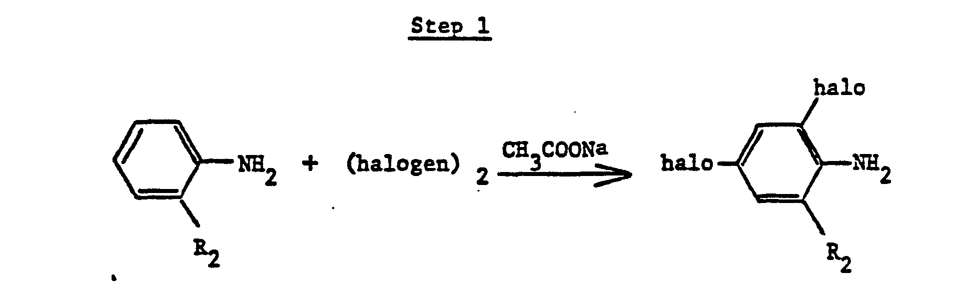 or 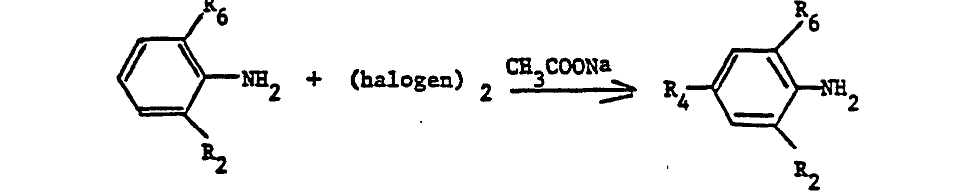 wherein R₂ and R₆ are haloalkyl or haloalkoxy, and R₄ is chlorine or bromine.

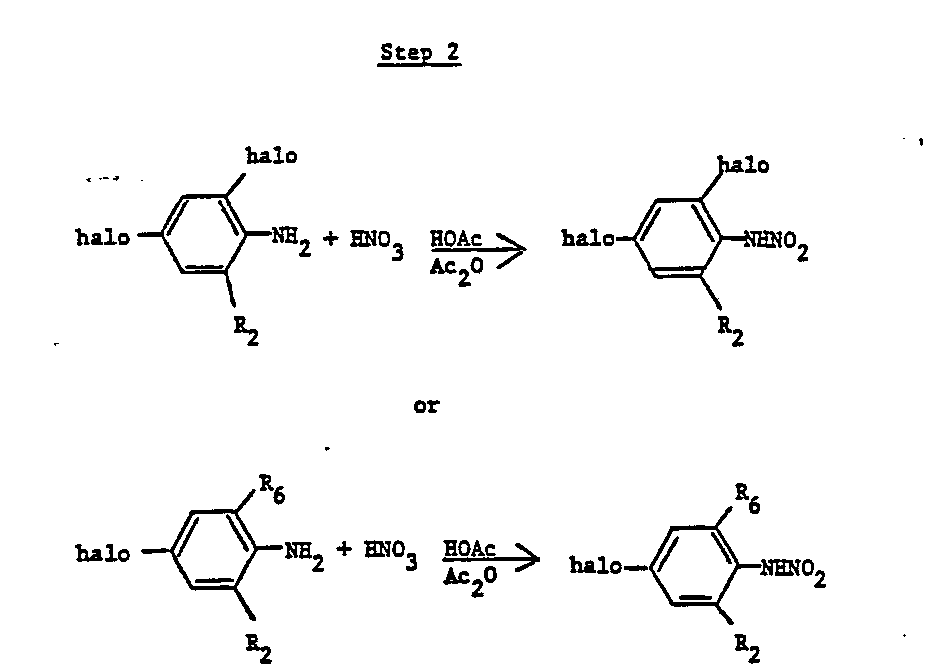

The formula (II) salts of the substituted -N-nitroaniline compounds can be conveniently prepared from the substituted nitroaniline compounds by one or more procedures, hereinafter referred to as Methods A,B,C,D or E.

### Methods for the preparation of salts of substituted N-nitroanilines

### Method A

The N-nitroaniline is dissolved in dilute aqueous sodium or potassium hydroxide and filtered. To this solution is added one molar equivalent of the appropriate salt and dissolved in water. In most cases the product separates as a solid, and is removed by filtration and purified by recrystallization. Whenever no precipitation occurs or the product separates as an oil, an extraction with methylene chloride is performed. Concentration of this extract yields the product which may be further purified by recrystallization.

### Method B

A solution of the N-nitroaniline in anhydrous ether is treated with a solution of one equivalent of the appropriate nitrogen base in ether. The desired product is removed by filtration and purified by washing and recrystallization. Precipitation of the product may be promoted by the addition of petroleum ether.

### Method C

To a methanolic suspension of the silver salt of the N-nitroaniline is added one equivalent of the halide salt of the appropriate amine dissolved in methanol. The mixture is stirred at 25⁰C for one hour. The precipitated silver halide is removed by filtration, the product is recovered by evaporating the methanolic solution and purified by recrystallization.

### Method D

An aqueous solution of the sodium salt of the N-nitroaniline is saturated with a large excess of the desired metal salt until solids remain undissolved. After stirring for one hour, the solids are removed by filtration and recrystallized or washed with a suitable solvent,.and dried.

### Method E

A methylene chloride solution of the free N-nitroaniline is treated with a three-fold excess of an aqueous solution of the appropriate metal hydroxide, acetate or carbonate. If precipitation occurs, the product is removed by filtration and recrystallized. Otherwise, the aqueous layer is separated and evaporated to dryness. The residue is treated with ethyl acetate and filtered. Removal of the ethyl acetate affords the product which may be recrystallized, if necessary.

Formula (I) compounds wherein R₇ is a substituent other than hydrogen may be conveniently prepared as follows:

The appropriately substituted formula (I) compound (R₇ = hydrogen) is reacted in with an alcoholic solution of 1 equivalent of sodium hydroxide in the presence of a lower alcohol such as methanol, and the thus formed sodium salt is isolated by evaporating the reaction mixture to dryness. Next, the sodium salt is dissolved in dimethyl sulfoxide (DMSO) and treated with the appropriate alkyl halide at a temperature range from about 20°C to about 30°C, preferably 25°C for a period of time sufficient to essentially complete the reaction. The reaction mixture is then poured into water, the product extracted with a water immiscible solvent such as ether and recovered from said extract by standard laboratory procedures. The above reaction sequence may be graphically illustrated as follows: 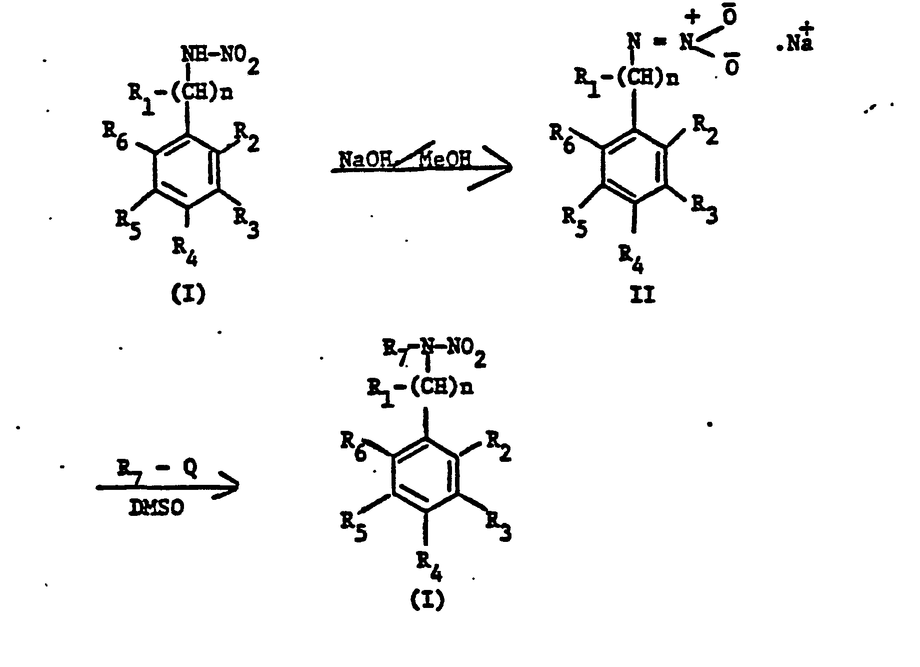 Wherein in the above reaction sequence Q is halogen, n, R, to R₇ are as hereinabove defined except that in the R₇ - Q formula R₇ cannot be hydrogen.

Formula (Ia) phenylnitramines may be prepared by the following route:
A formula II silver salt is suspended in ether under an inert atmosphere such as N₂, and reacted with 1·1 equivalent of the appropriate R₇ - Q compound at a temperature range of from about 20 C to about 30°C and preferably 25°C for a period of time sufficient to essentially complete the reaction. The thus obtained 0 - alkyl derivative of formula (Ia) structure is isolated and purified by standard laboratory procedures. The above reaction sequence may be graphically illustrated as follows:

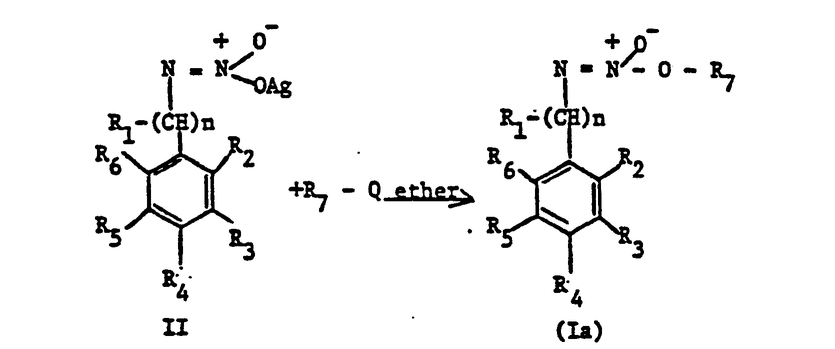

It is recognized of course, that in the course of the preparation of formula (I) compounds wherein R₇ is other than hydrogen formula (Ia) compounds are also formed, and vice versa.

Formula (I) compounds wherein n is 1 and R₇ is hydrogen may be conveniently prepared as follows:
A solution of the appropriate benzyl halide or mesylate and ammonium nitrourethane is heated in the presence of dry dimethyl sulfoxide at about 80°C for about 4 hours or a period of time sufficient to essentially complete the reaction. The reaction mixture is then cooled down, poured in water extracted with a water immiscible organic solvent such as ether and the solution dried over anhydrous magnesium sulfate. Next, anhydrous ammonia gas is bubbled through the solution. The precipitated material is then isolated, dissolved in water and precipitated from the aqueous solution with a dilute acid. The thus obtained product is then isolated by standard laboratory methods and further purified, if so desired. The above reaction sequence may be graphically illustrated as follows: 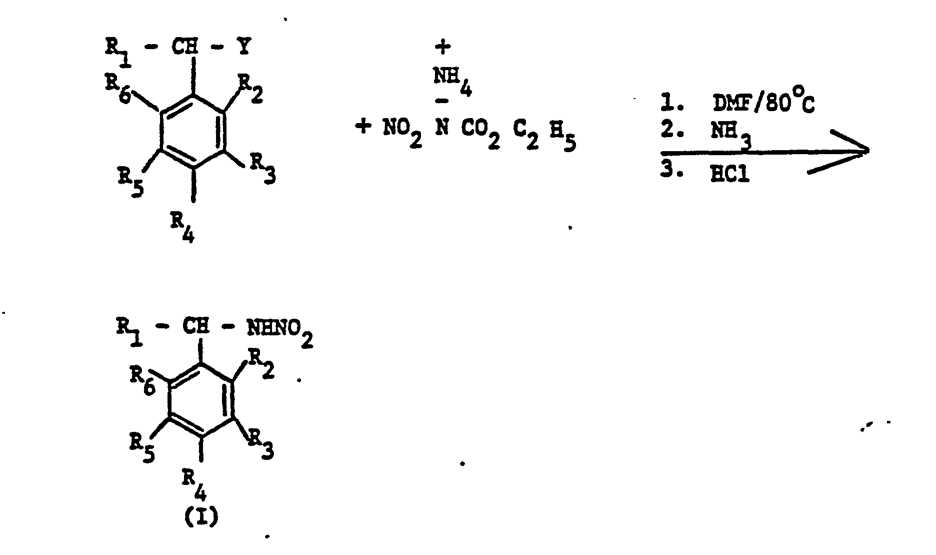 Wherein R₁ to R₆ are as hereinabove defined, Y is Br, Cl or CH₃SO₃.

The intermediate substituted anilines may also be N - nitrated to yield the corresponding N - nitroanilines as follows:

Diisopropylamine is lithiated with an equimolar amount of n - butyllithium in an inert solvent such as ether at about -75 to -80°C. Next a solution of the appropriately substituted aniline is added to the above mixture at about -60°C, the reaction mixture warmed to about 0°C and methyl nitrate added. The reaction mixture is allowed to warm slowly to room temperature and the product product isolated by standard laboratory procedures.

Surprisingly, it has also been found that the formula I :ompounds of this invention are effective for reducing the relative stem growth of broadleaf plants and for increasing the stem stiff- ness thereof, particularly for broadleaf agronomic crops such as sunflowers, when said compounds are applied preemergence to soil Ln which seeds of said plants have been sown. When used in this manner said formula I compounds are generally applied at rates of from 0.125 to 2.0 and preferably 0.25 to 0.75 kg/ha.

The invention is further illustrated by the Examples set forth below,

### EXAMPLE 1

### Methods for the preparation of salts of 2,4,6-trisubstituted N-nitroanilians

### Method A

The N-nitroaniline is dissolved in dilute aqueous sodium or potassium hydroxide and filtered. To this solution is added . one molar equivalent of the appropriate salt and dissolved in water. In most cases the product separates as a solid, and is removed by filtration and purified by recrystallization. Whenever no precipitation occurs or the product separates as an oil, an extraction with methylene chloride is performed. Concentration of this extract yields the product which may be further purified by recrystallization.

### Method B

. A solution of the N-nitroaniline in anhydrous ether is treated with a solution of one equivalent of the appropriate nitrogen base in ether. The desired product is removed by filtration and purified by washing and recrystallization. Precipitation of the product may be promoted by the addition of petroleum ether.

### Method C

To a methanolic suspension of the silver salt of the N-nitroaniline is added one_{.}equivalent of the halide salt of the appropriate amine dissolved in methanol. The mixture is stirred at 25°C for one hour. The precipitated silver halide is removed by filtration, the product recovered by evaporating the methanolic solution and purified by recrystallization.

### Method D

An aqueous solution of the sodium salt of the N-nitroaniline is saturated with a large excess of the desired metal salt until solids remain undissolved. After stirring for one hour, the solids are removed by filtration and recrystallized or washed with a suitable solvent, and dried.

### Method E

A methylene chloride solution of the free N-nitroaniline is treated with a three-fold excess of an aqueous solution of the appropriate metal hydroxide, acetate or carbonate. If precipitation occurs, the product is removed by filtration and recrystallized. Otherwise, the aqueous layer is separated and evaporated to dryness. The residue is treated with ethyl acetate and filtered. Removal of the ethyl acetate affords the product which may be recrystallized, if necessary.

By the above methods a number of compounds are prepared, these are listed in Tables appended hereto.

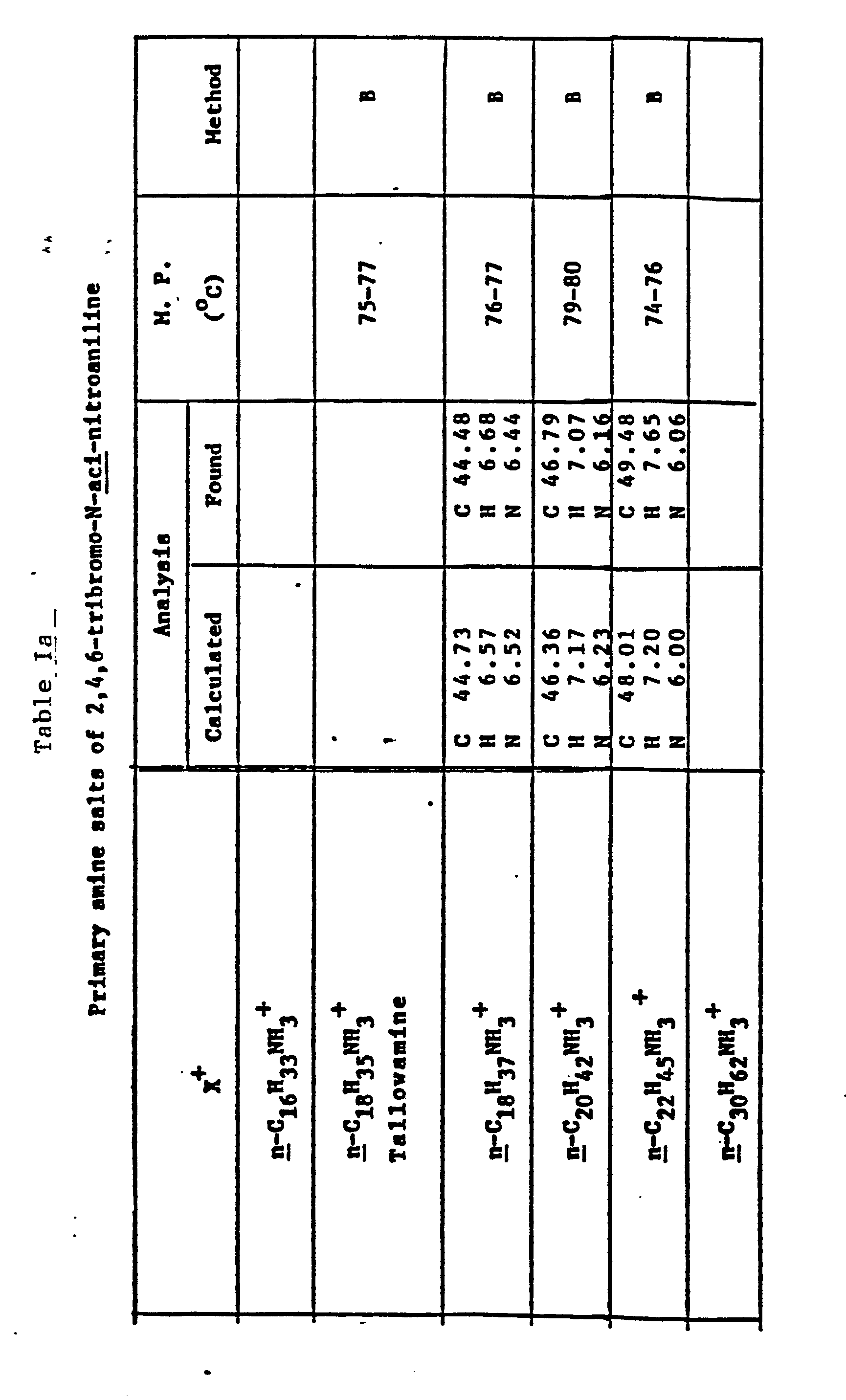

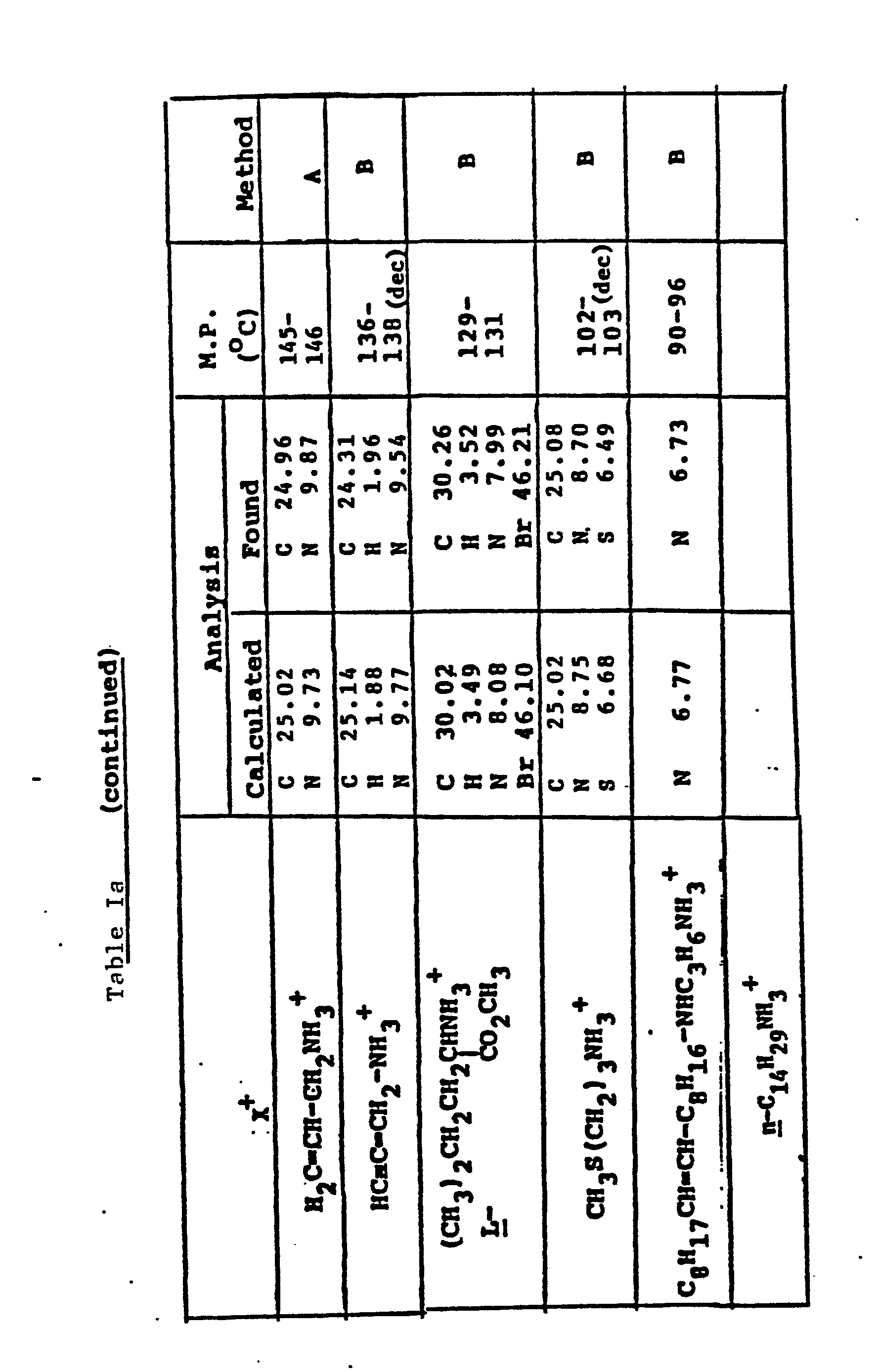

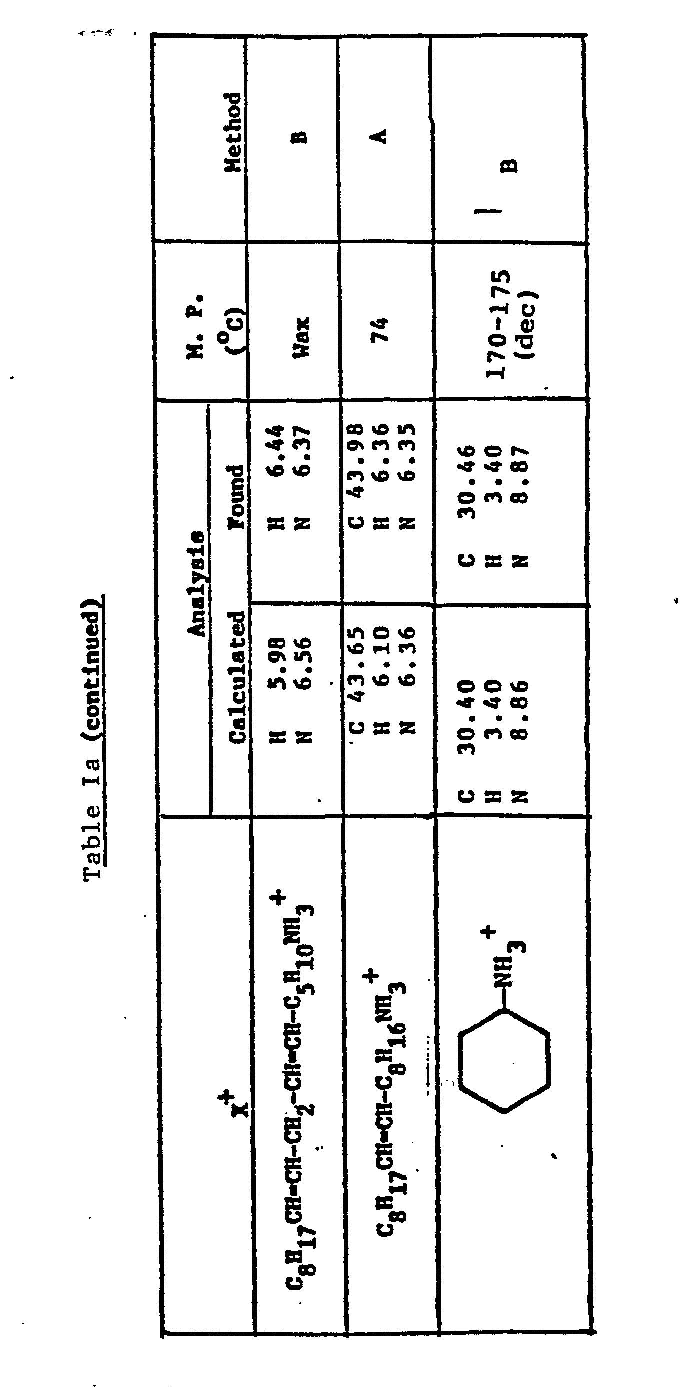

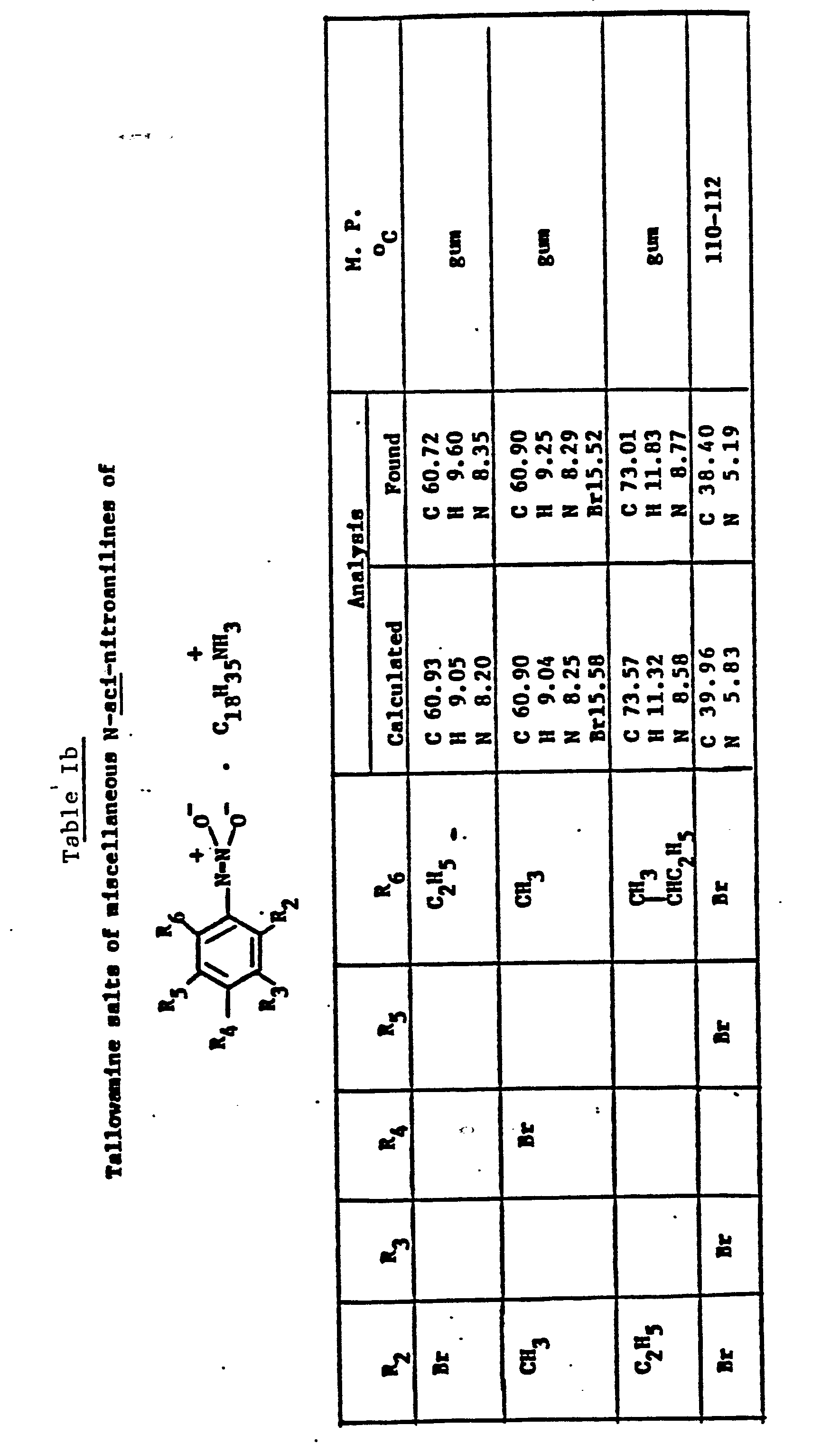

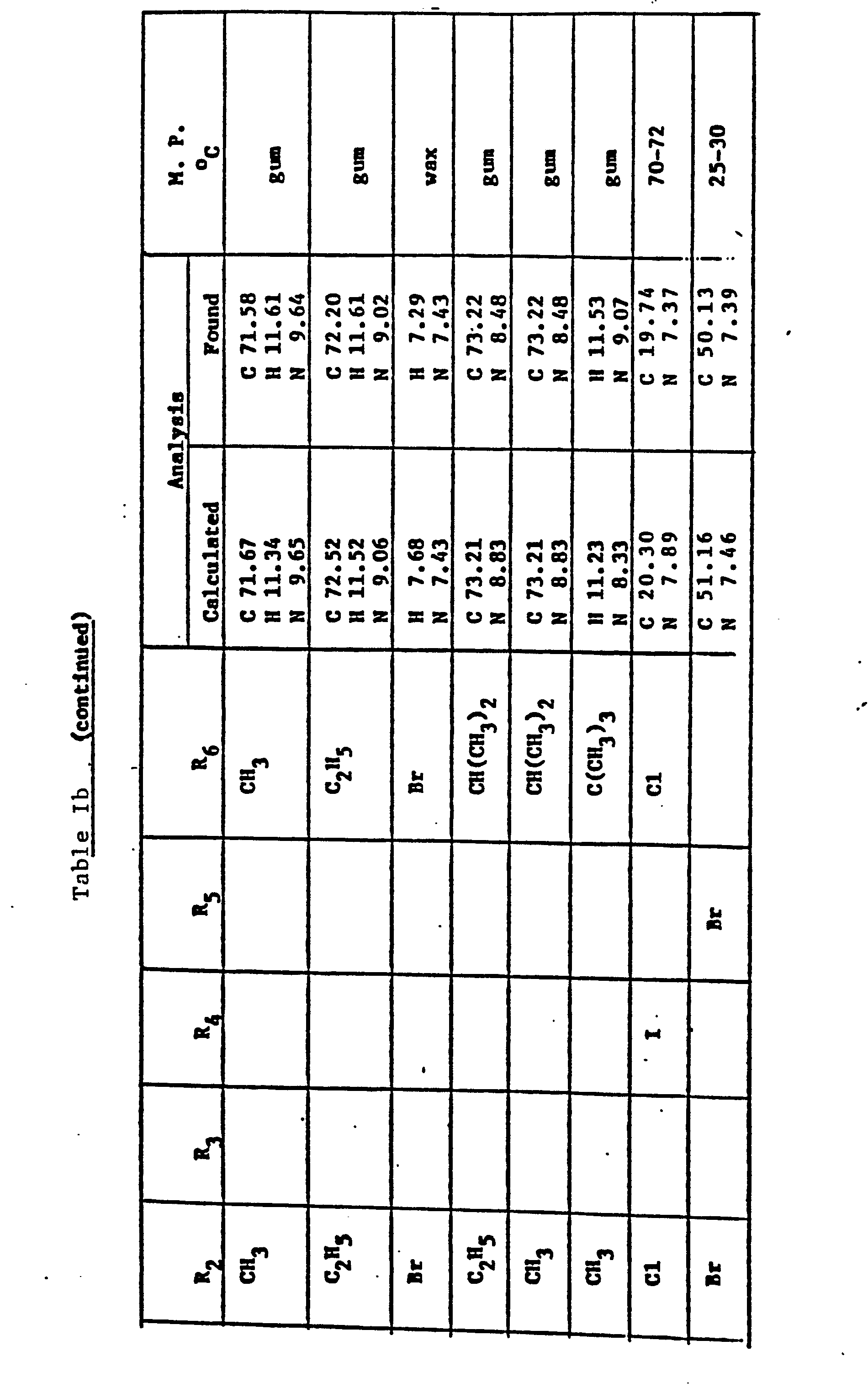

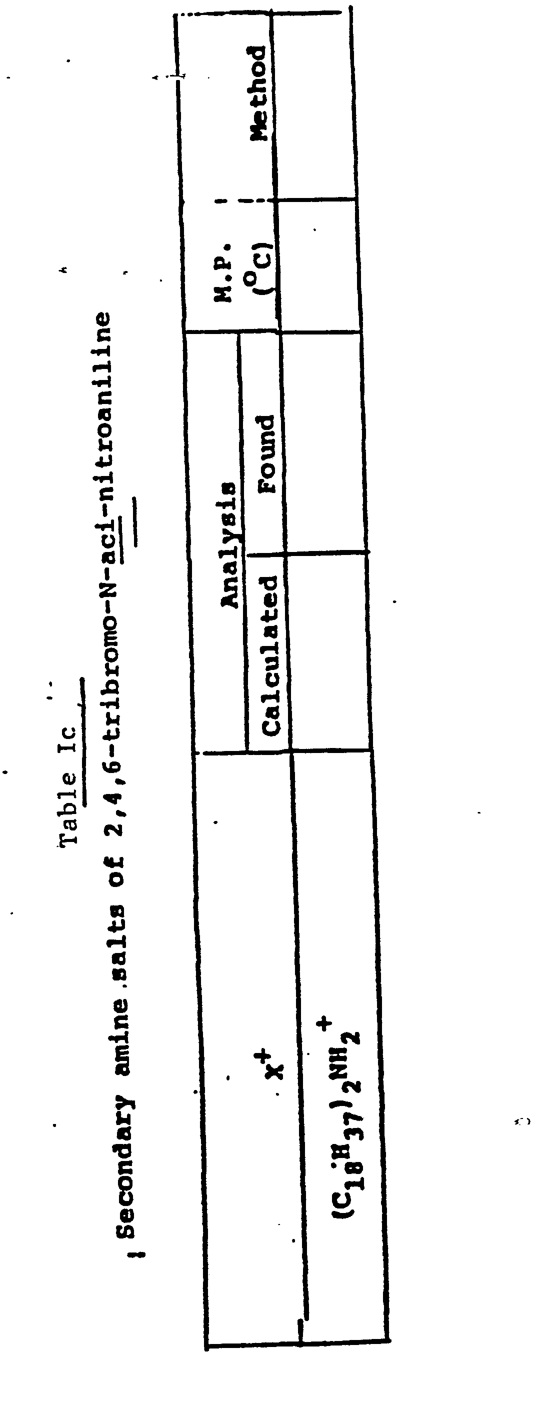

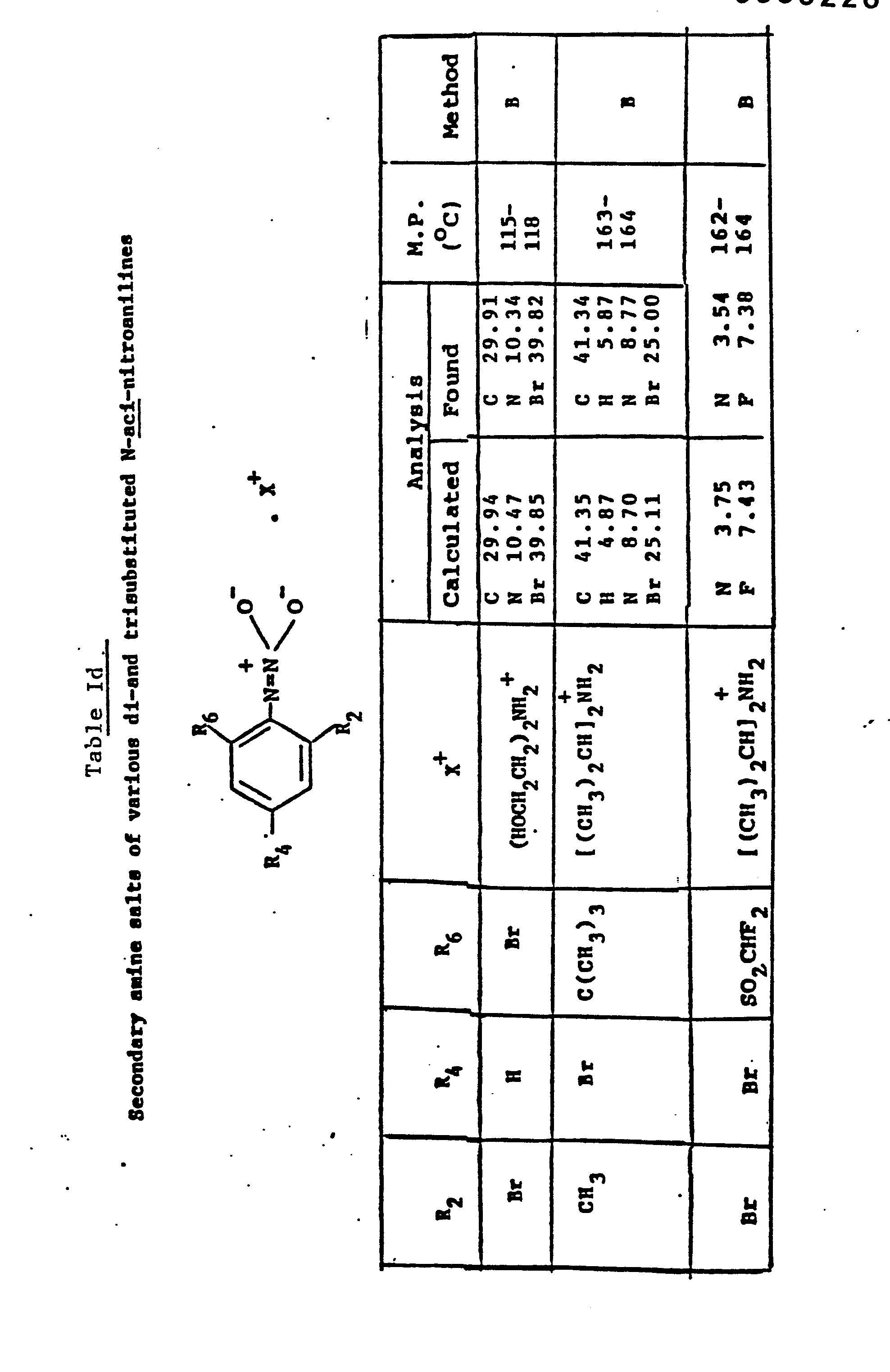

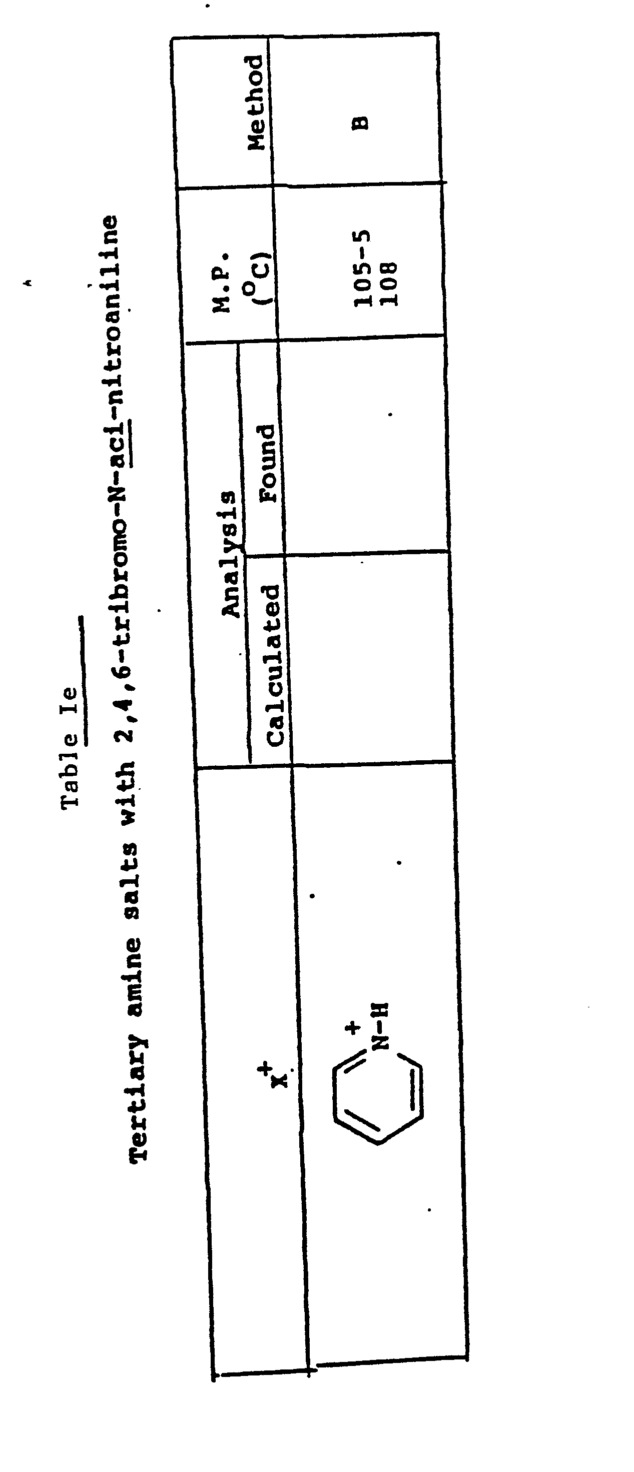

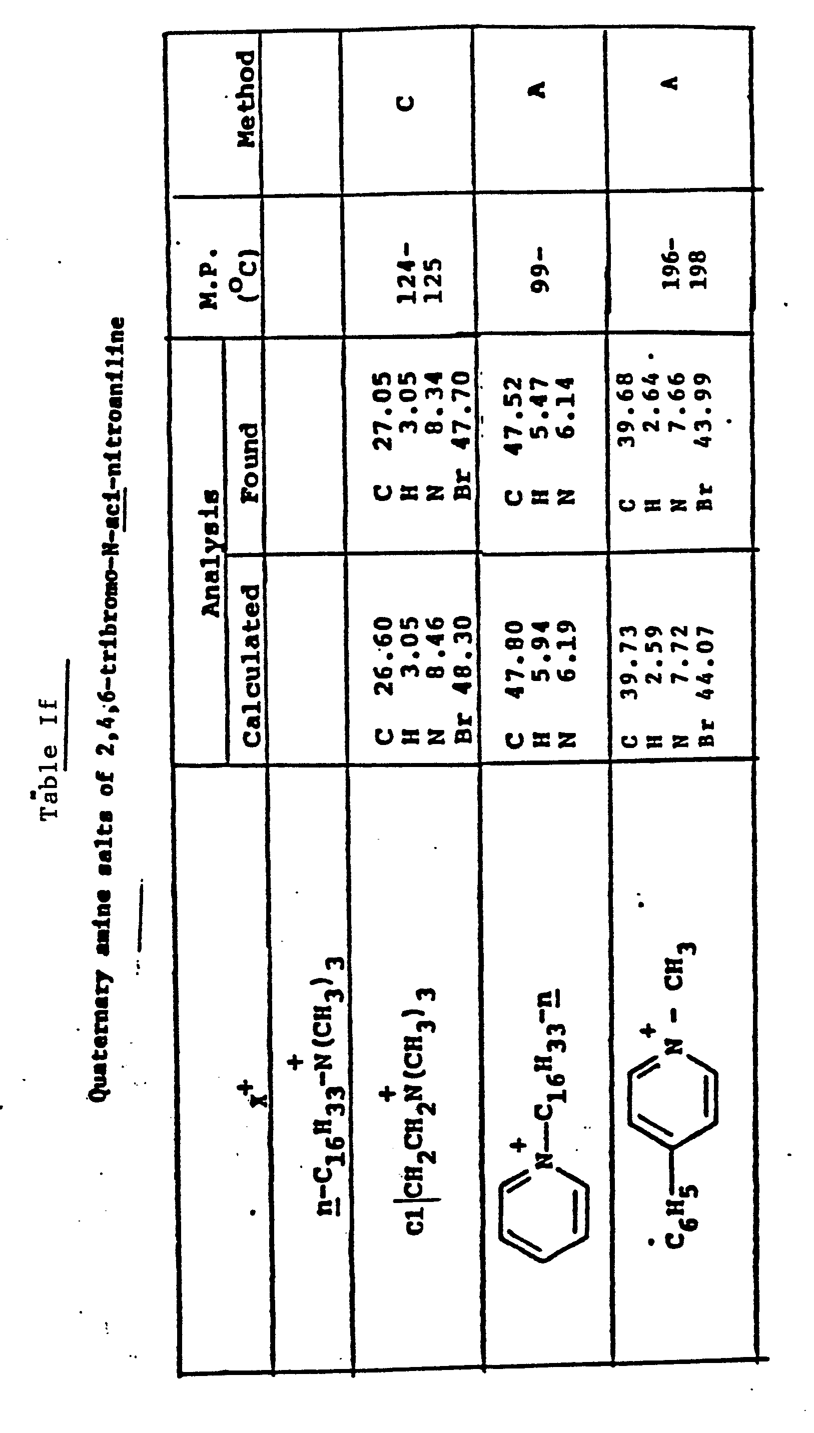

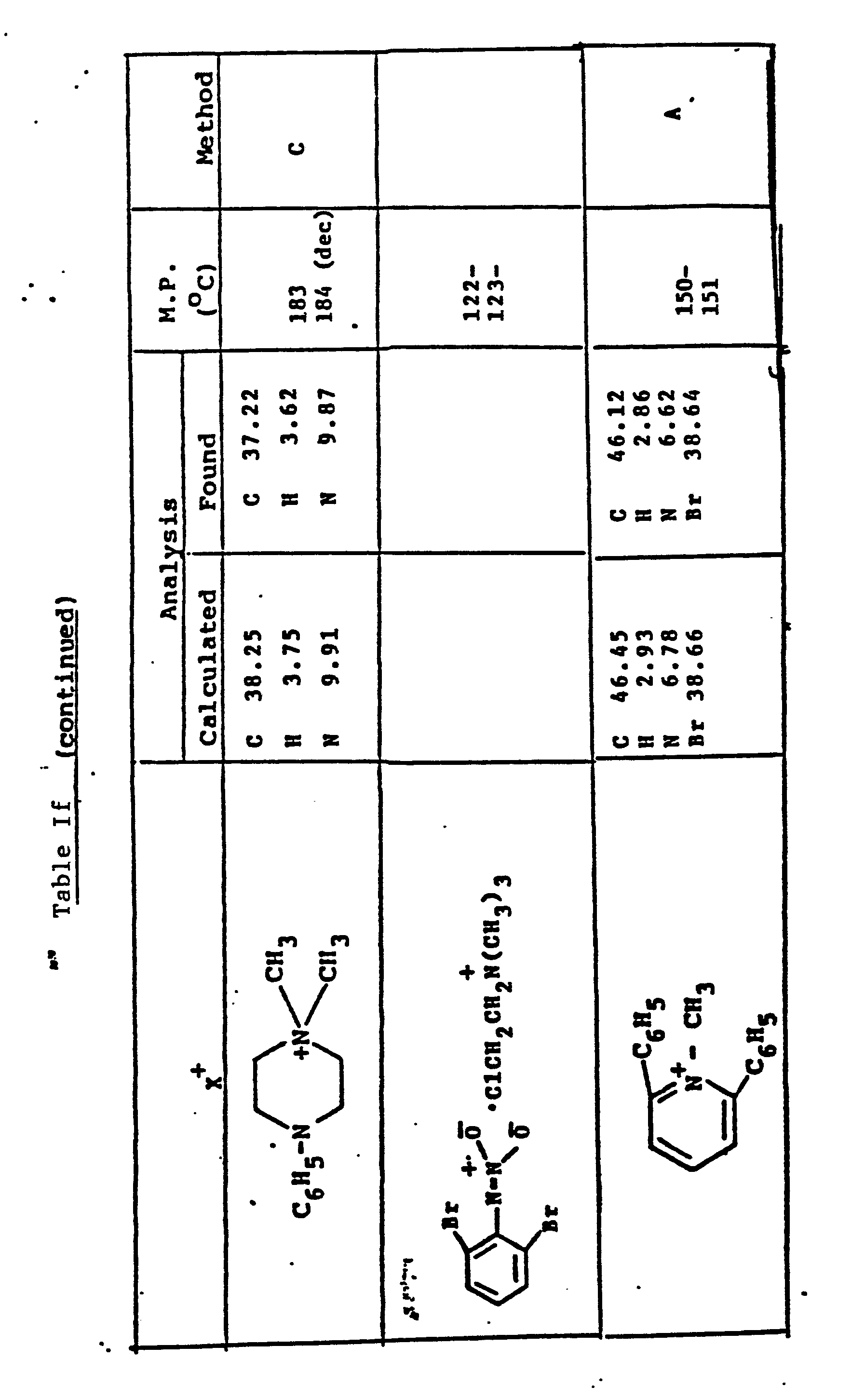

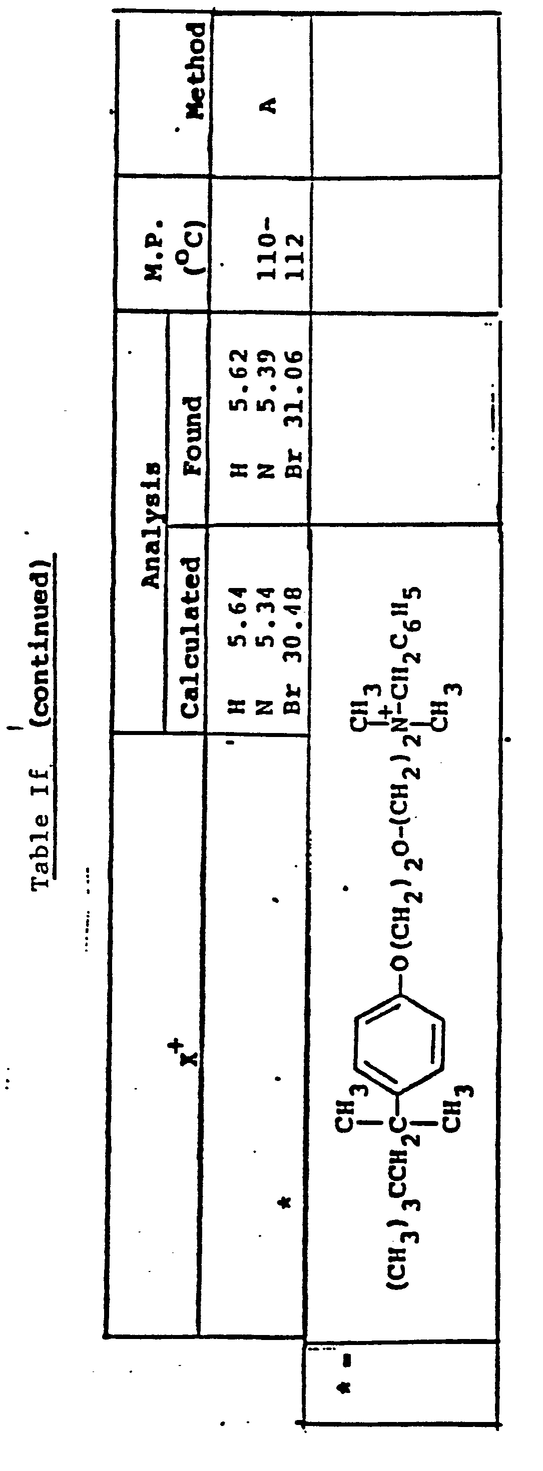

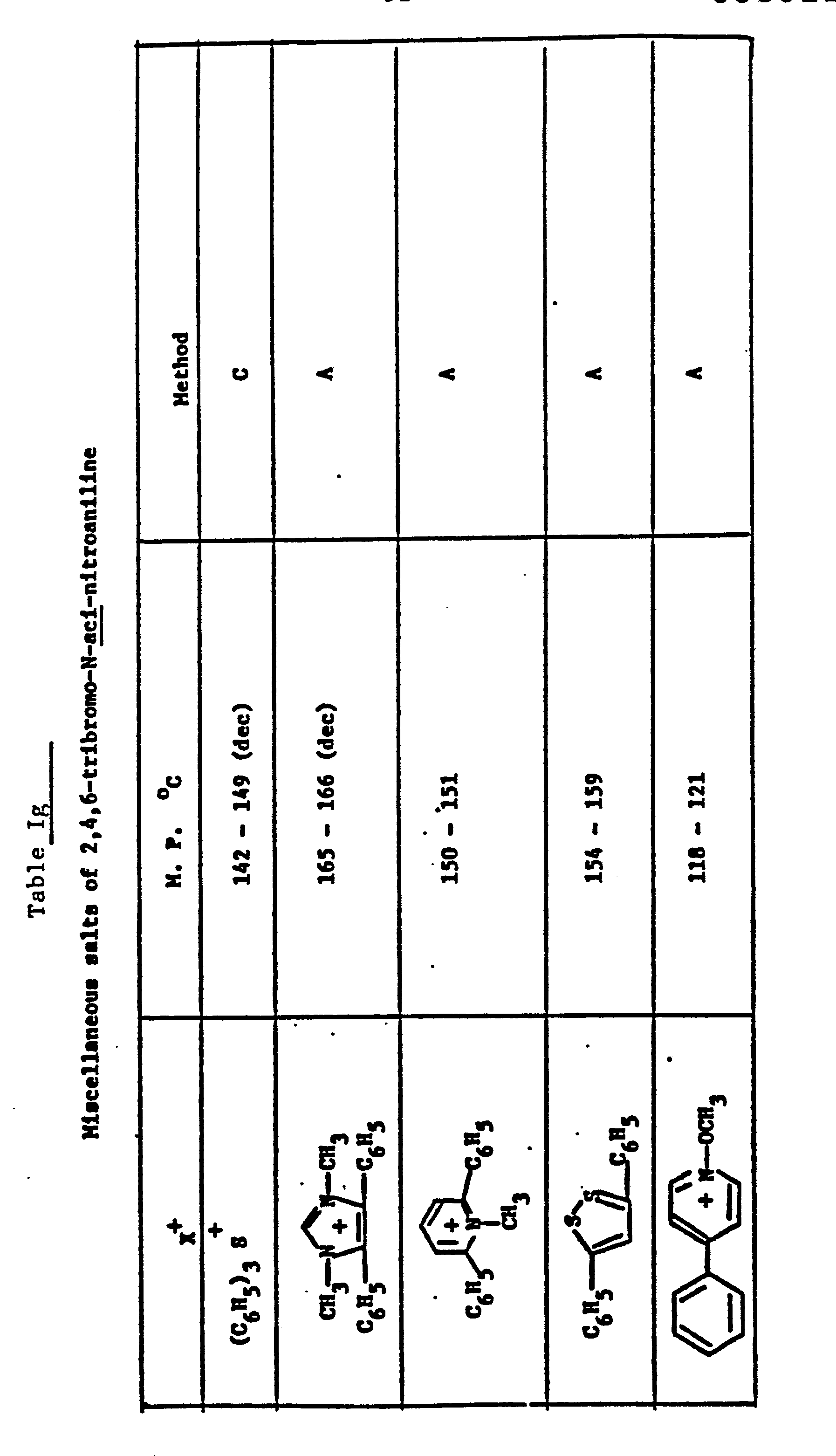

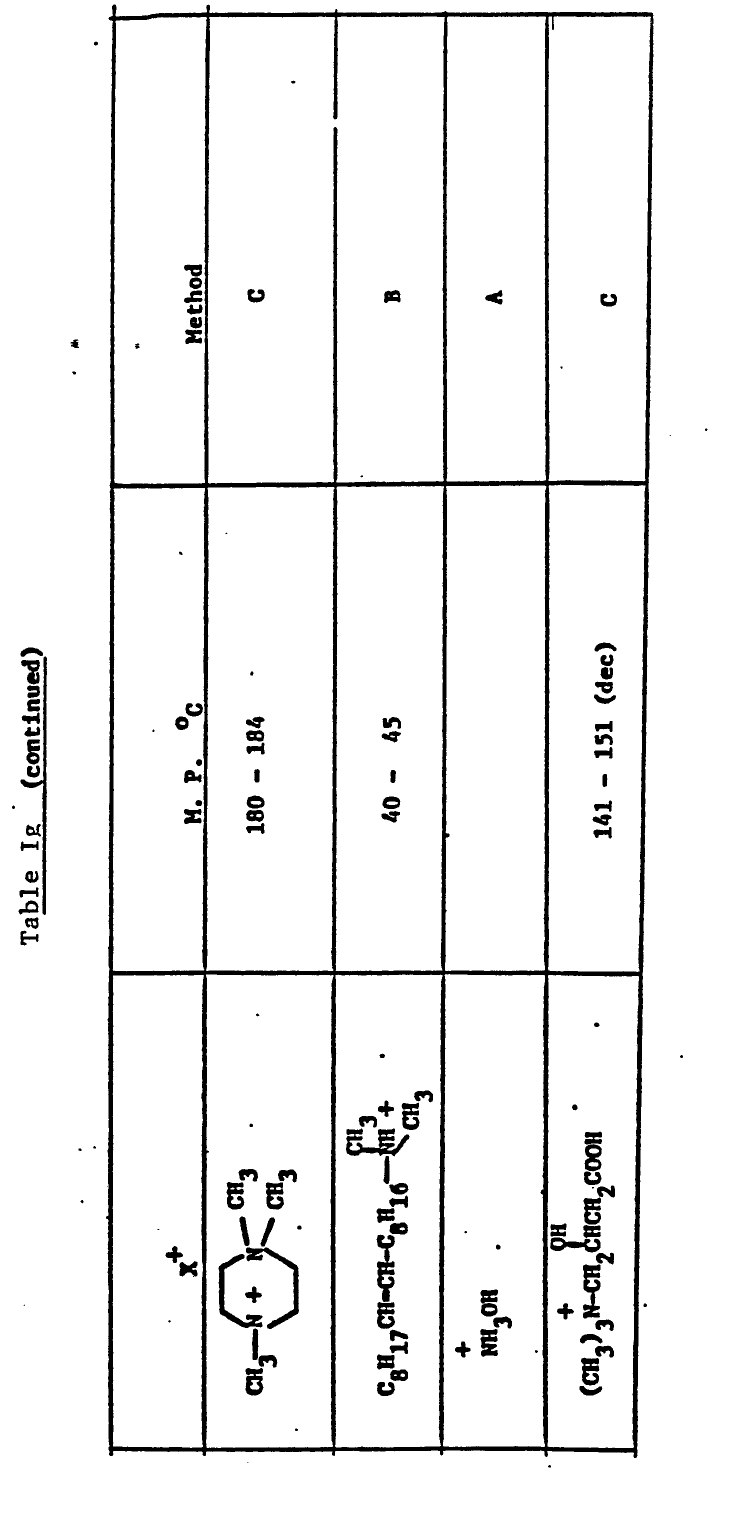

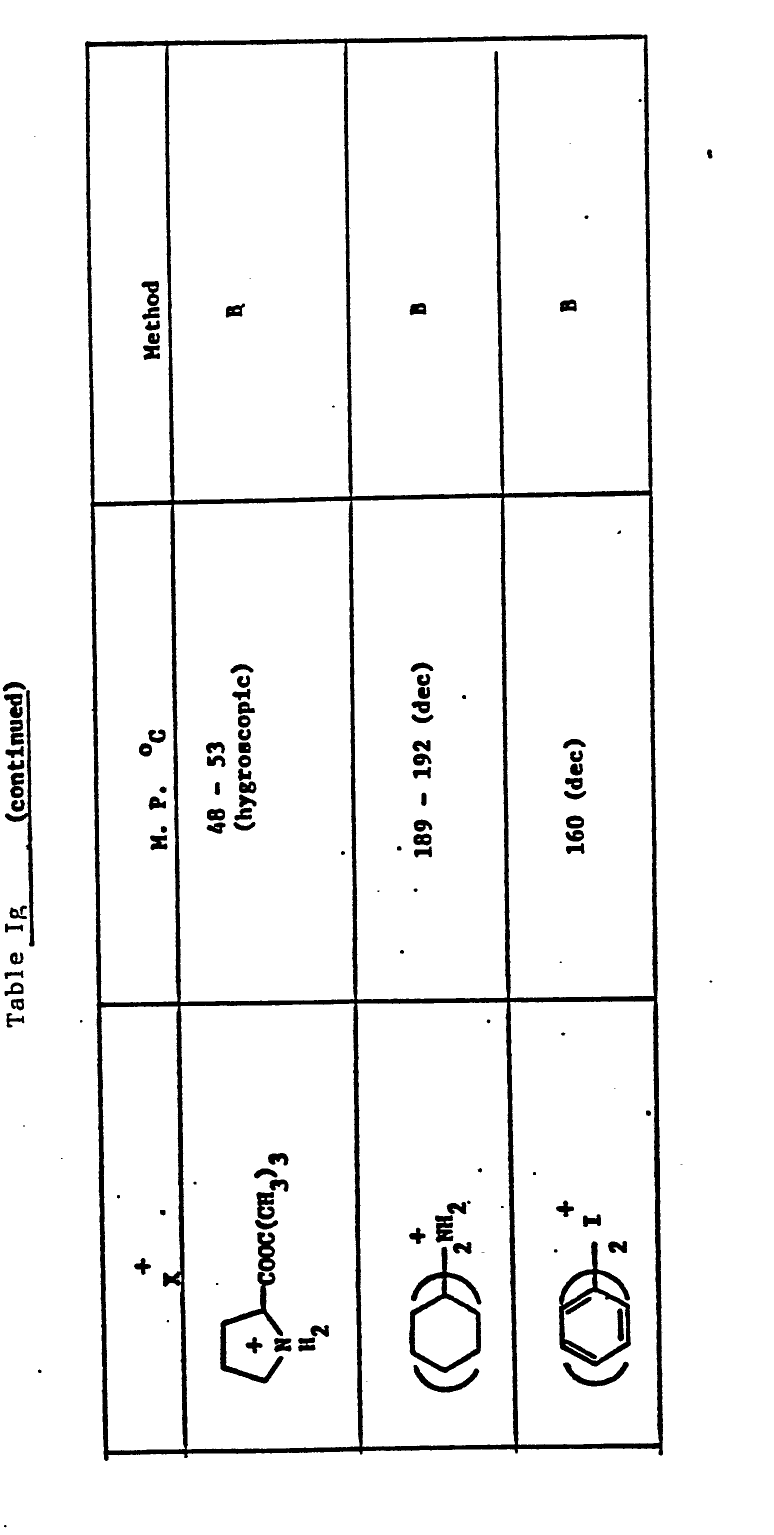

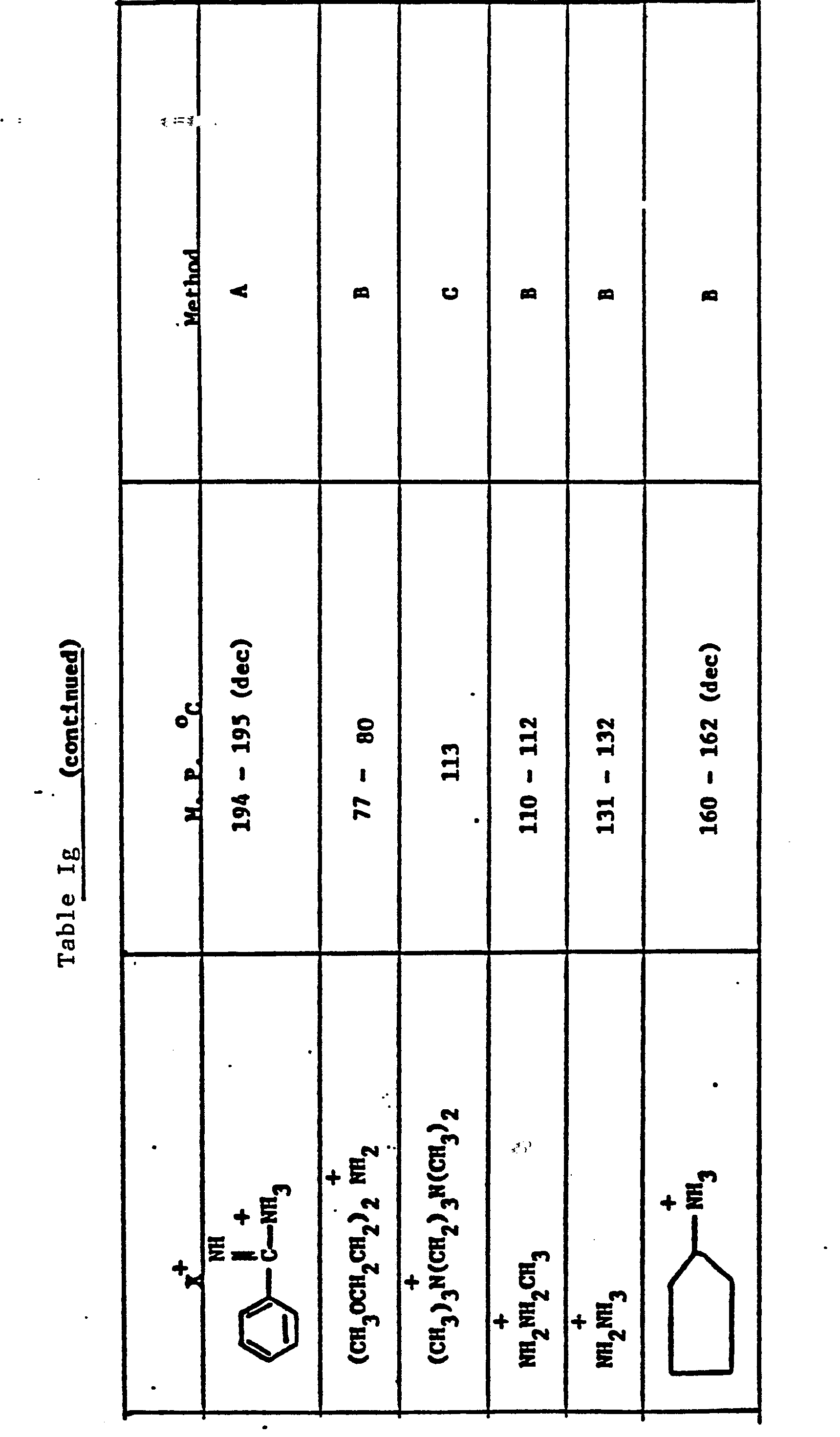

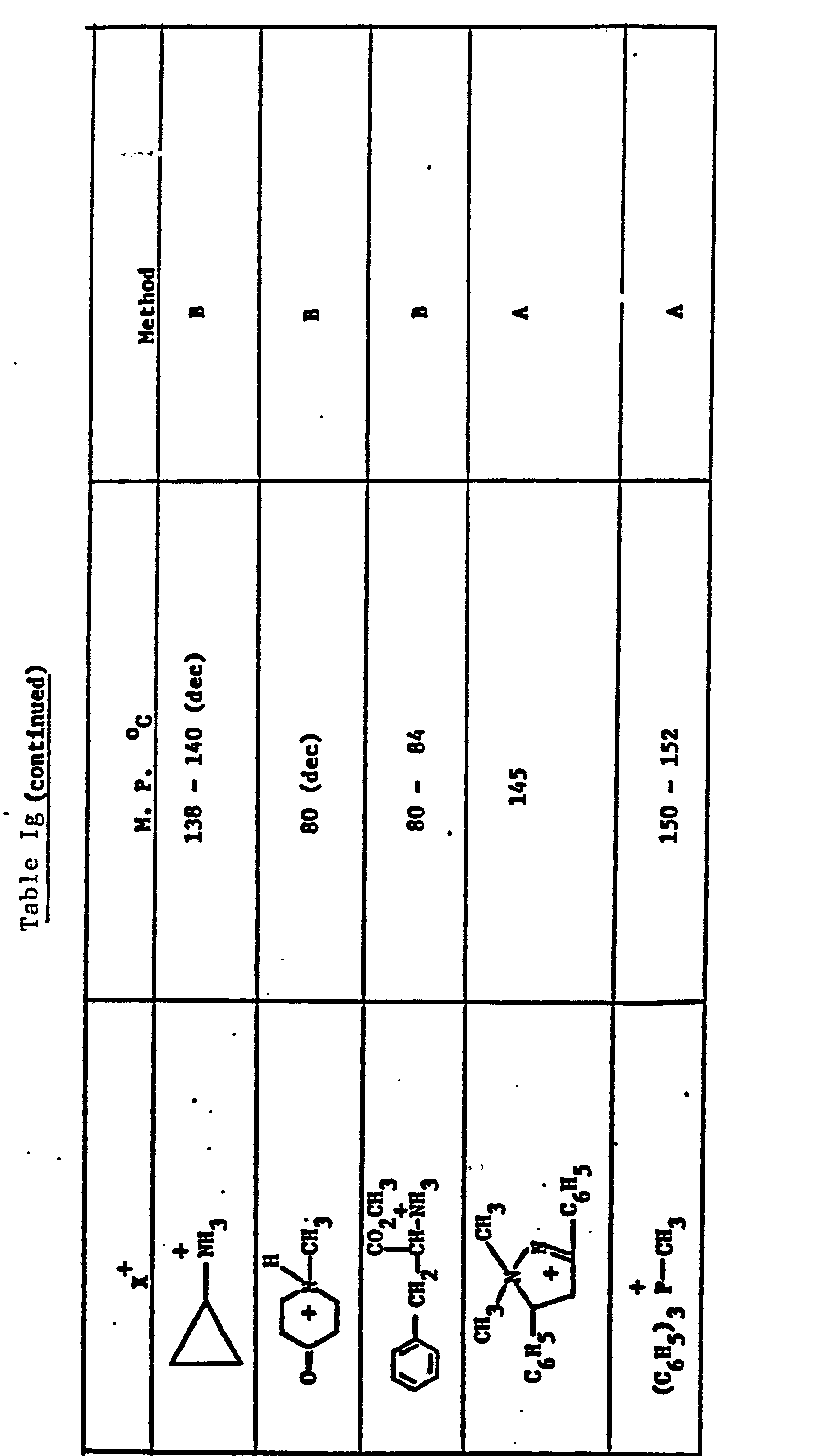

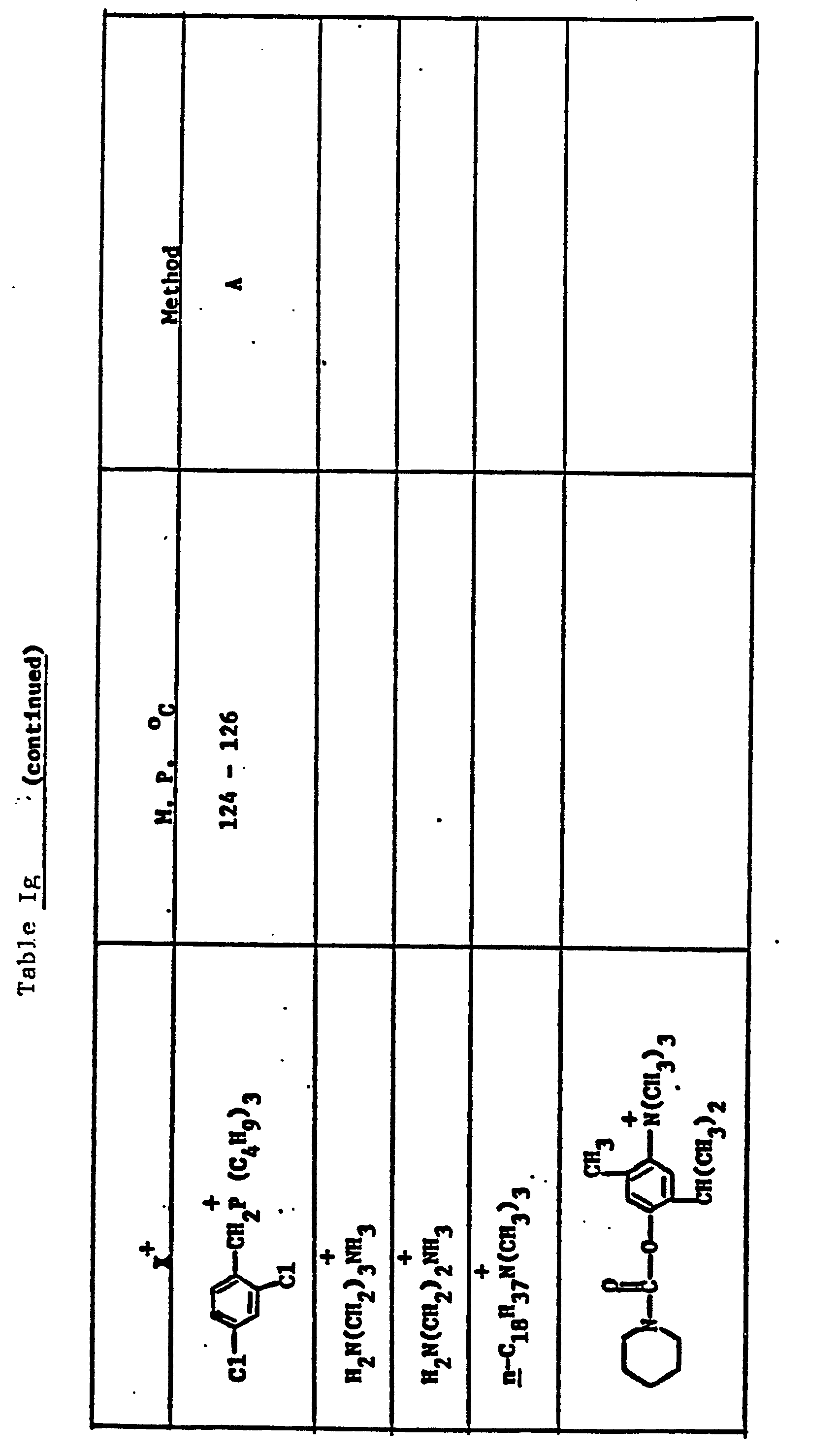

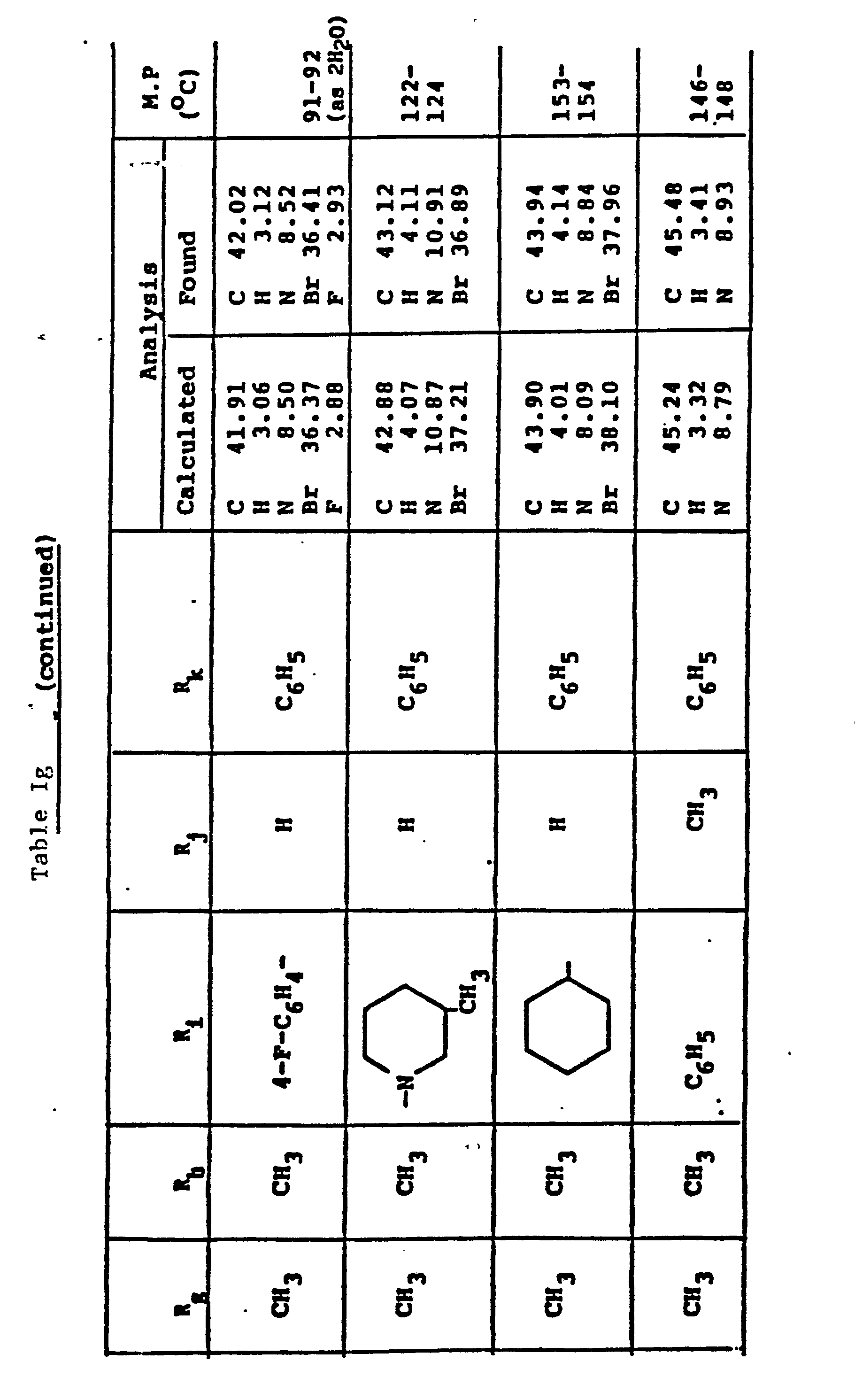

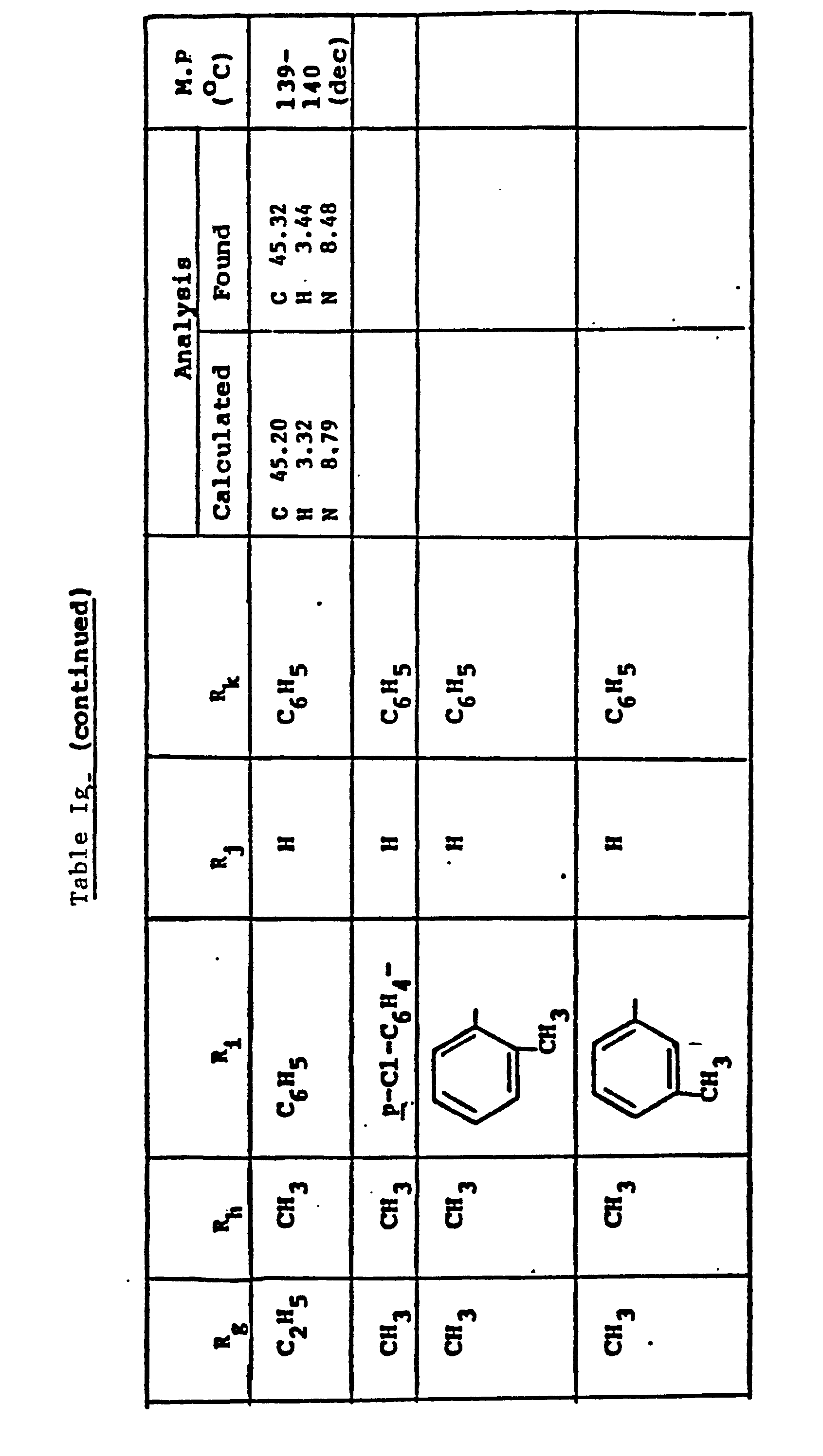

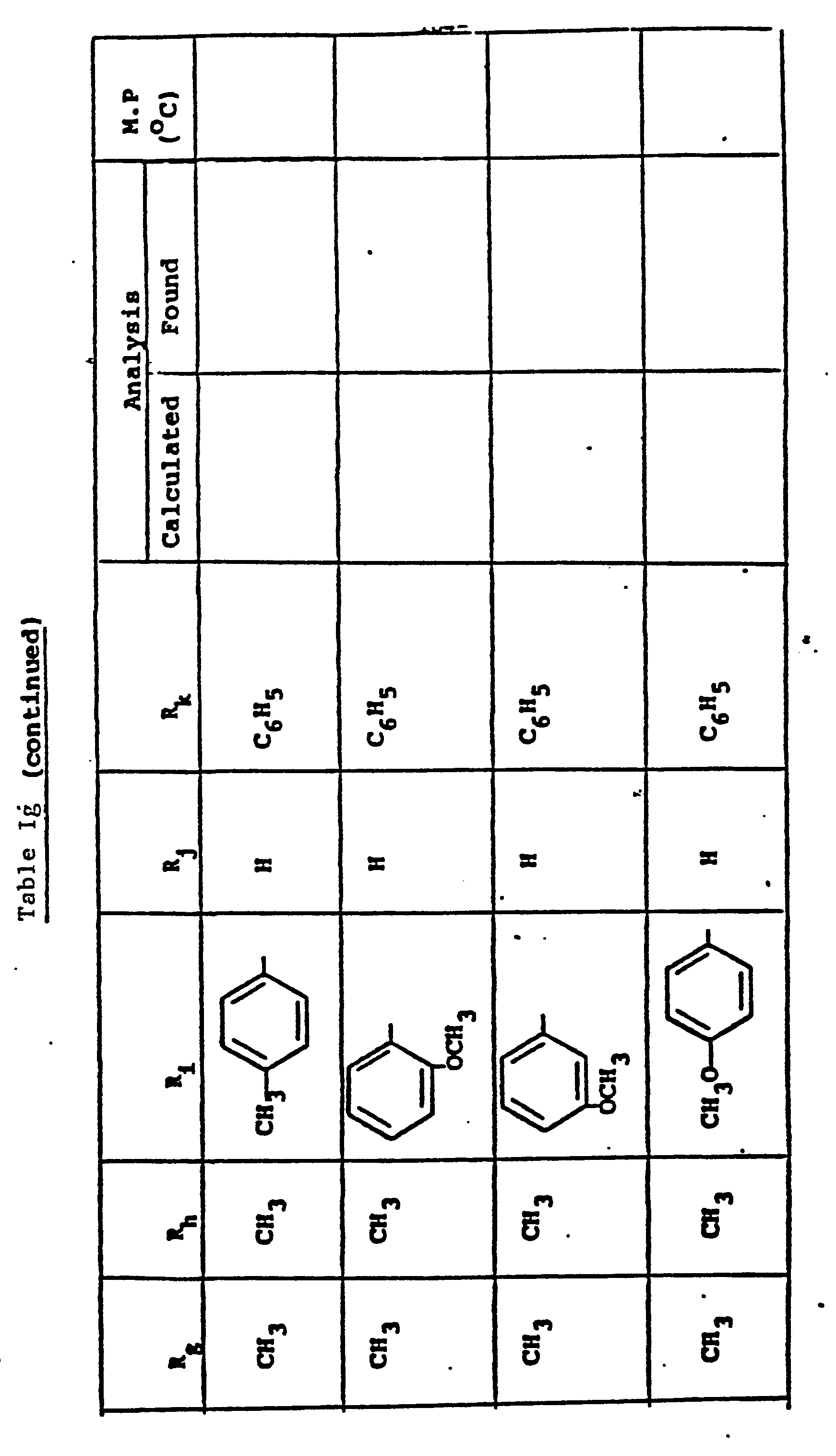

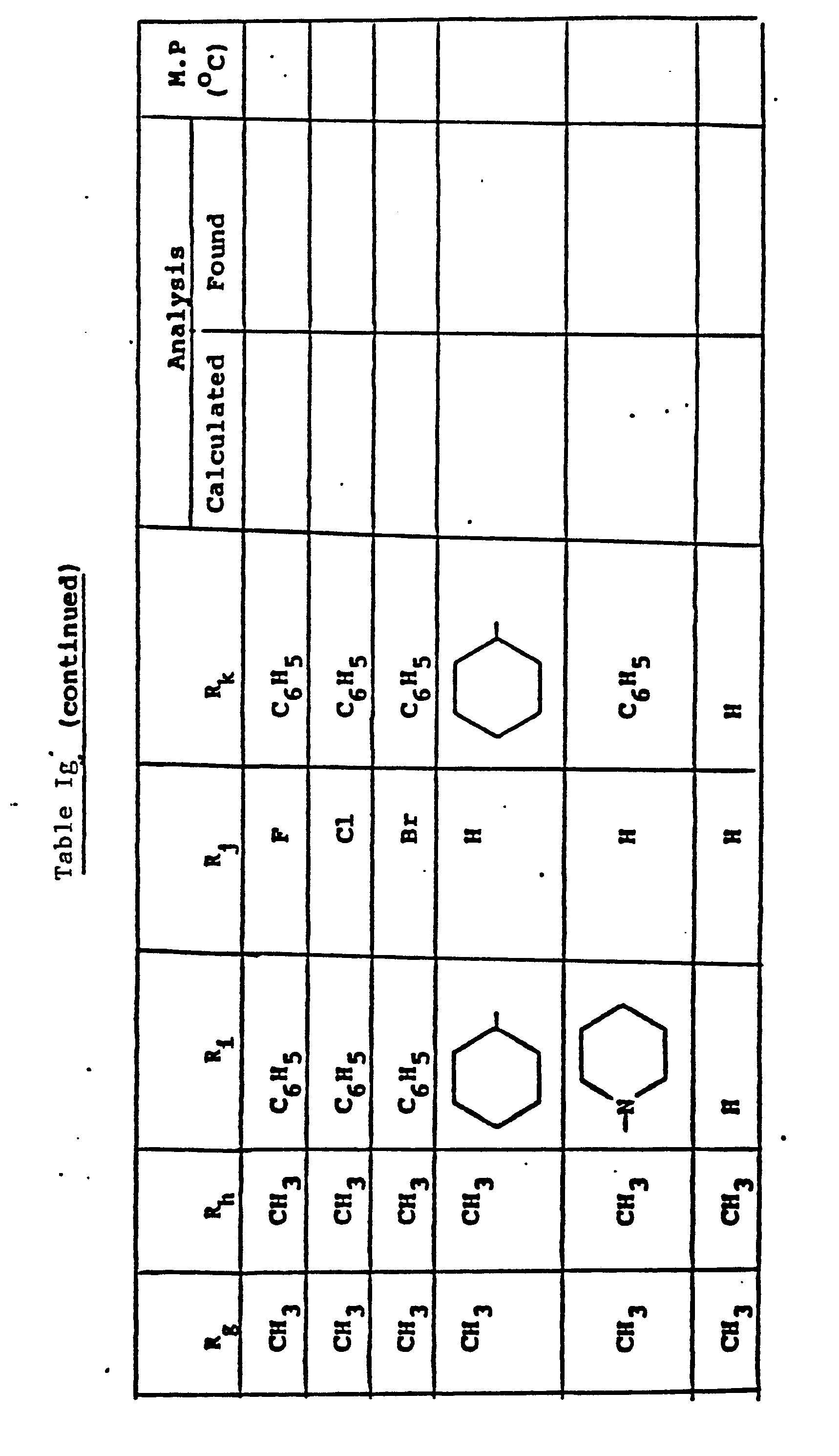

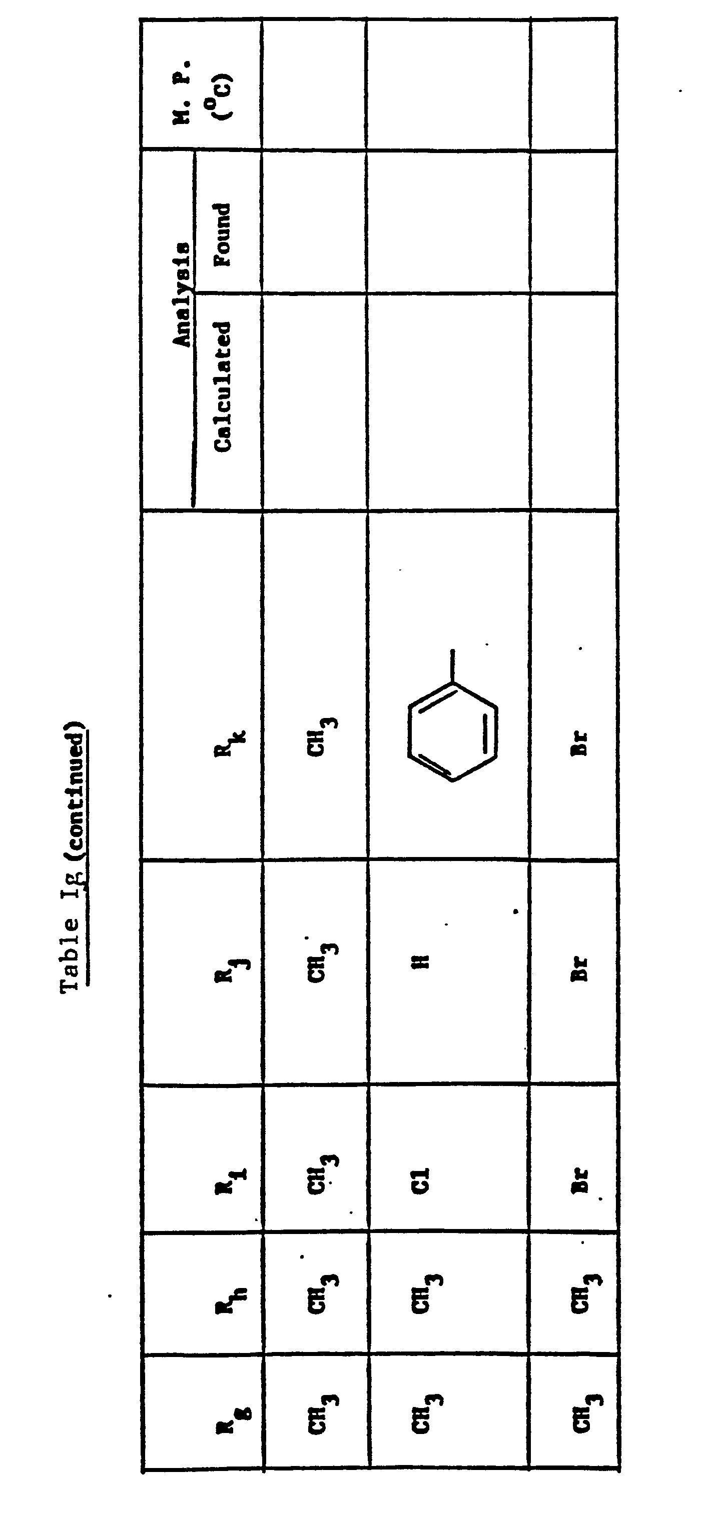

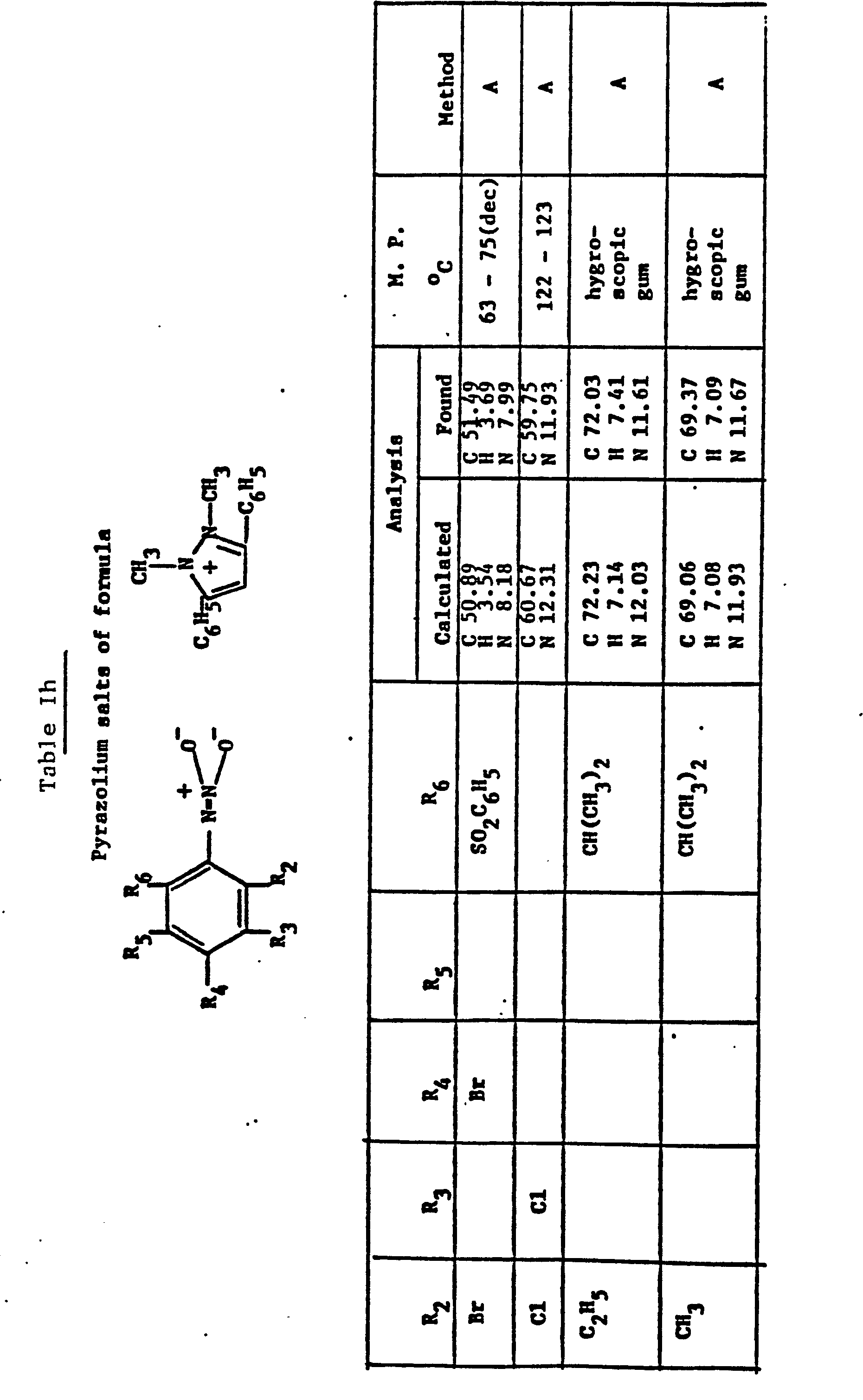

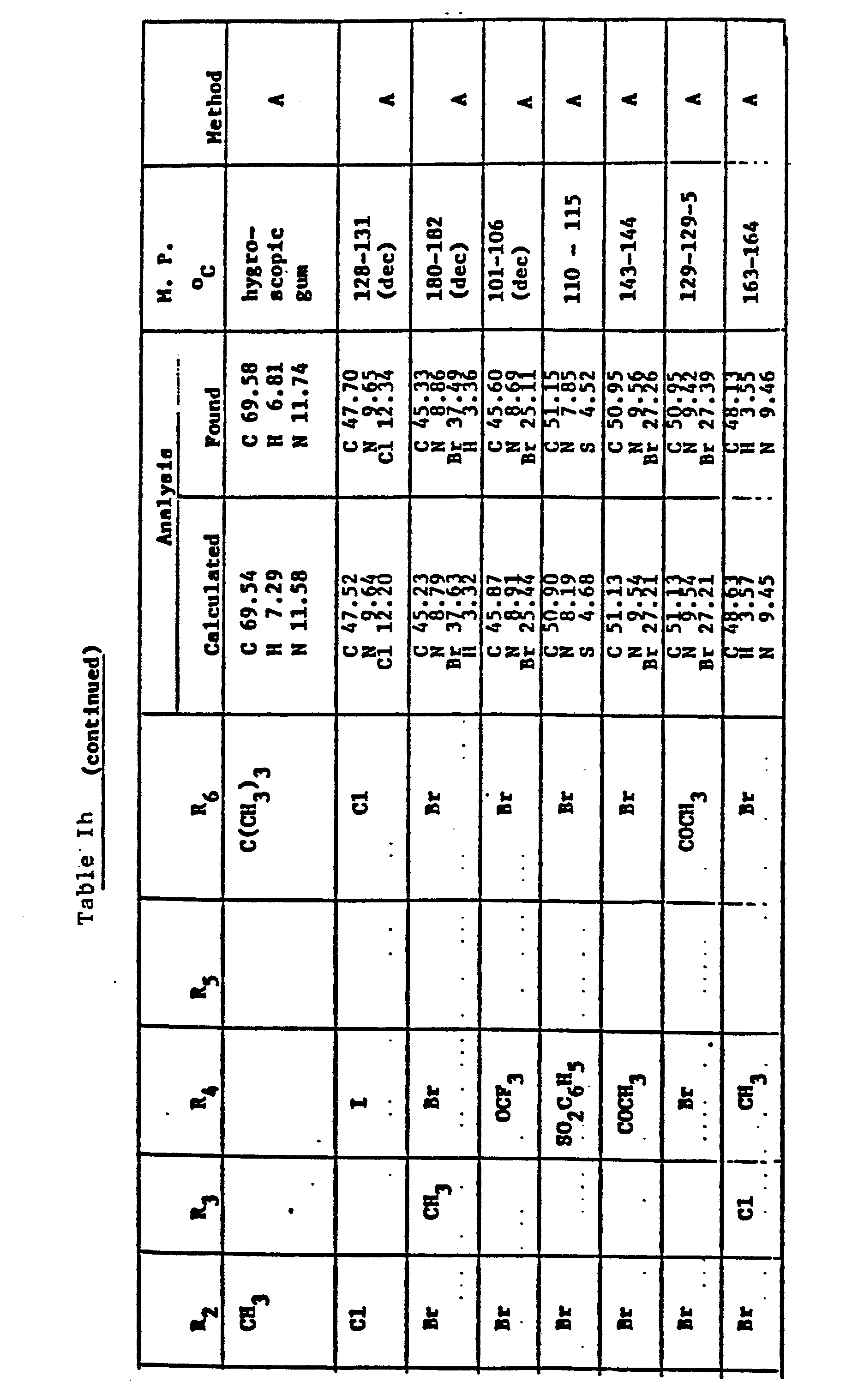

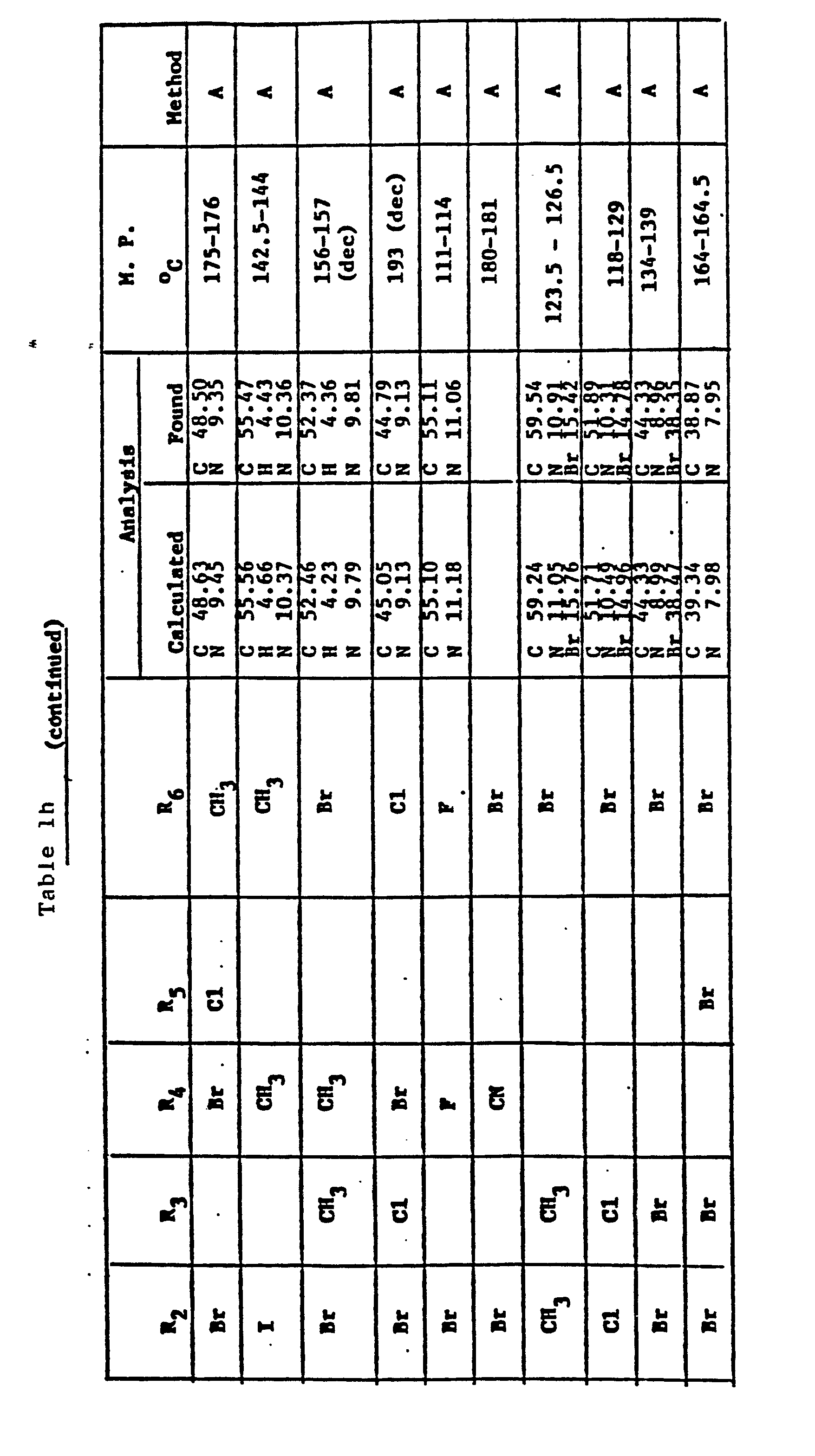

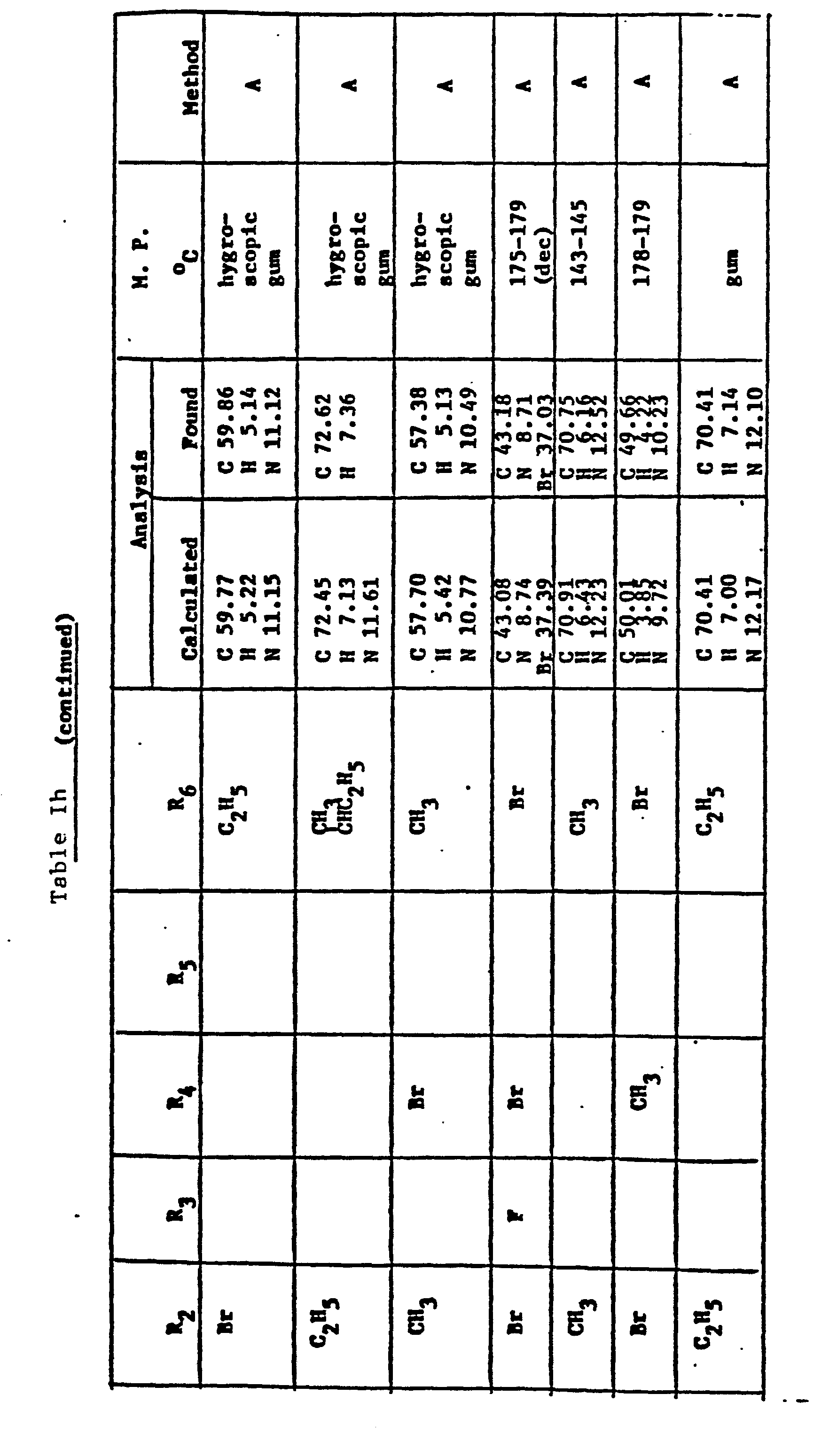

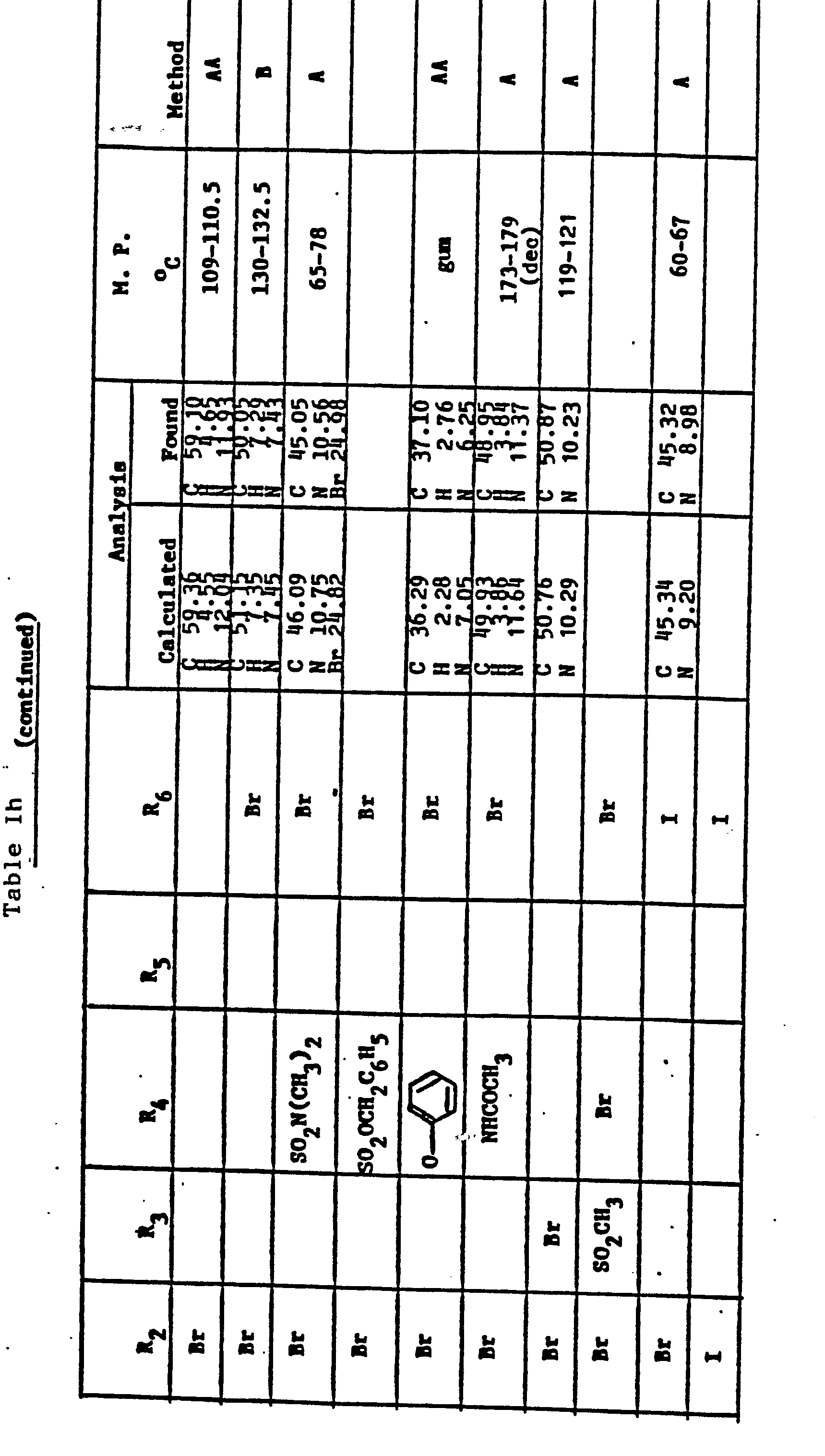

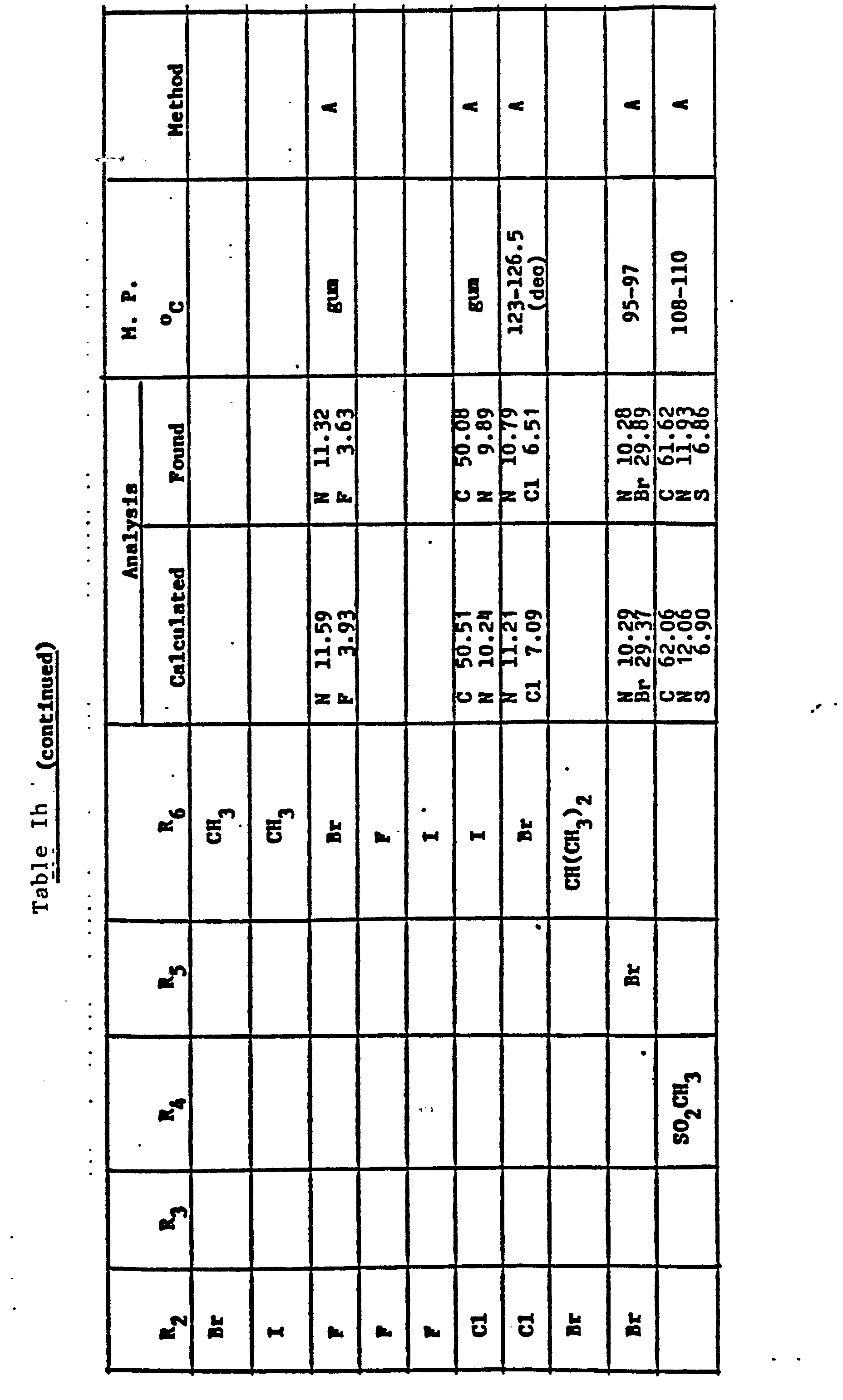

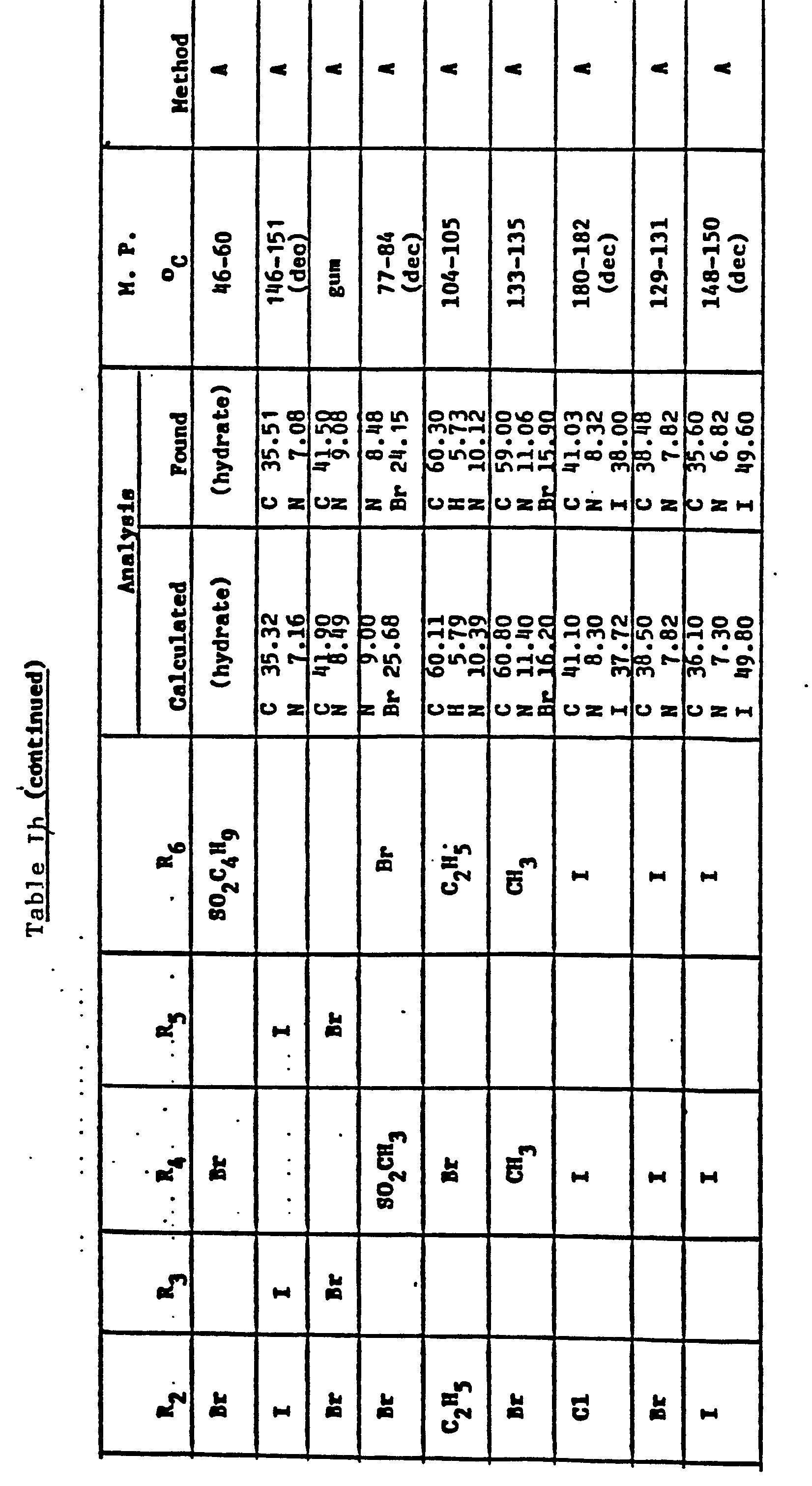

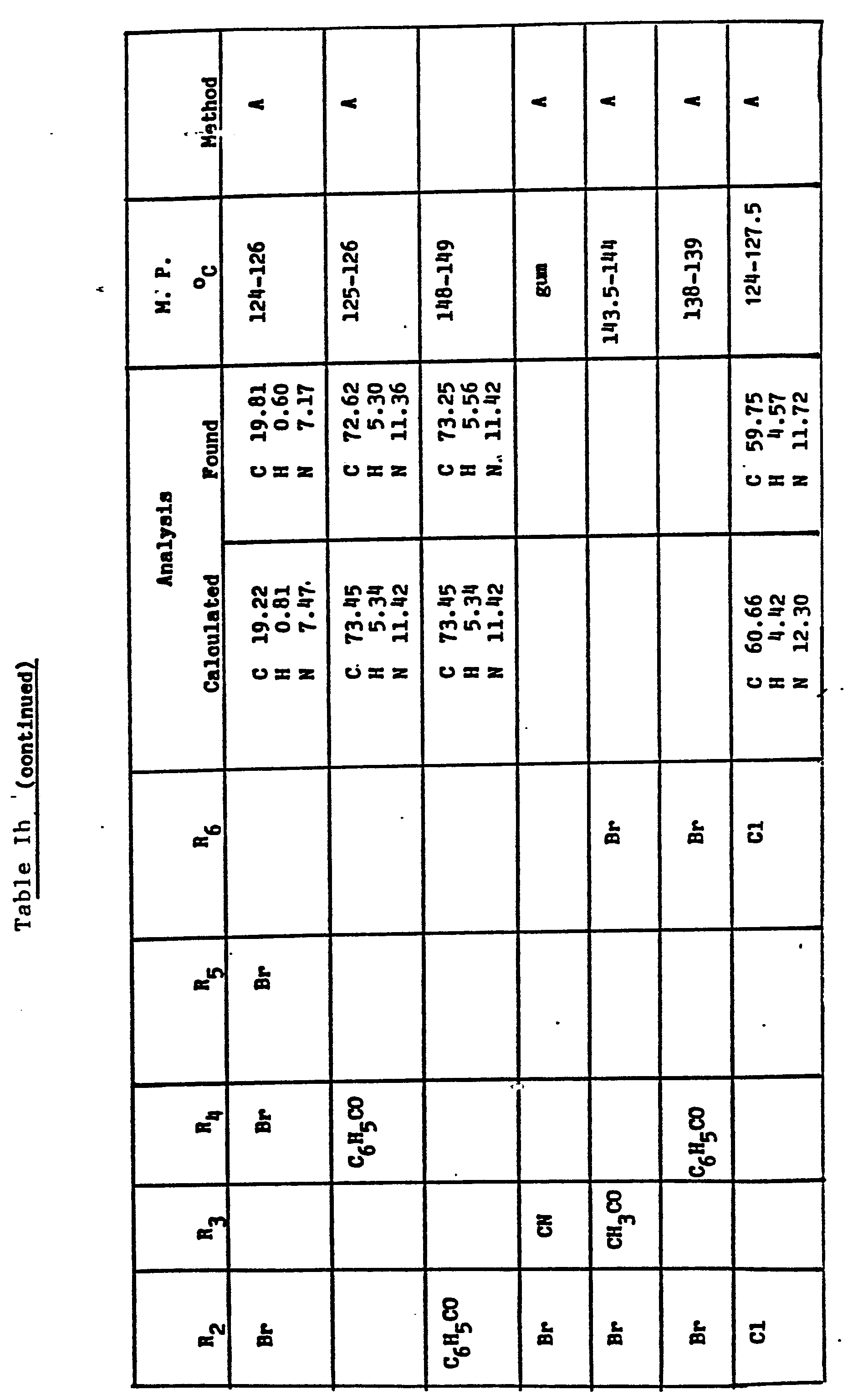

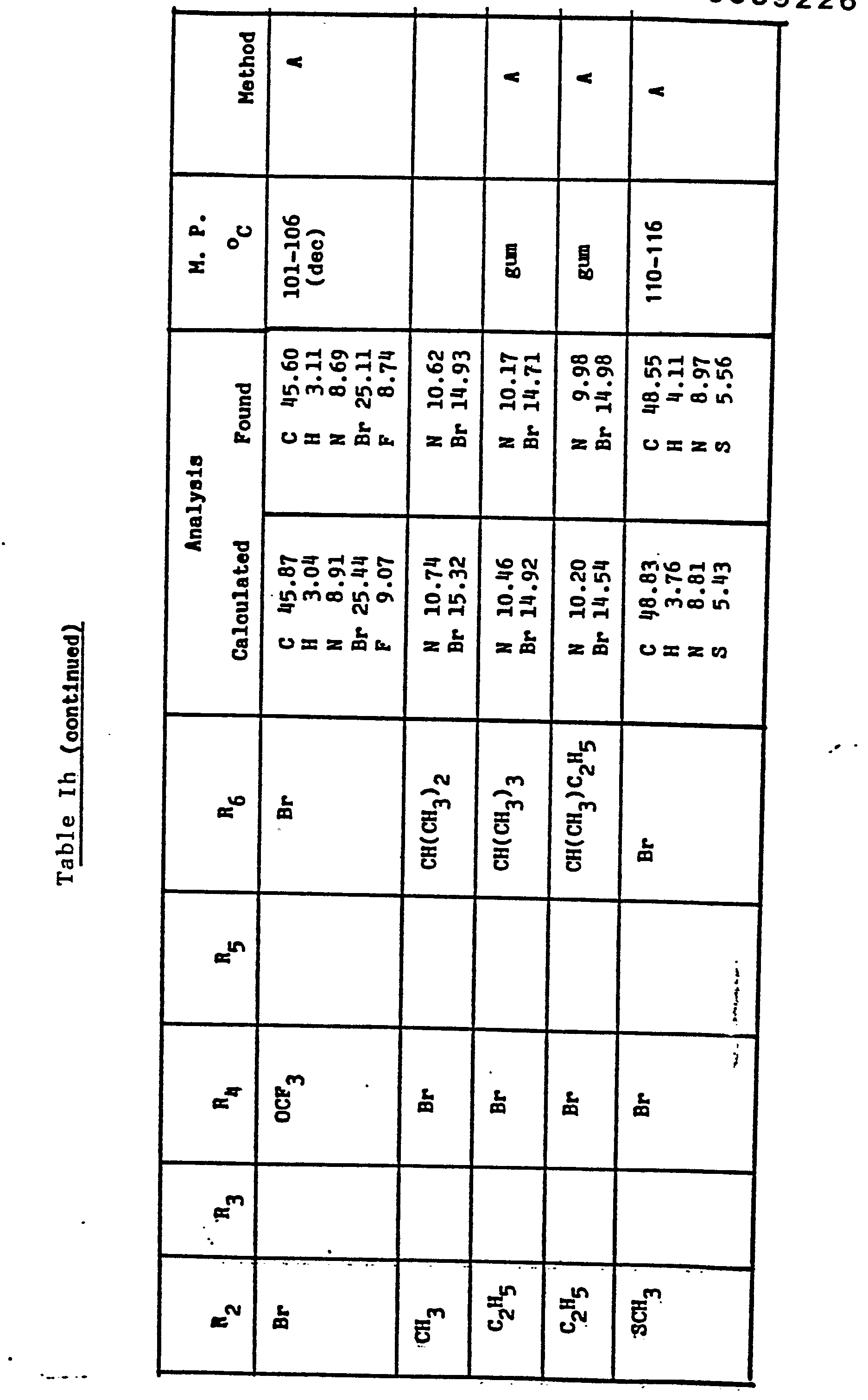

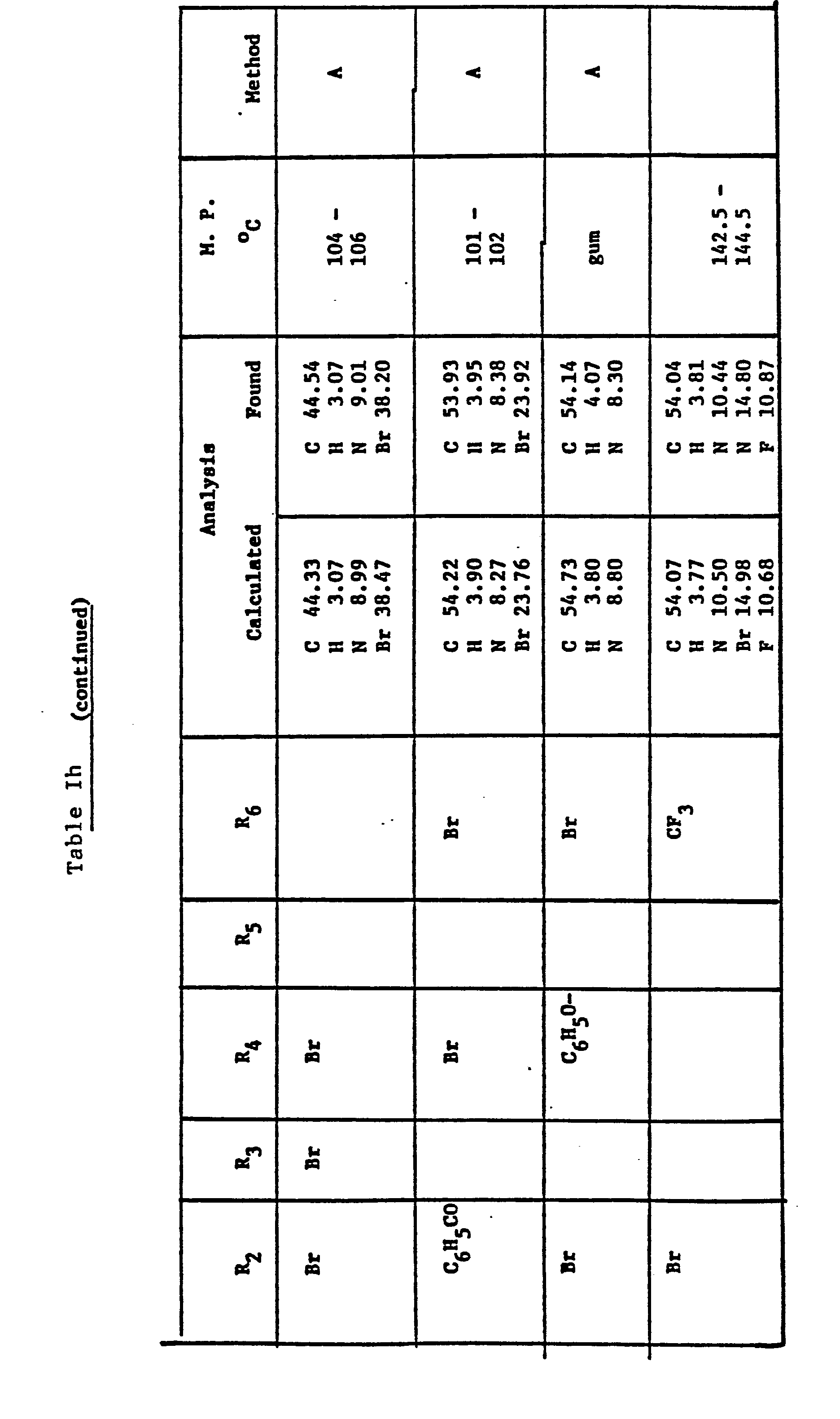

### Example 2

### Preparation of 2,4,6-tribromo-N-nitro-N-(2-propynyl) aniline

2,4,6-tribromo-N-nitroaniline (5.0g; 0.01334 mol) is reacted with a methanolic solution of sodium hydroxide (0.53g; 0.01324 mol). in the presence of methanol (60 ml). The solvent is then removed, and the residue dried. The thus obtained sodium salt is dissolved in dimethyl sulfoxide (DMSO) and reacted with 3-bromopropyne (1.52g; 0.0147 mol) at 25°C. The reaction mixture is stirred 16 hours and then poured into water. The title product is extracted with ether (3 x 125 ml), the combined extract washed with water, dilute sodium hydroxide, water and brine. The ethereal solution is then dried over magnesium sulfate and the solution concentrated to afford the product, mp. 58-58,5°C.

By the above method, a number of.N-substituted phenylnitramines are prepared. These are listed in Table II below.

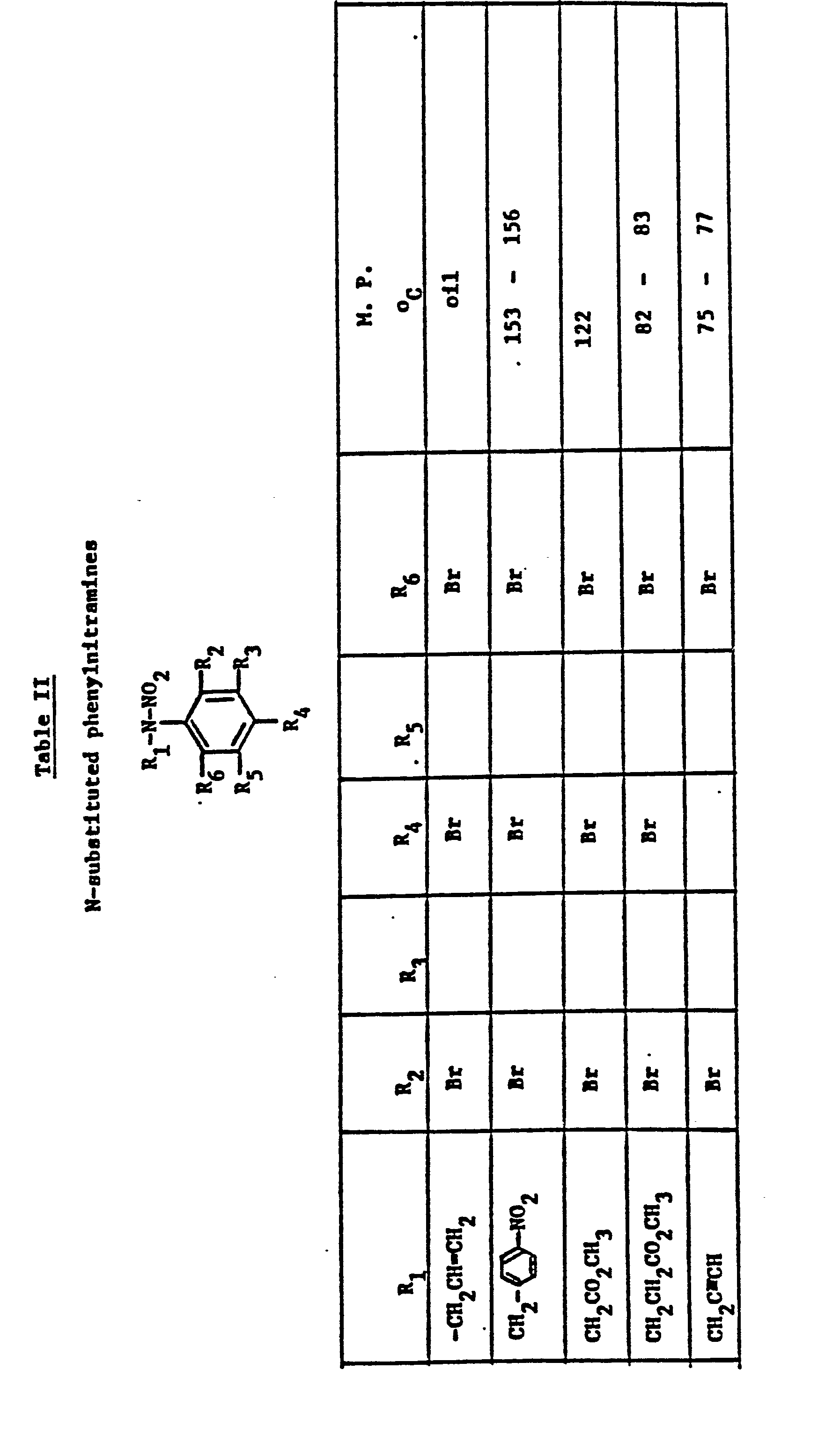

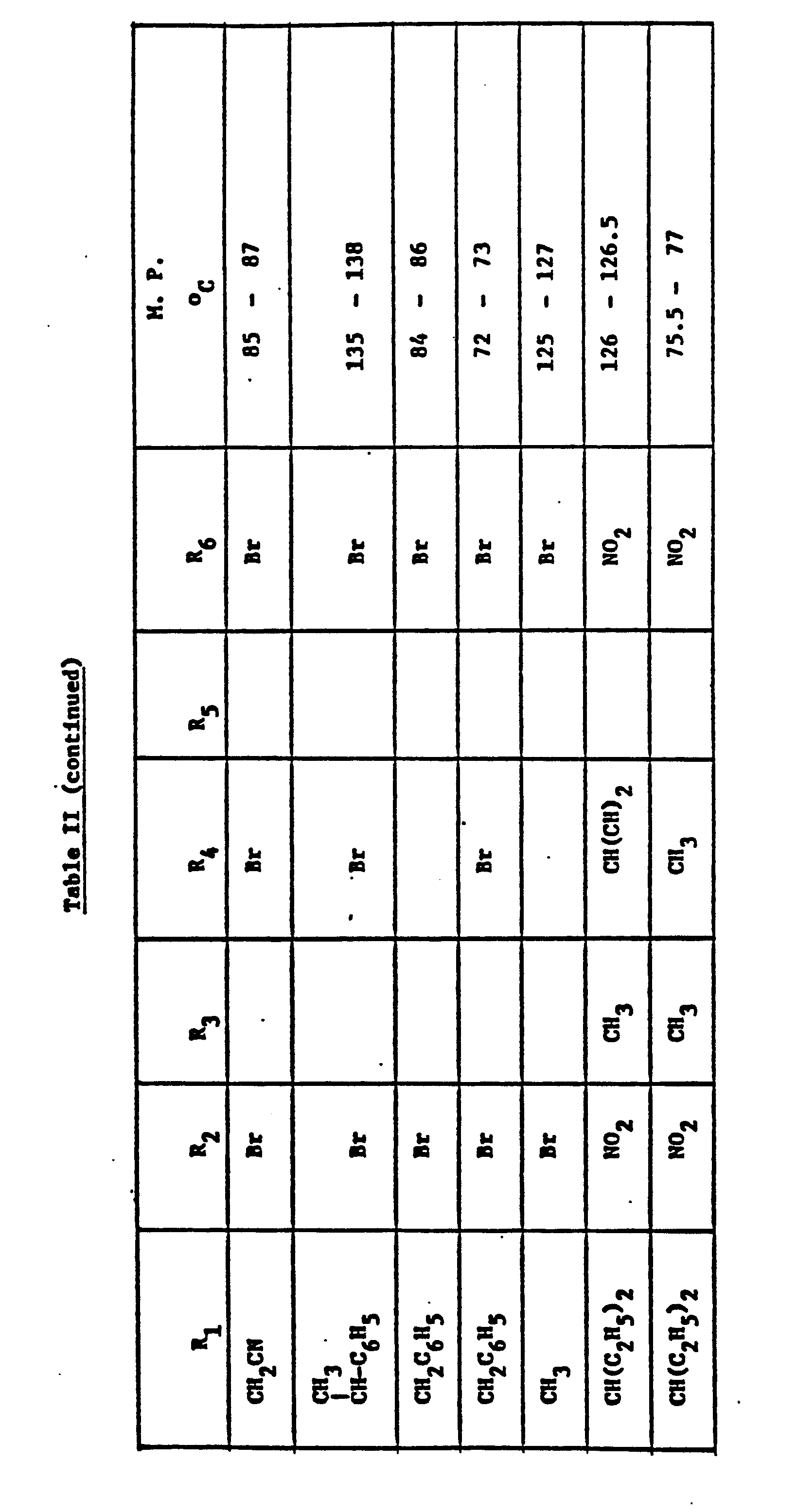

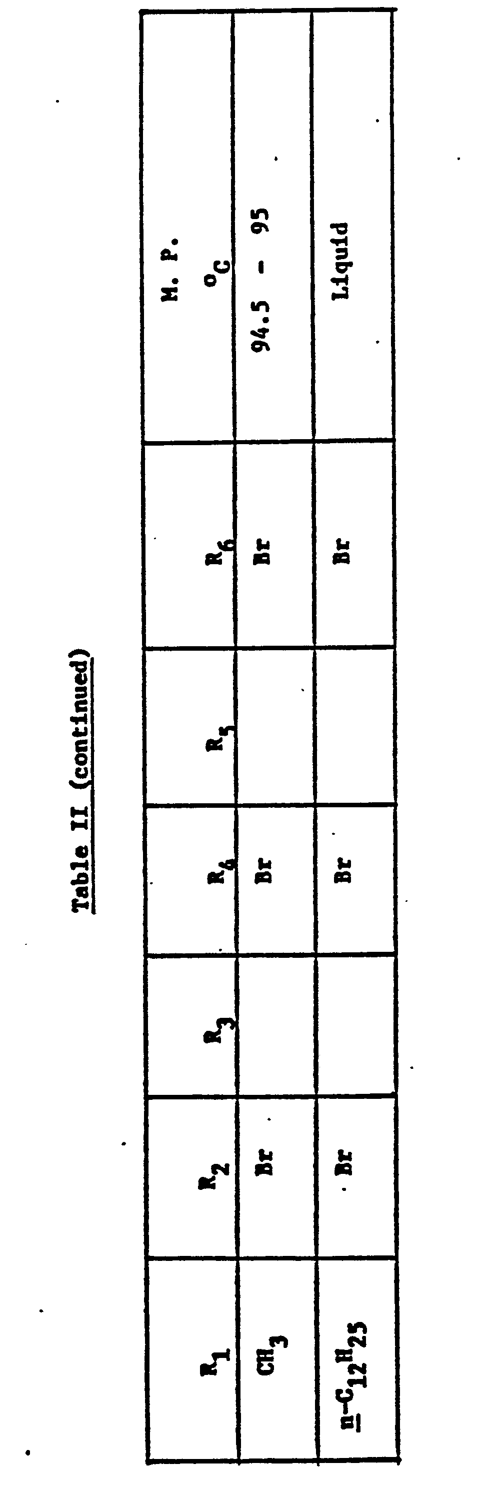

### Example 3

### Preparation of (2-Propynyloxy) (2,4,6-tribromophenyl) diimide-1- oxide

A suspension of the silver salt of 2,4,6-tribromo- phenylnitramine (4.82g; 0.01 mol) in ether is reacted with 3-bromopropyne (1.3g; 0.011 mol) under a nitrogen atmosphere at 25°C. After 16 hours the mixture is filtered through a layer of celite, the ether solution is washed with water, dilute sodium hydroxide and again with water and then dried over anhydrous magnesium sulfate. The solvent is then removed under vacuum, the residue dissolved in methylene chloride and filtered through layer of alumina. The filtrate is concentrated and crystallized from petroleum ether. Thin layer chromatography (silica gel; haxane : CH₂Cl₂ in a 1:1 ratio) and NMR indicate the product to be a 83 to 17 percent mixture of the 0 and N substituted products.

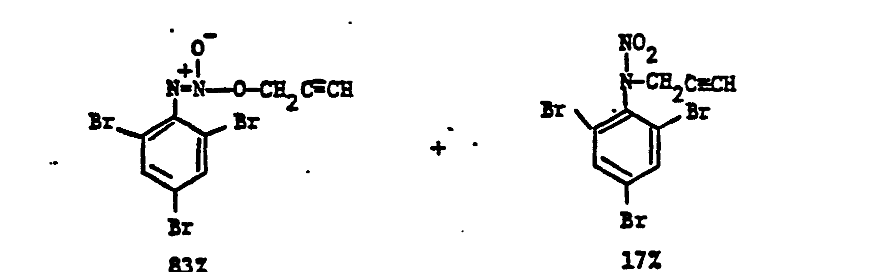

Substituting methyl iodide or n-dodecyl bromide for 3-bromopropyne-1 in the above reaction, the corresponding 0-methyl and O-n-dodecyl analogs are obtained, respectively.

Similarly reacting the silver salt of 2,6-dibromo- phenylnitramine with methyl iodide affords the corresponding 0-methyl compound.

### Example 4

### Preparation of substituted benzylnitramines

A solution of the appropriate benzyl halide or mesylate (0.03 mol) and ammonium nitrourethane in dry dimethylformamide (50 ml) is heated at 80°C for four hours. The reaction mixture is cooled and poured into ice water, and then extracted three times with ether. The combined extracts are washed with water and dried over anhydrous magnesium sulfate. Next, anhydrous ammonia gas is passed into the solution for 30 minutes. The precipitated material is isolated, washed in the ether and then dissolved in water (100 ml). The aqueous solution is stirred vigorously and its pH adjusted to 5. The precipitated product is filtered, washed with water and dried. If desired, the product may be recrystallixed from a mixture of hexane: toluene (1:1).

The thus prepared products are shown in Table III below.

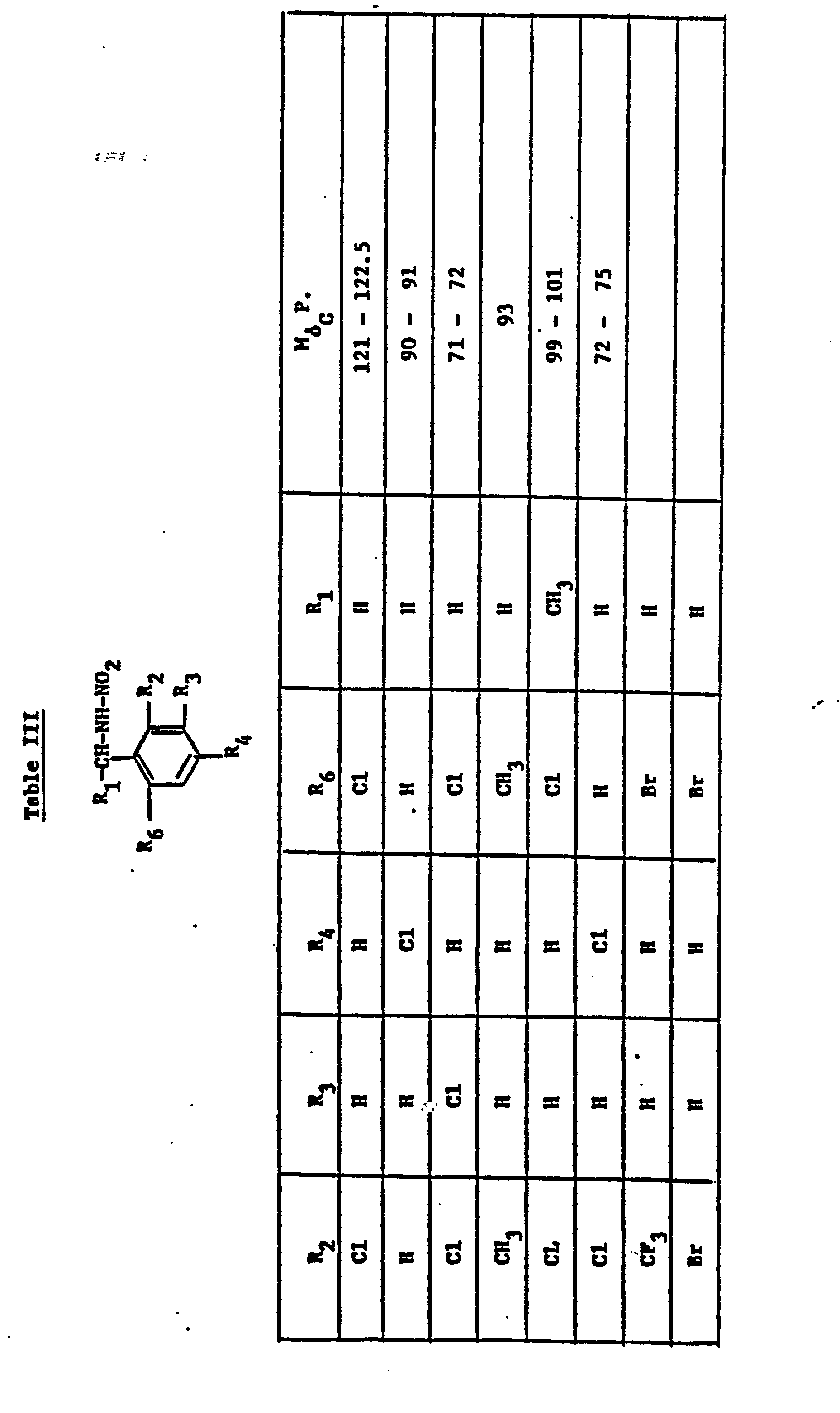

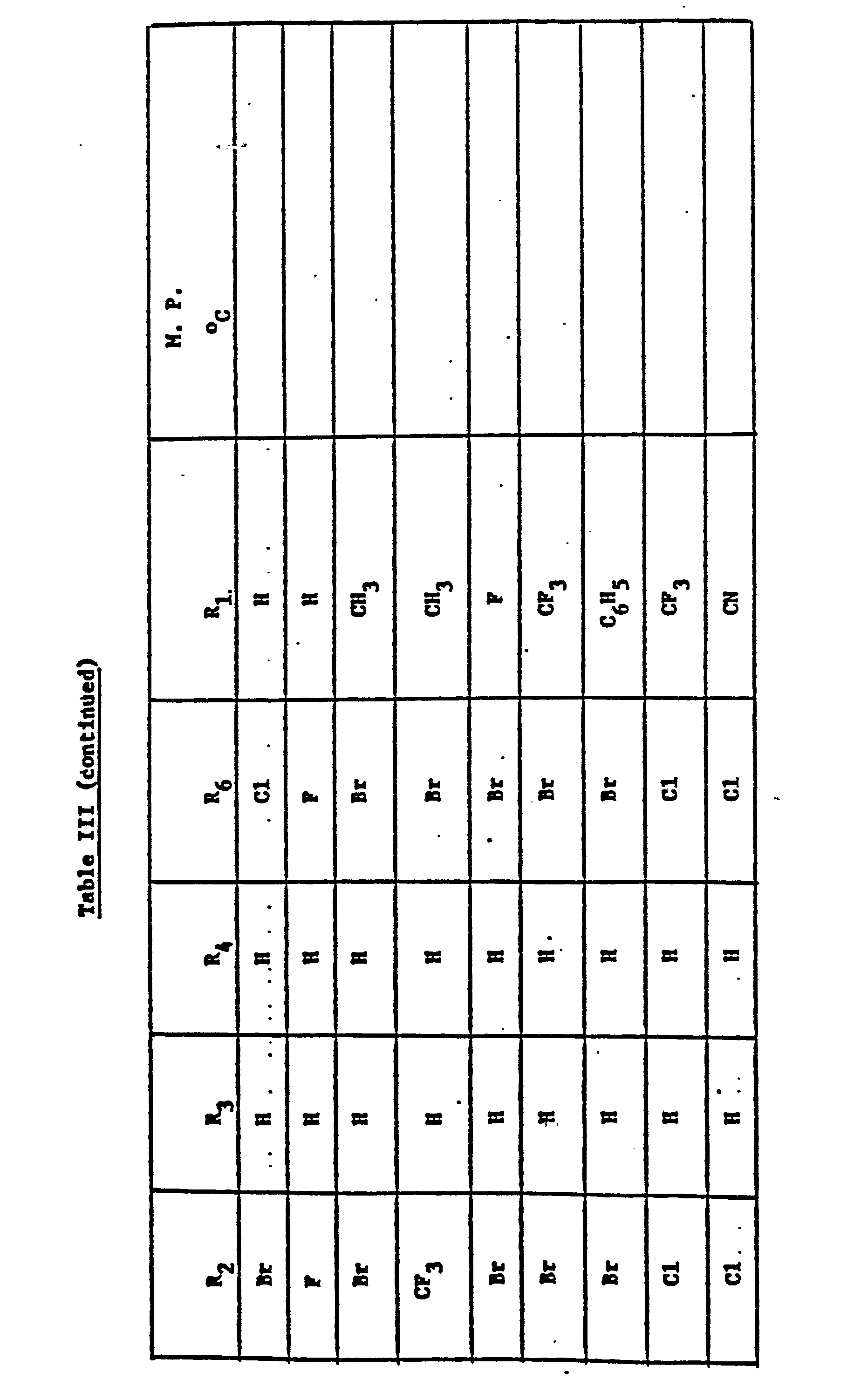

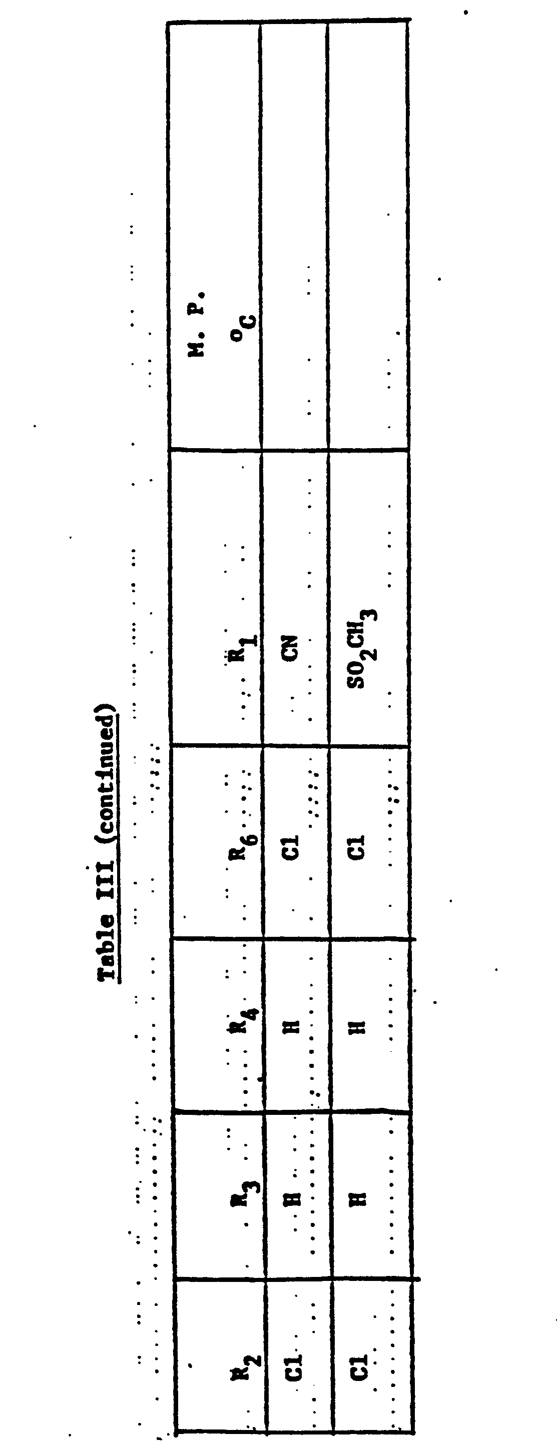

### Example 5

### Preparation of miscellaneous salts of substituted benzylnitramines

By the methods of Example 1, a number of salts of various benzylnitramines are prepared. These are shown in Table IV below.

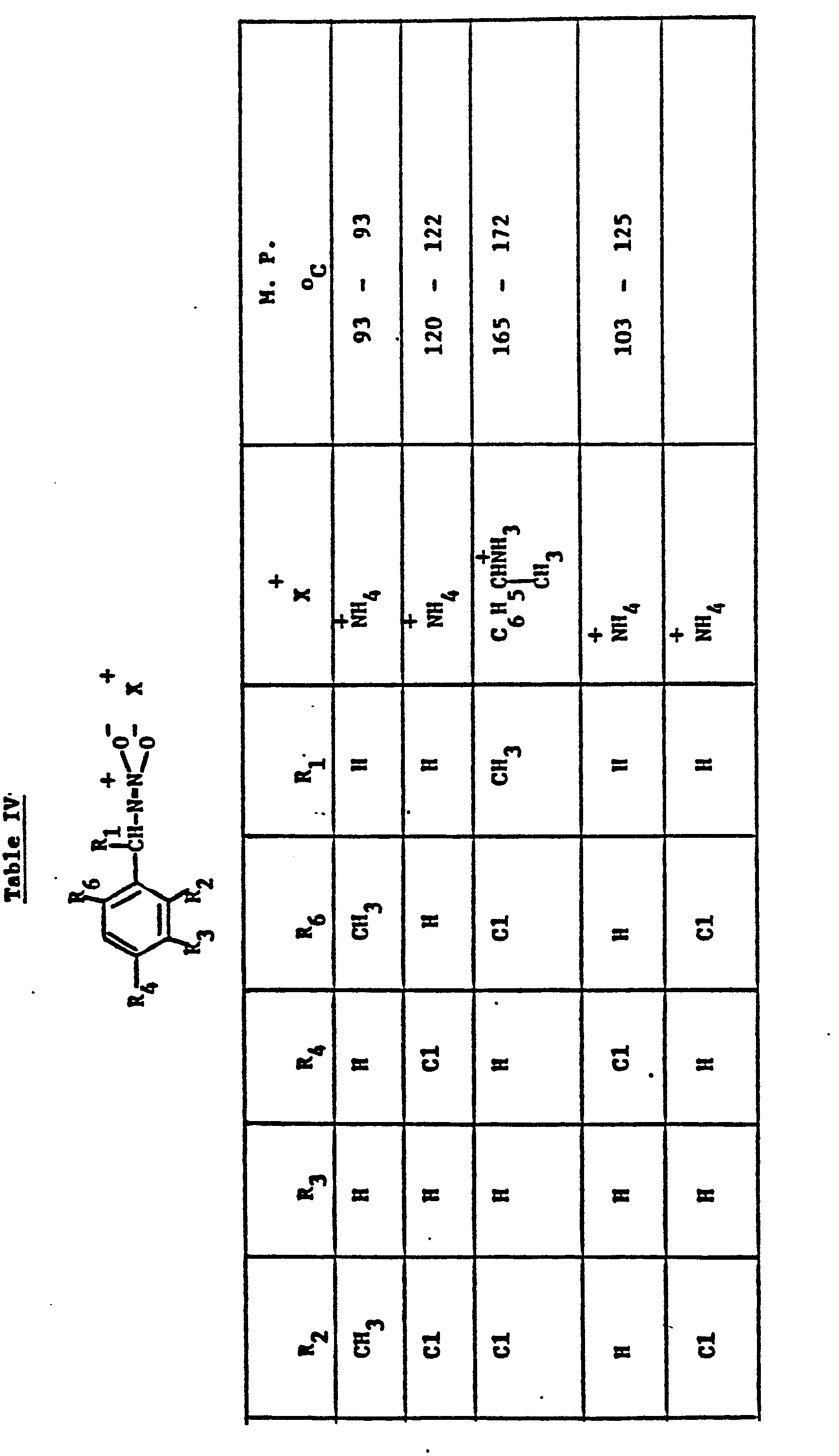

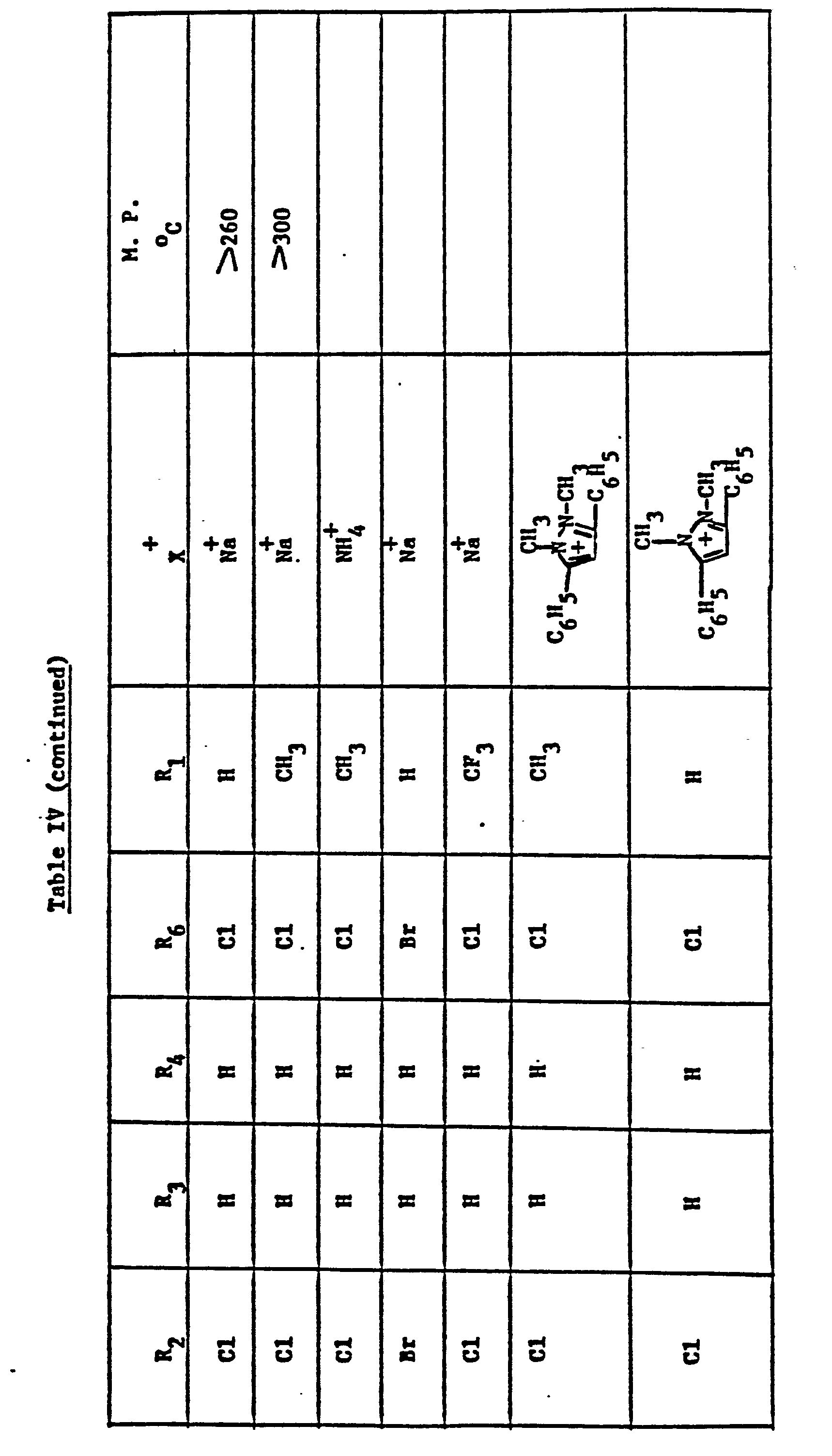

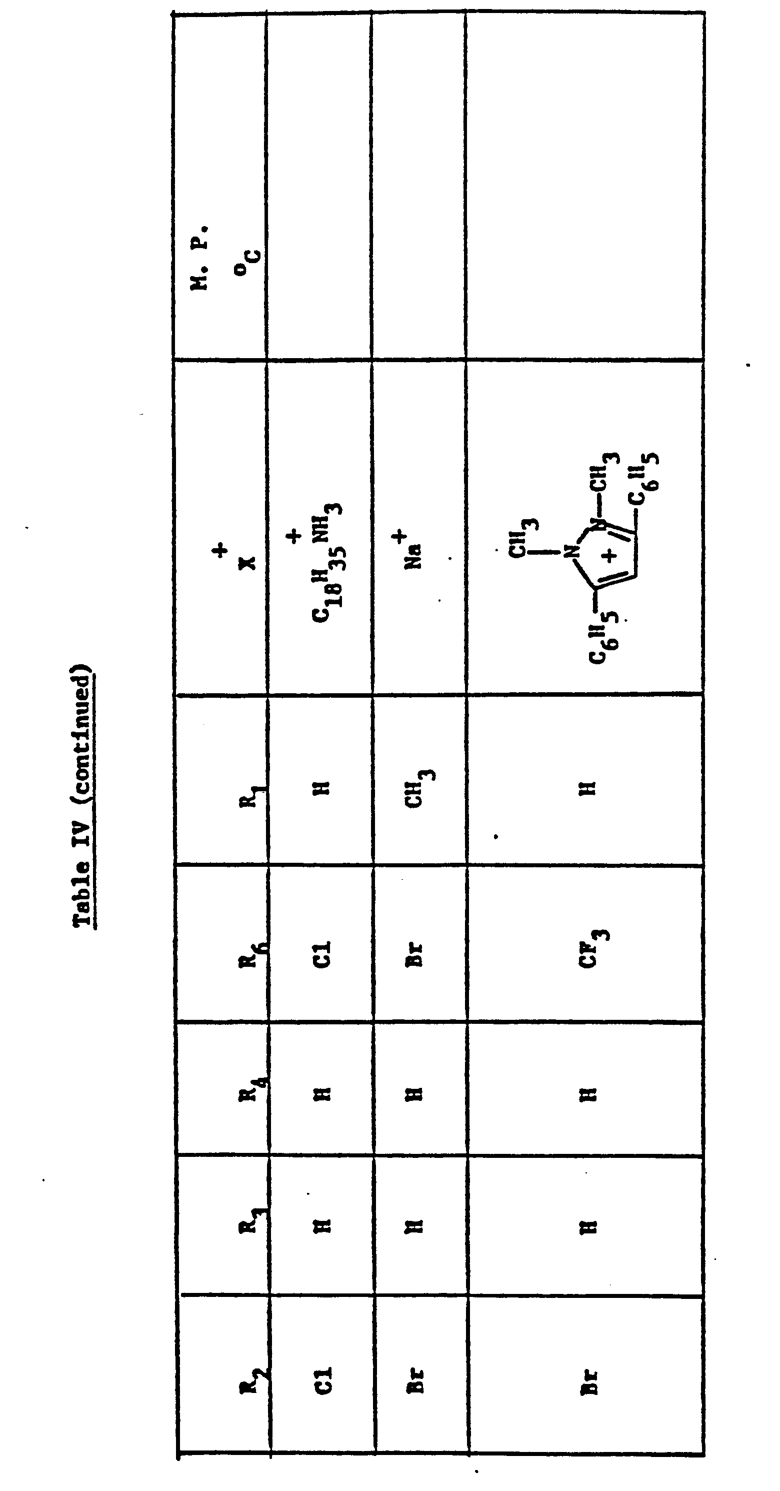

### Fxample 6

### Lithium diisopopylamide mediated N-nitration of anilines

A solution of diisopropylamine (0.20 mol) in diethylether (300 ml) is cooled to -75°C under N₂ and lithiated with n-butyllithium (0.20 mol of a 1.6M solution hexane). The solution is allowed to warm slowly to 0°C over four hours, then is cooled down to -60°C. A solution of the appropriate aniline (0.10 mol) in ether (300 ml) is added dropwise and the reaction mixture is allowed to warm to 0°C over one hour. Cooling the reaction mixture back to -60°C is followed by the addition (dropwise) of methyl nitrate (0.10 mol). The reaction mixture is allowed to warm to ambient temperature overnight, and poured onto 300 ml water. The aqueous layer is washed with ether, cooled in an ice bath, and acidified dropwise with concentrated hydrochloric acid. The mixture is extracted with ether, the combined ether extracts are washed successively with cold 5% HCl, water and brine. Removal of the solvent under vacuum affords the product.

By the above method and other methods known in the art, a number of N-nitroanilines are prepared, and are shown in Table V below.

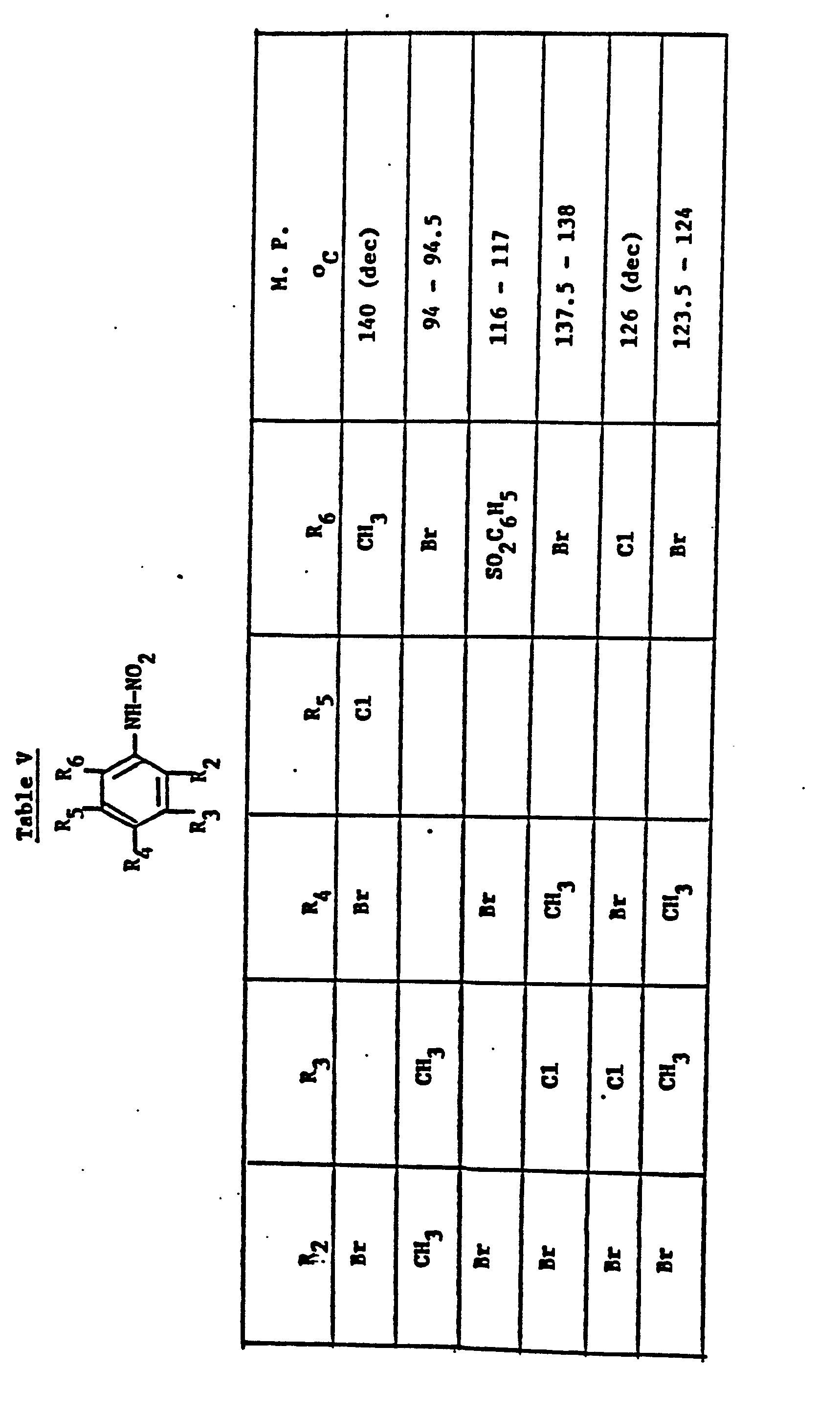

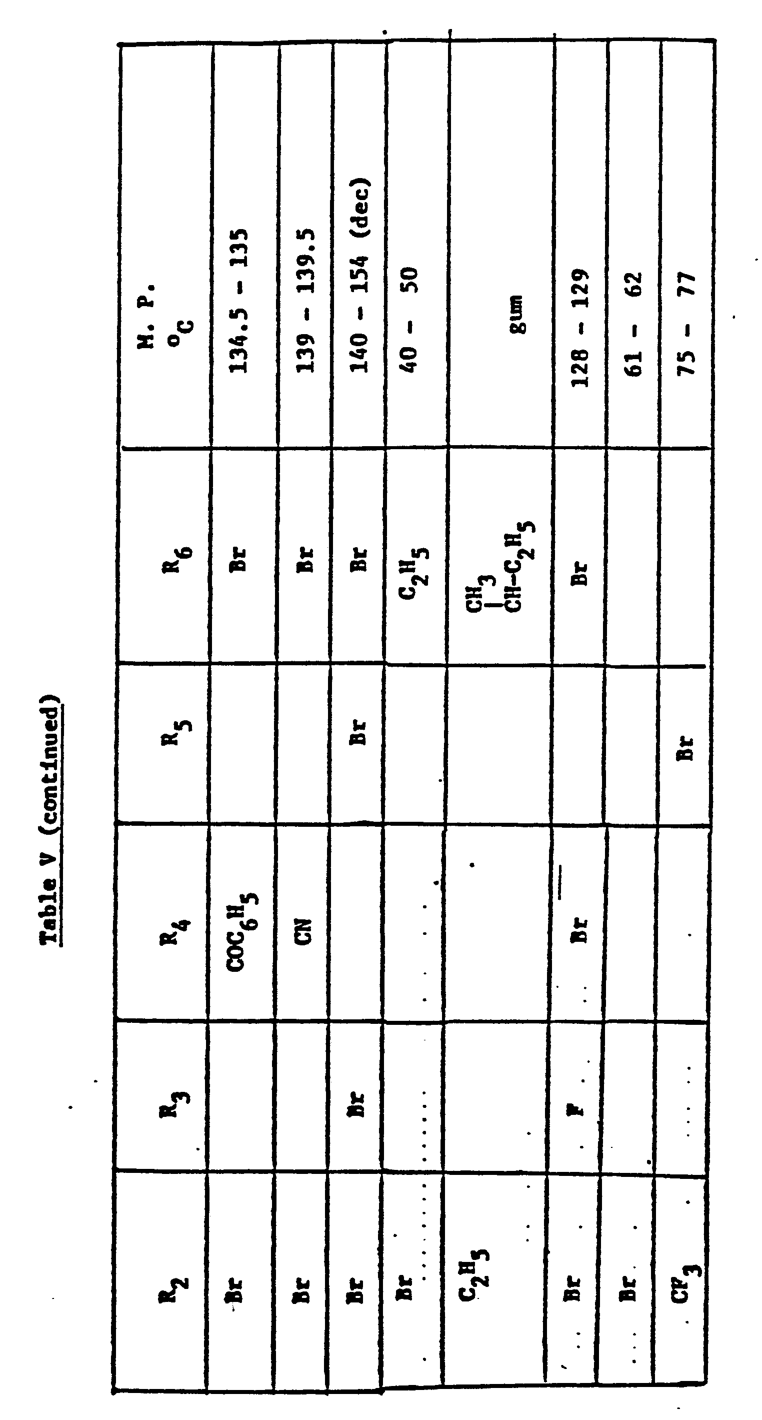

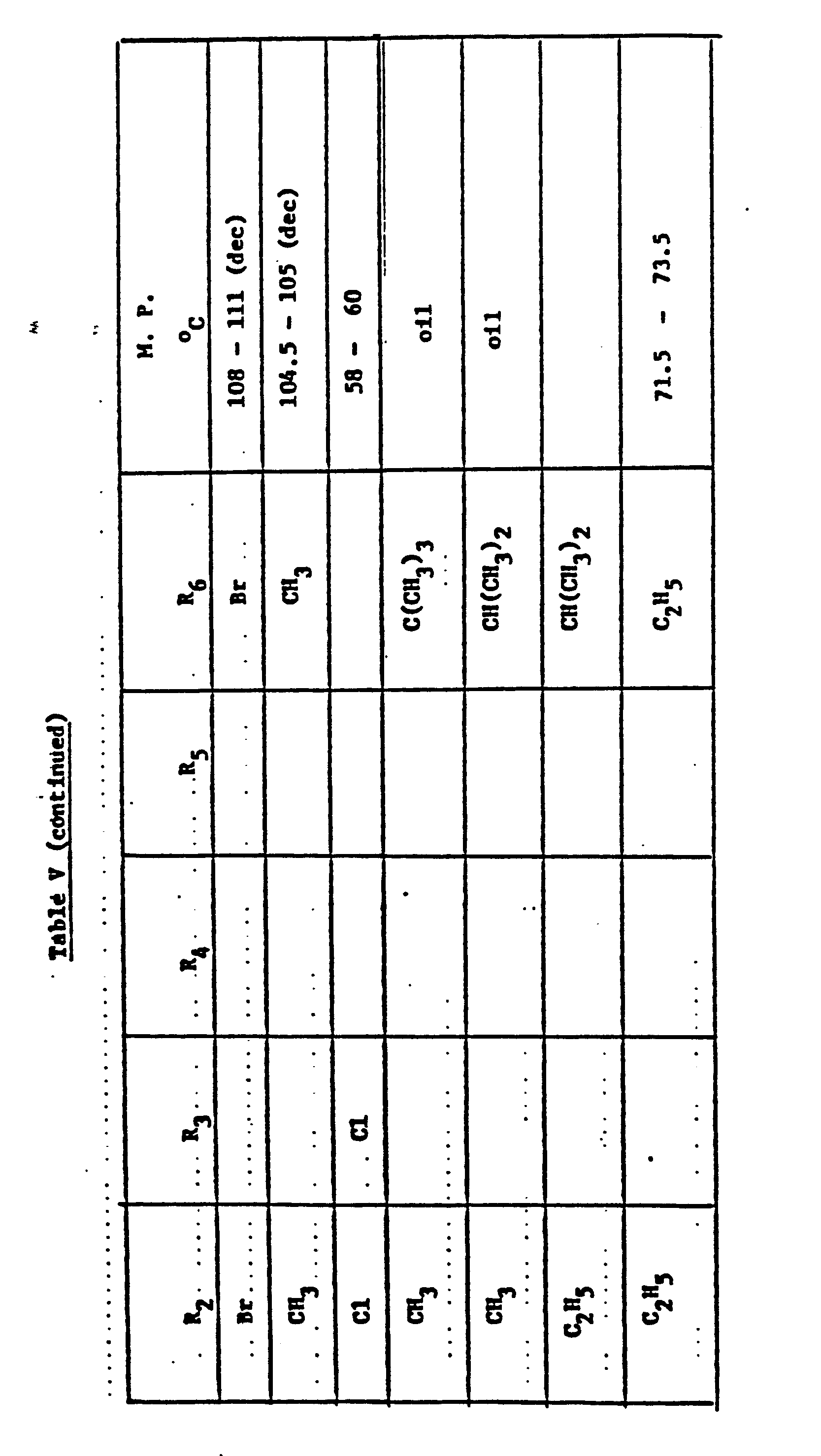

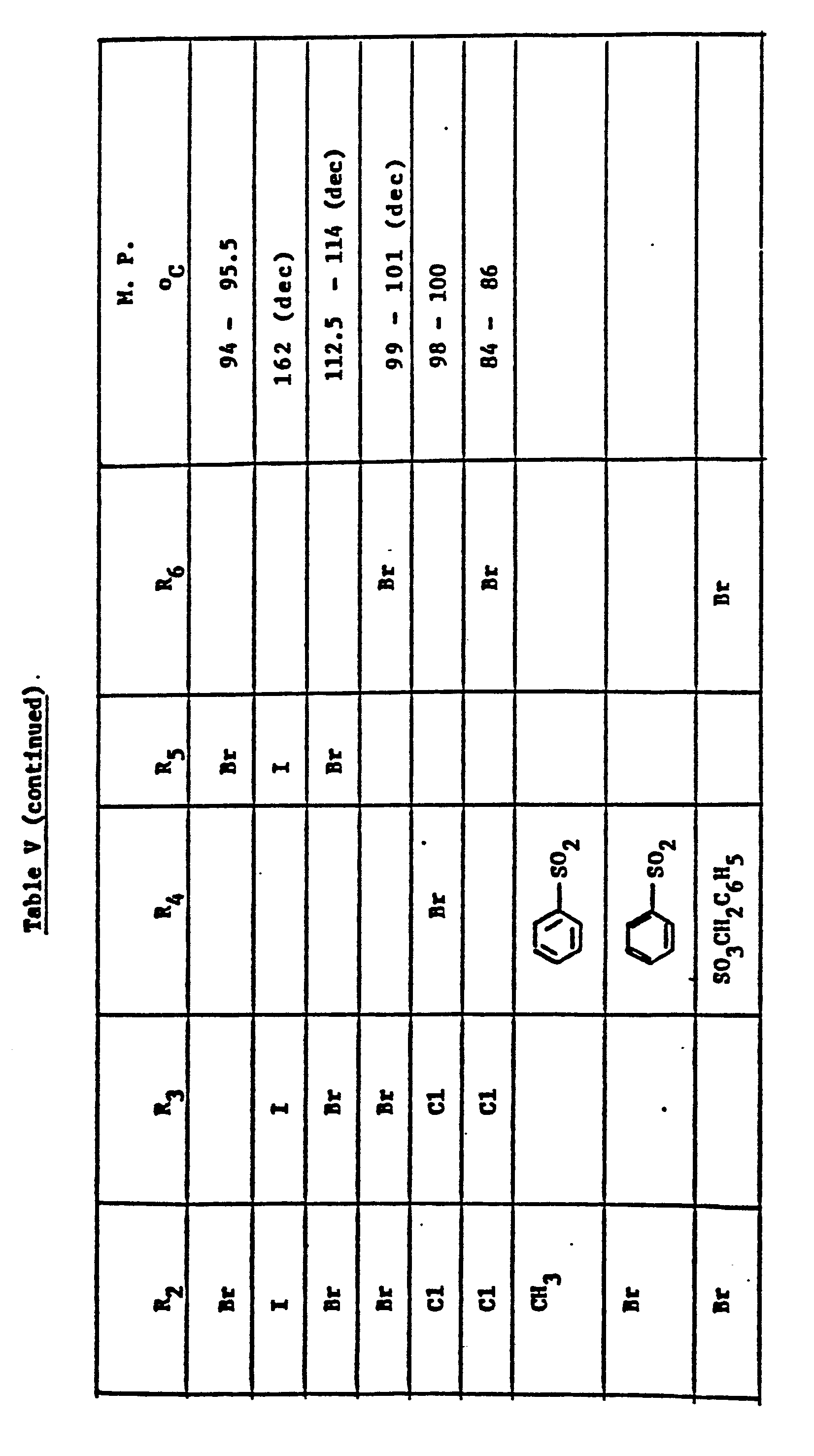

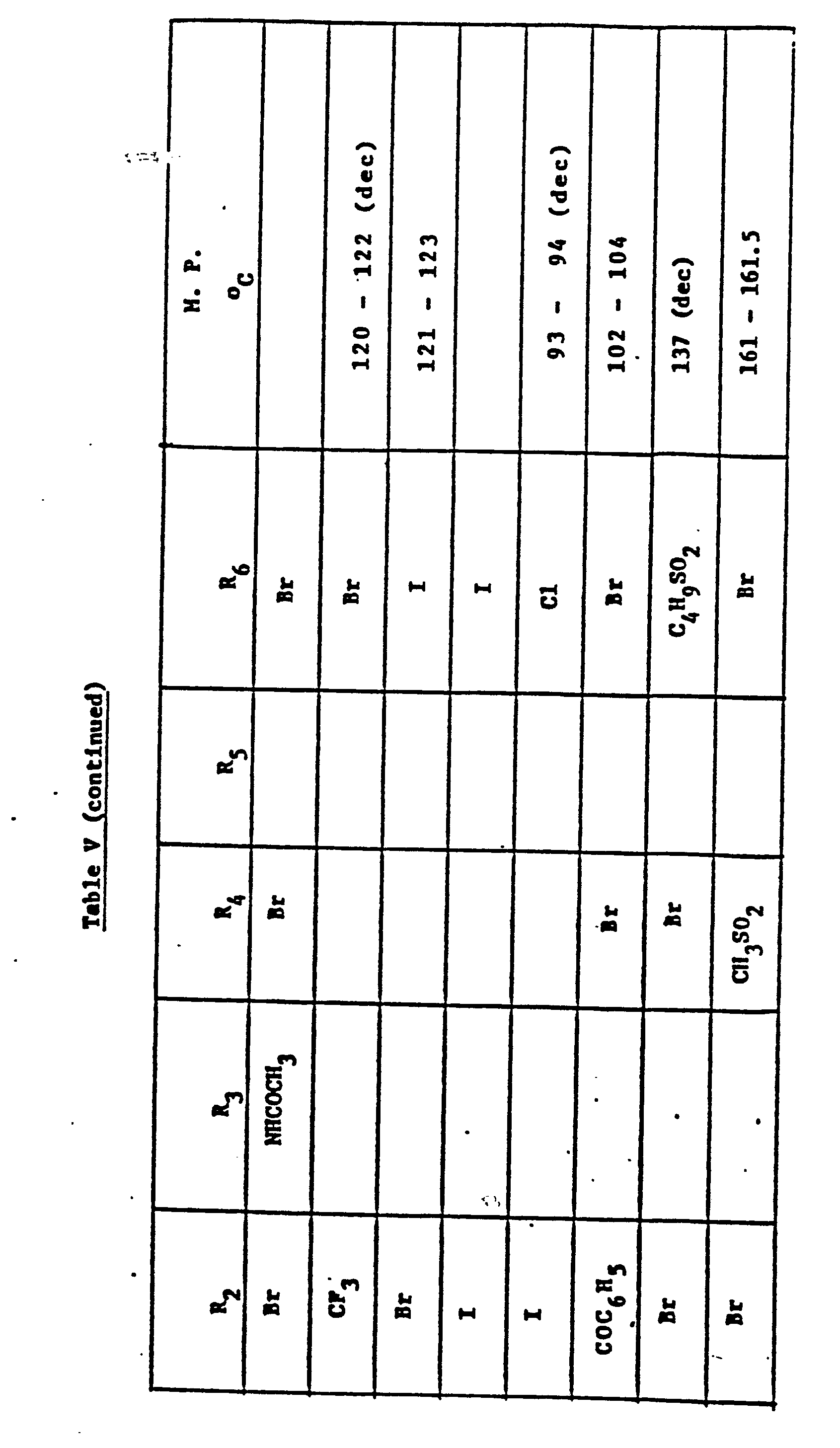

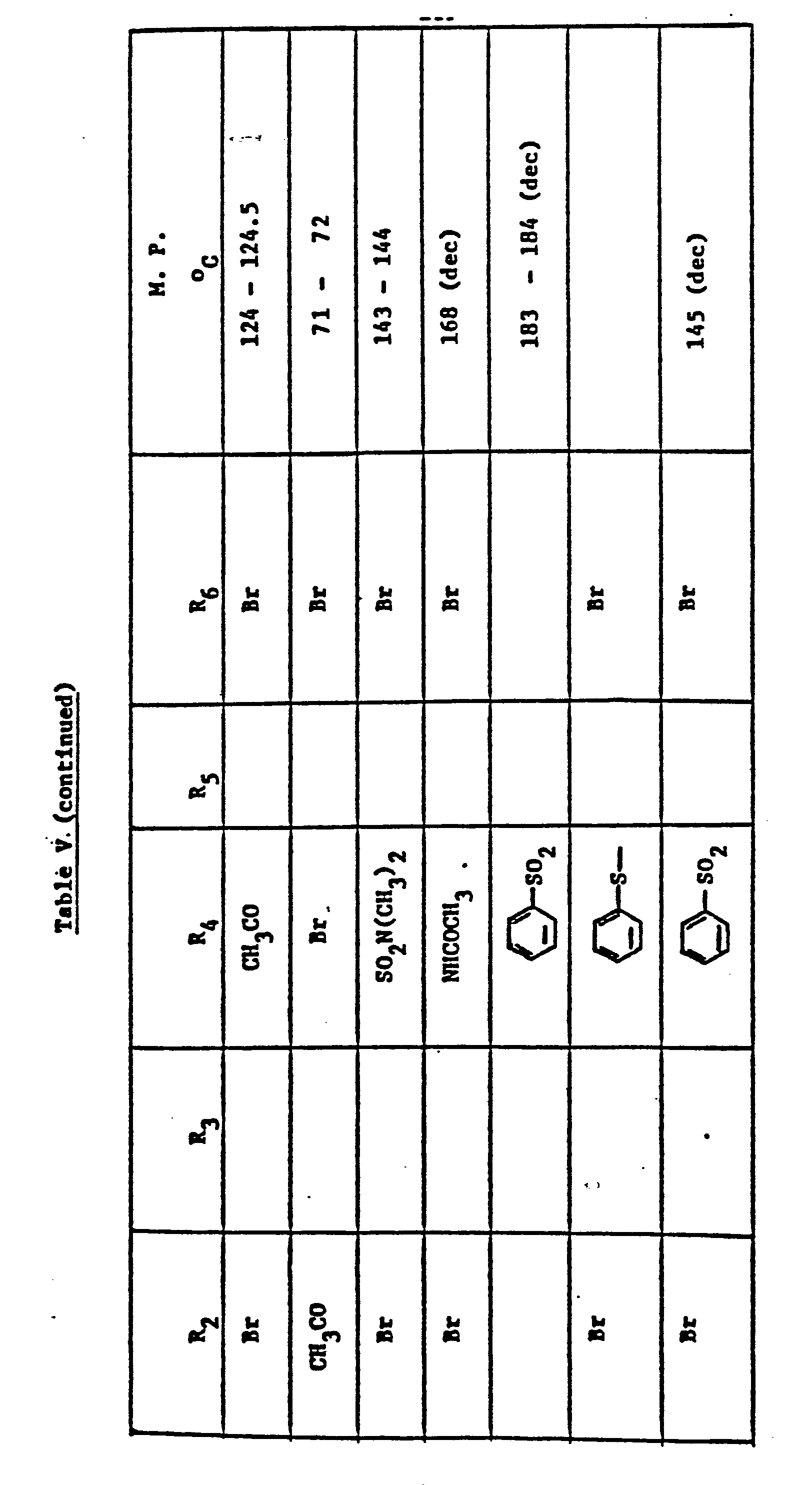

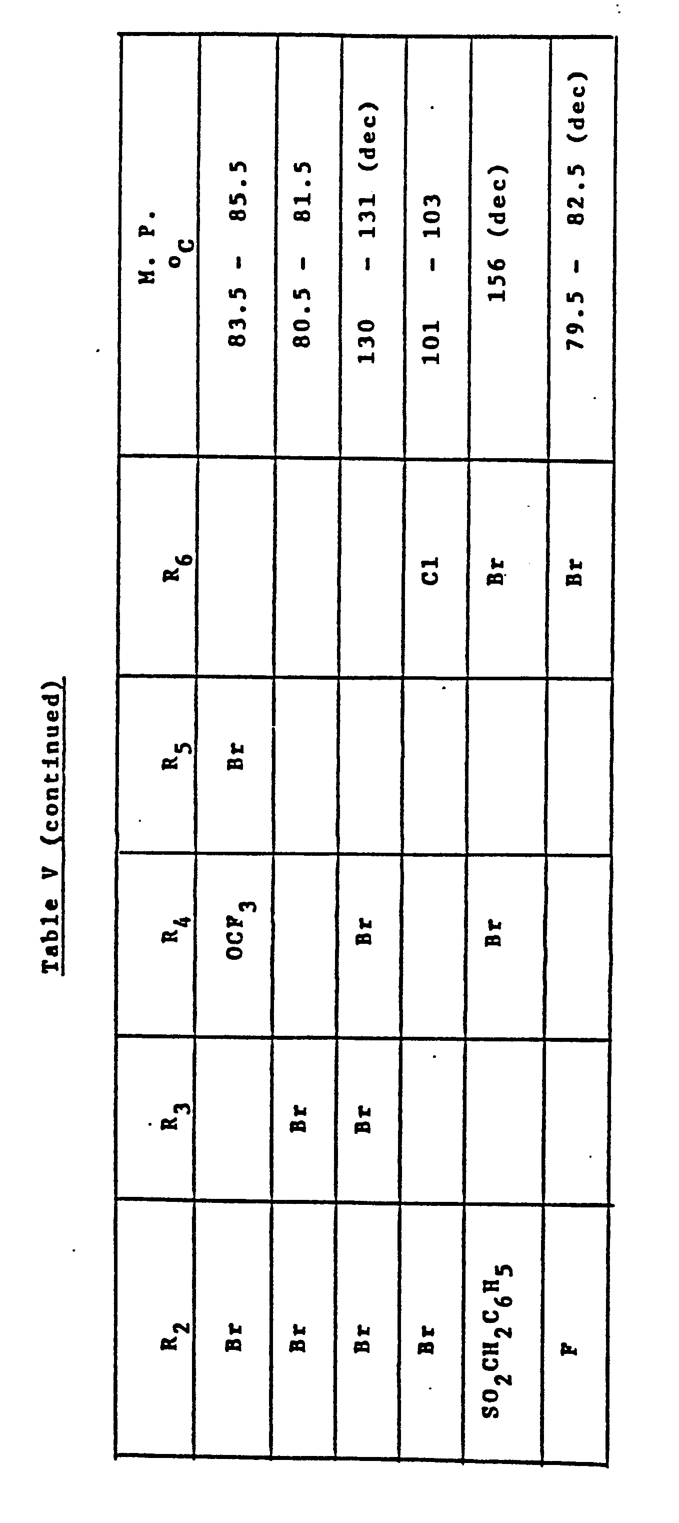

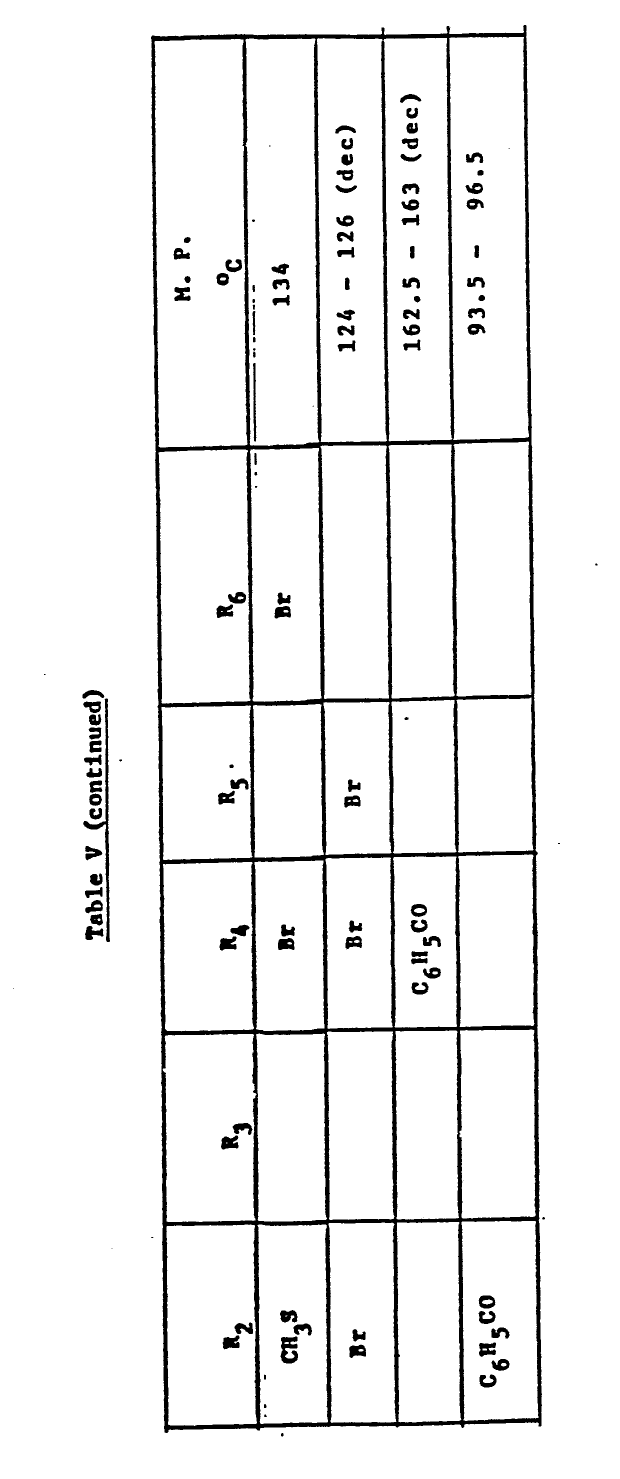

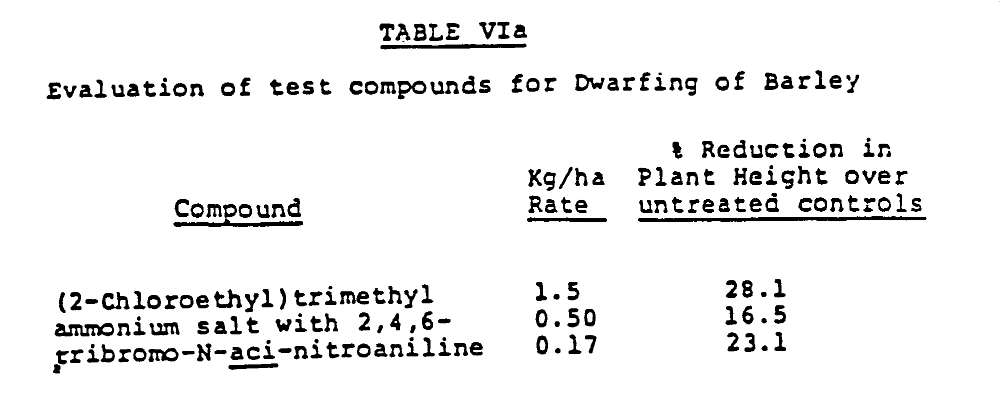

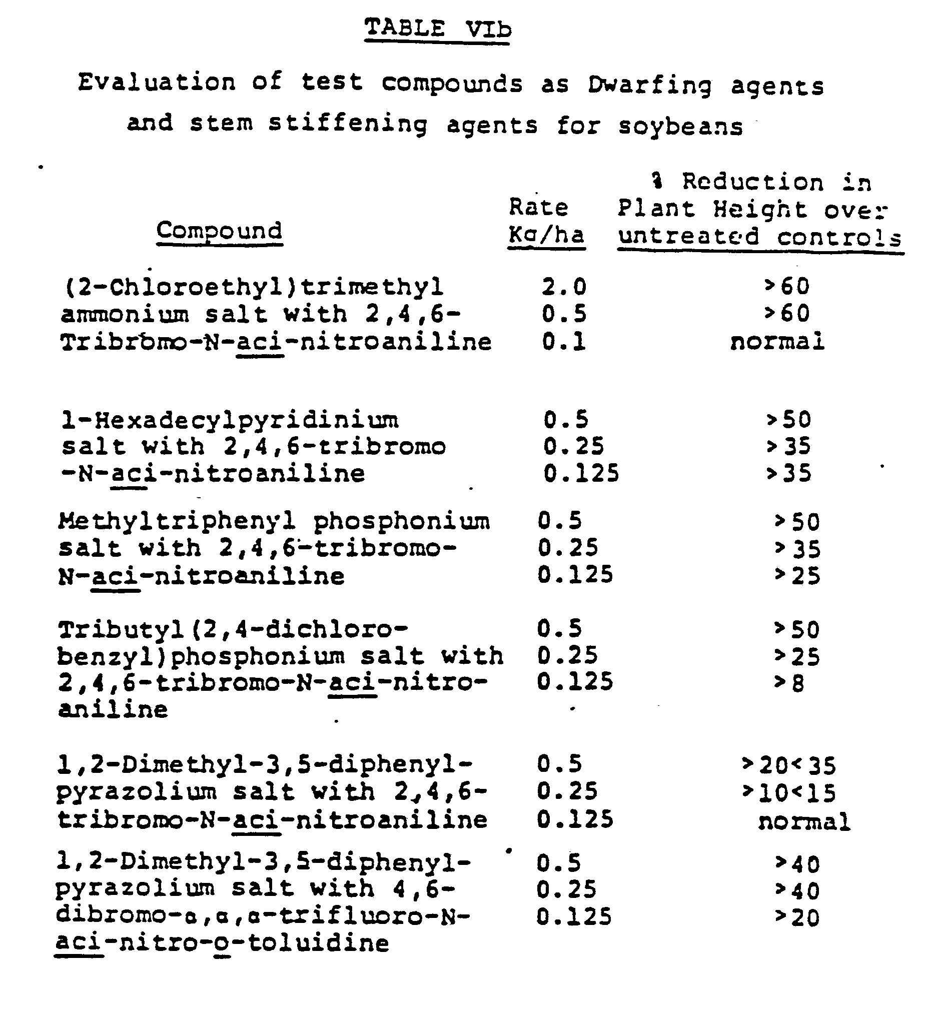

### EXAMPLE 7

### Evaluation of test compounds for Increasina axillarv Branching and improving the canopy of Broad leaf plants using Soyceans (Glycine max) Adelphia variety as the plant species

In these tests seedling soybean plants approximately three weeks old, and grown in plastic pots to the 2nd to 3rd trifoliate stage, are sprayed with solutions or dispersions of test compound prepared as described in Example7 above. The same spraying apparatus, described in Example 7 above, is used and after spraying the plants are placed on greenhouse benches and watered and fertilized. Three weeks after treatment the plants are examined to determine what effect the application of test compound has had on the axillary branching and canopy development of the treated plants.

Increased axillary branching and improved canopy are biological responses which are highly advantageous in the growing of ornamental plants. These responses also have advantage in many crops since increased branching may result in the greater production of fruit, while improved canopy tends to shade and suppress the growth of undesirable weeds that compete with the crops for sunlight, water and plant nutrients. Data obtained are reported in table VII below where axillary branching and canopy are reported as percent increase over untreated controls.

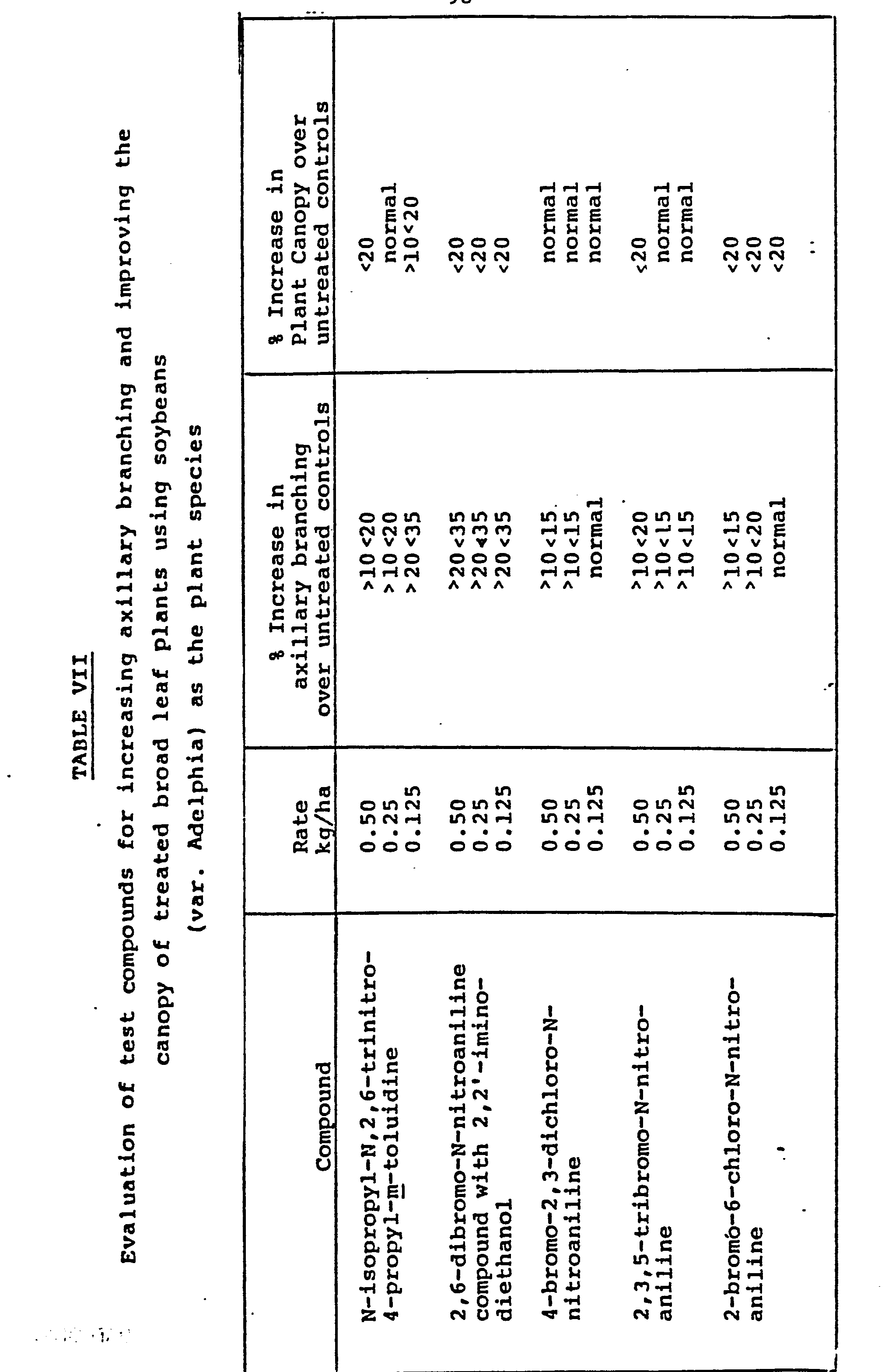

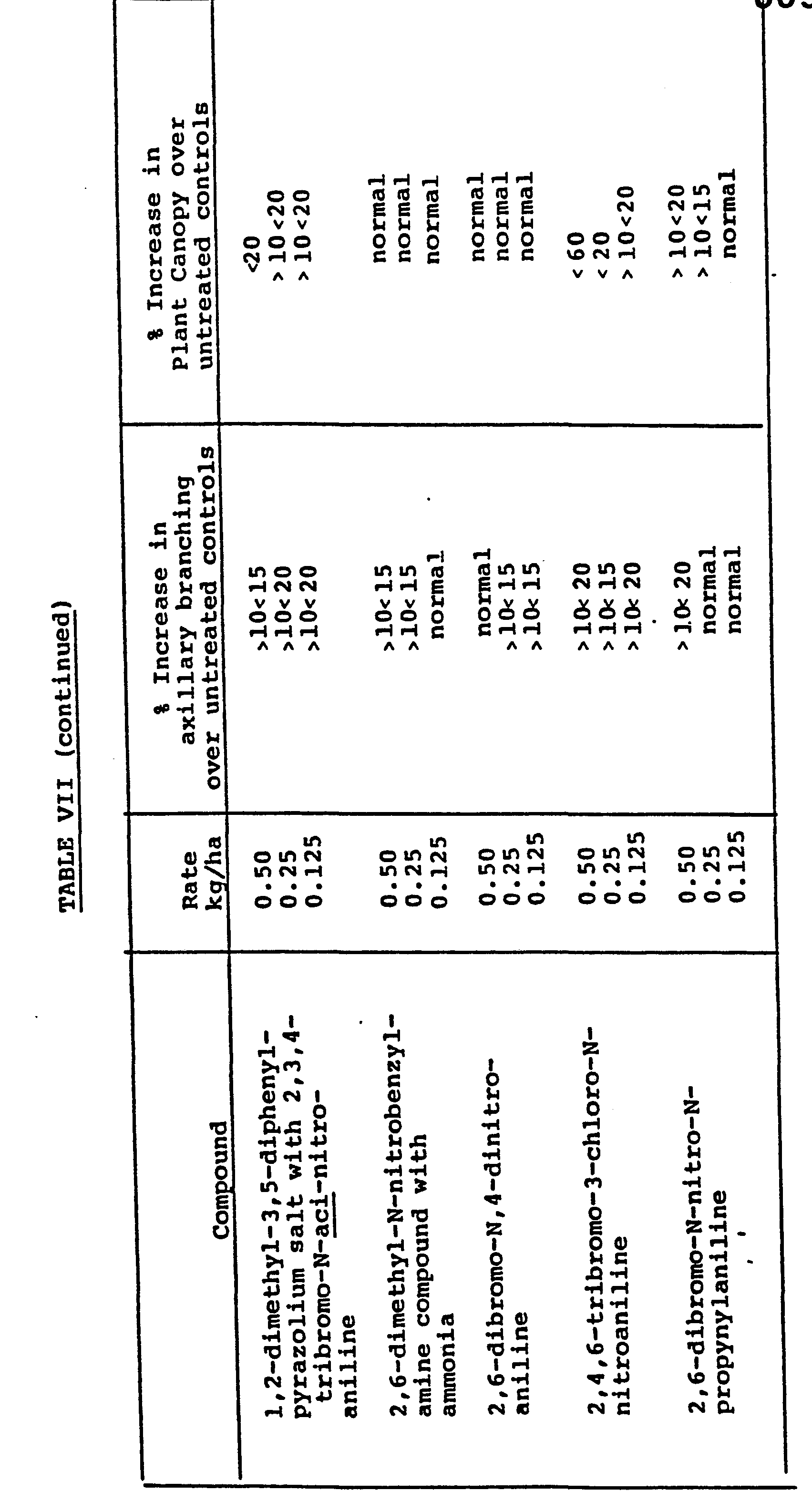

### EXAMPLE 0 0059226

### Evaluation of test compounds to determine their effectiveness for increasinq flowering of plants using soybeans (Glycine max) var. Adelphia as the plant species

In the following tests seedling soybean plants growing in plastic cups are sprayed with aqueous-acetone solutions or dispersions of test compounds when they have reached the 2nd to 3rd trifoliate stage. Test solutions or dispersions are prepared with 0.25 v/v Colloidal BIOFILM® surfactant and sufficient test compound to provide the plants with 0.5, 0.25, 0.125 or 0.06 kg/ha of said compound when the test solutions are sprayed on said plants from an overhead sprayer delivering 747 1/ha of solution.

After spraying the plants are placed on greenhouse benches and watered and fertilized. Three weeks after spraying the plants are examined and the amount of flowering determined for all plants. Data obtained are reported in the table below as percent increase in flowering over untreated controls.

From the data reported in Table VIII below it can be seen that the compounds of the present invention applied at rates of from 0.06 to 0.25 kg/ha enhance flowering of treated plant more than 10% over untreated controls. This response is particularly desirable for treatment of flowering varieties of ornamental plants as well as agronomic crops.

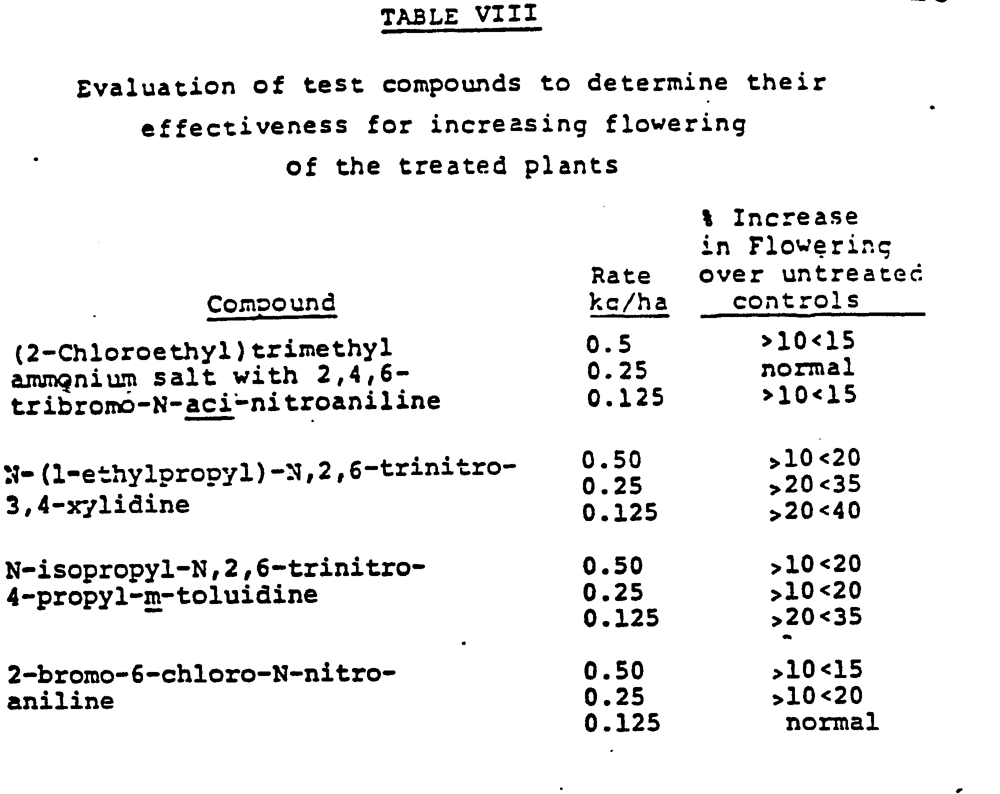

### EXAMPLE 9 0059226

### Evaluation of the antilodging effects of certain N-nitroanilines and salts thereof on Barlev .

In this experiment the procedure of Example 7 is repeated excepting that the crop used was barley (Hordeum vulgar, var. Georgie; European lodging prone variety). The plants are treated at the 3-4 leaf stage. Plants are watered prior to treatment and then sprayed to provide the kg/ha rate of test compound desired. After spraying the plants are placed on greenhouse benches and watered and fertilized in accordance with normal greenhouse procedures.

Three weeks after spraying the plants are measured and harvested. All treatments are replicated six times and companions are made against untreated controls. The data obtained are averaged and reported in Table XII below.

The following rating system is used:
5 _{"} No increase over controls
4 = Slight increase over controls
3 = Moderate, significant increase over controls.

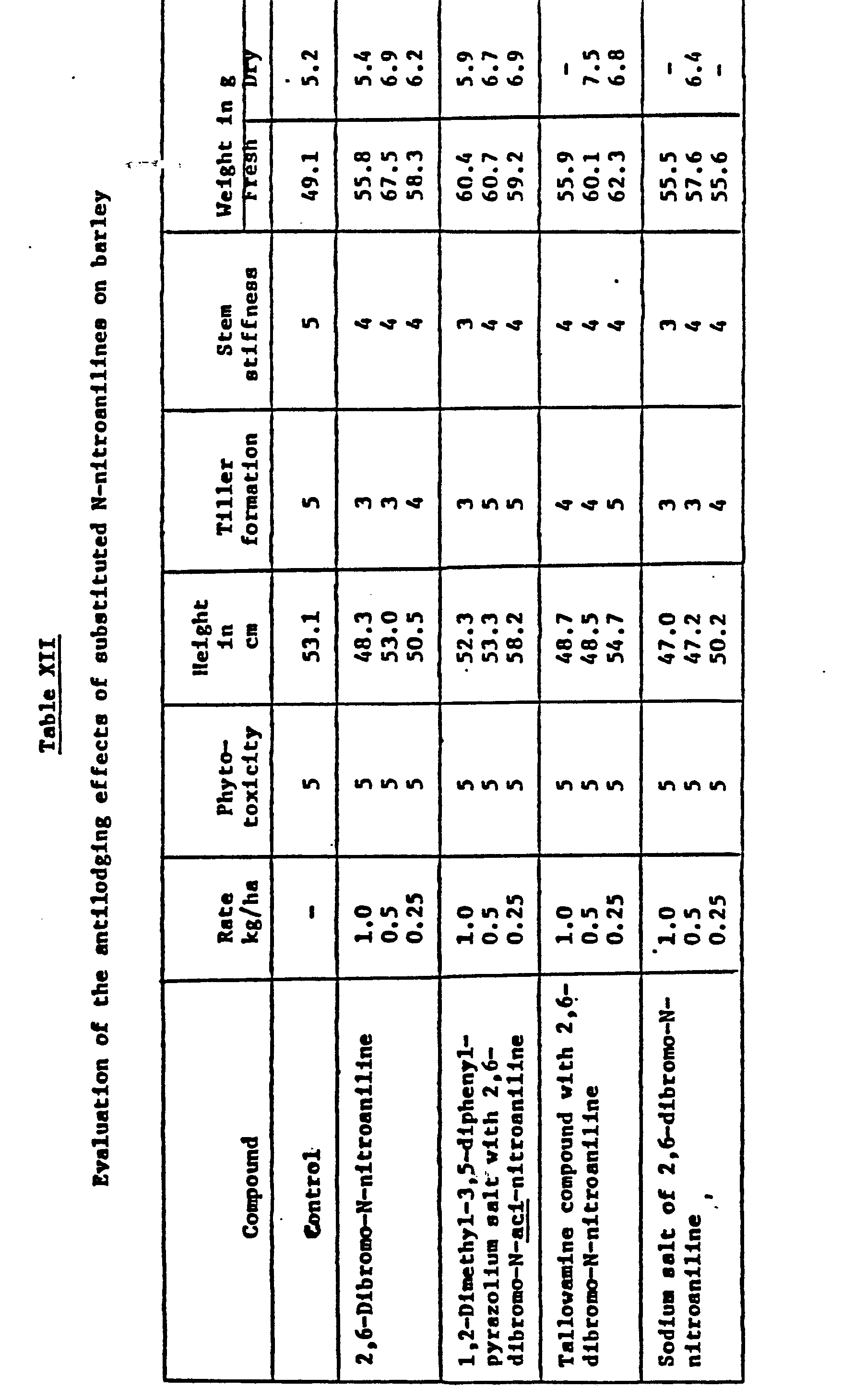

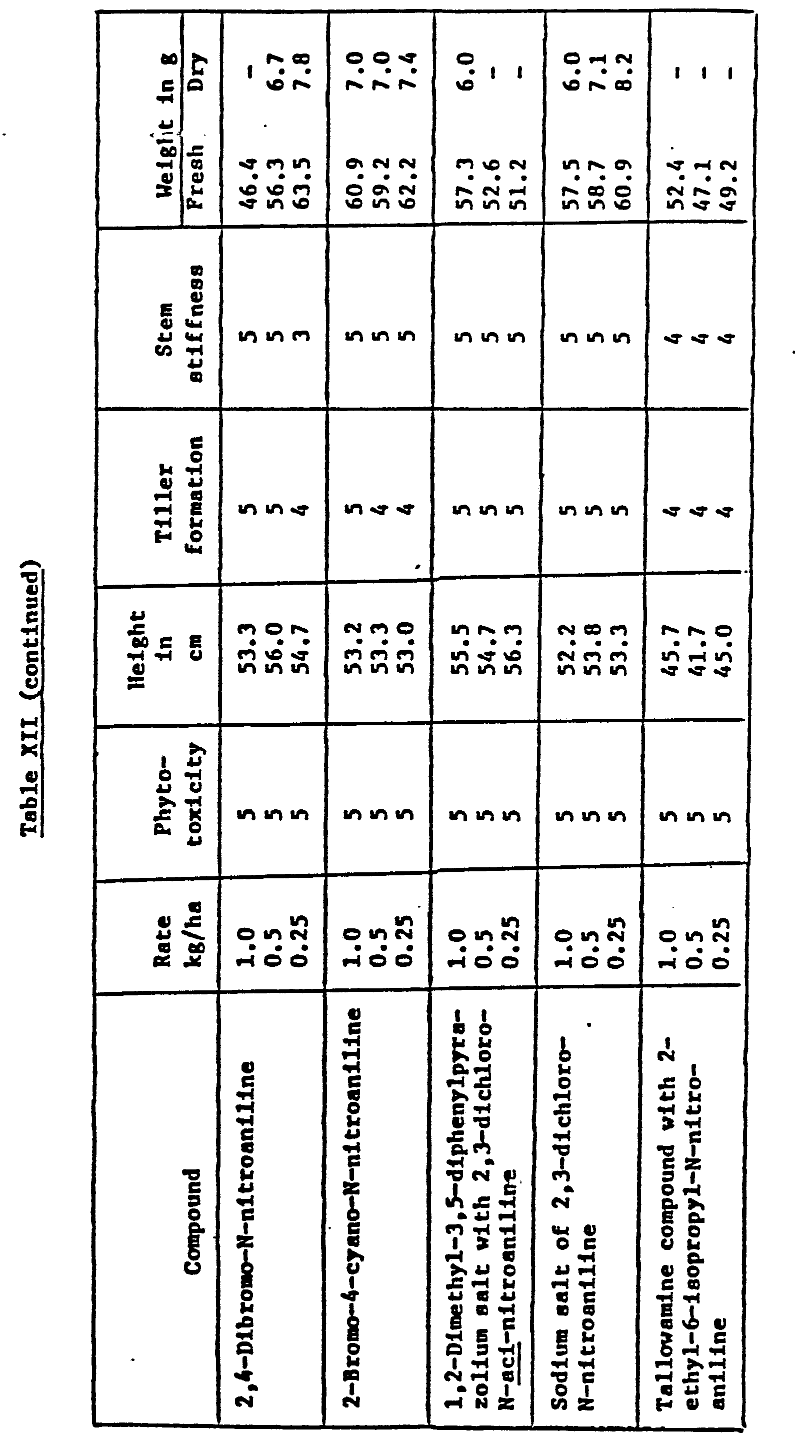

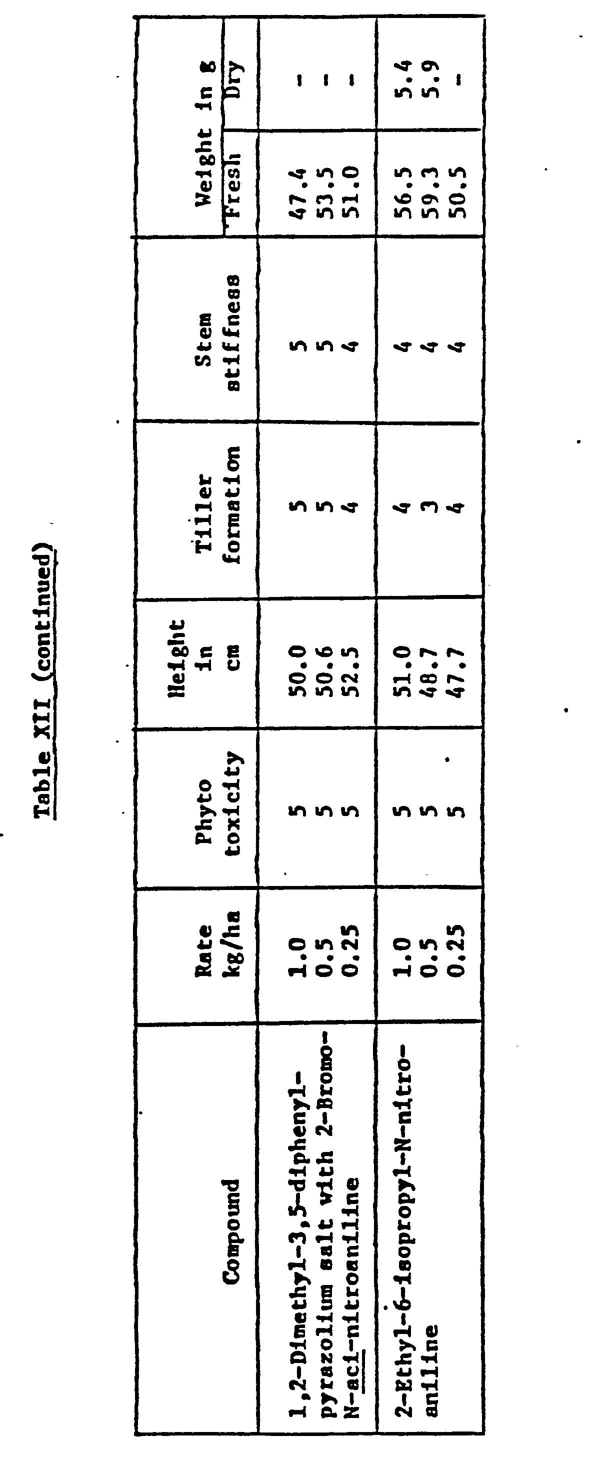

## Claims

1. Substituted N-nitroanilines represented by formulae (I) or (Ia) below: 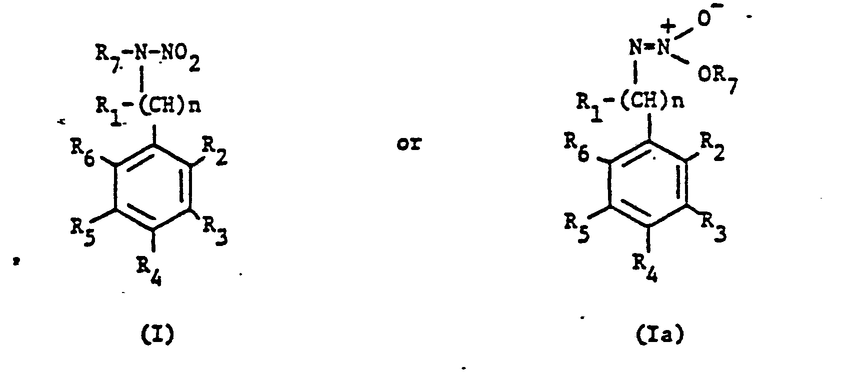 wherein n is an integer of 0 or 1; R₁ is hydrogen, Cₗ₋C₃ alkyl, C₁-C₃ haloalkyl, CN, halogen, N0₂, SO₂R₈, COR₉, phenyl or substituted phenyl and the substituents are CH₃, CF₃, CH₃O, halogen, NO₂ or OCH₂ CO₂C₂H₅; R₂ and R₆ are hydrogen, halogen, C₁-C₄ alkyl, C₁-C₄ haloalkyl, C₁-C₄ haloalkoxy, SO₂R₈, SR₈ or COR₁₀; R₃ is hydrogen, C₁-C₄ alkyl, C₁-C_{4 alkox}y, _{NH2}, NH-COCH₃, halogen, N0₂, SO₂R₈, CN or CO₂CH₃; R₄ is hydrogen, halogen, C₁-C₂ alkyl, SO₂R₈, SR₈,OCF₃, COR₉, ^{C}N, NH COCH₃, SO₂NR₁₀R₁₁, 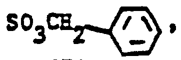 phenoxy or substituted phenoxy and the substituents are CH₃, CF₃, CH30, halogen, NO₂ or OCH₂CO₂C₂H₅;R₅ is hydrogen, Cₗ₋C₃ alkyl or halogen; . and with the proviso that where R₃, R₅ and R₇ are all hydrogen, and n is an integer of 0 and the compounds are of formula I, then R₂ cannot be halogen, C₁-C₄ alkyl, or SO₂CH₃, C₁-C₃ haloalkyl or C₁-C₃ haloalkoxy; R₄ cannot be halogen or methyl; R₆ cannot be halogen, CF₃ or C₁-C₄ alkyl; R₇ is hydrogen, C₁-C₁₂ alkyl optionally substituted with halogen or CN, C₃-C₄ alkenyl, C₃-C₄ alkynyl, the methyl esters of lower alkanoic acids or the moiety 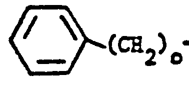 - wherein o is an integer of 0, 1, 2 or 3, and the moiety may optionally be substituted with halogen, C₁-C₂ alkyl, C₁-C₂ alkoxy, (C₁-C₃ alkyl)₃ SiCH₂ - or N0₂; R₈ is C₁-C₆ alkyl, phenyl or benzyl; R₉ is hydrogen, hydroxyl, Cₗ-^{C}₃ alkyl, C₁-C₃alkoxy or phenyl; R₁₀ and R₁₁ each are hydrogen, Cₗ-C₄ alkyl or phenyl; with the proviso that not less than two and not more than four of R₂ to R₆ are to be substituted with groups other than hydrogen in any one of the compounds represented by formulae . (I) or (Ia) or mixtures thereof; and when R₇ is hydrogen, the above compounds of formulae (I) or (Ia) may form inorganic or organic salts, the salt being represented by formula (II) below: 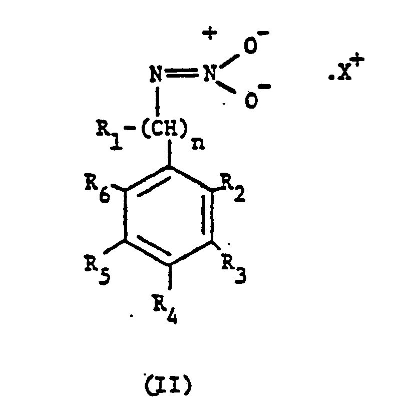 wherein X is Co, Cu, or formula IIIa 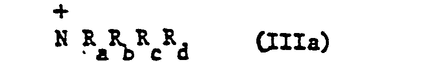 wherein Rₐ is hydrogen, C₁-C₃₀ alkyl straight chain or branched optionally mono - or disubstituted with HO-, CH₃0₂C-, CH₃S-, C₃-C₁₈ alkenylaminoalkylene (C₁-C₃), H₂N-, (CH₃)₂N-, 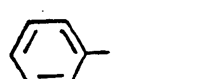 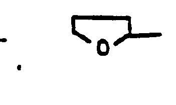 ], C₃-C₆ alkenyl, C₃-C₆ alkynyl, C₃₋C₆ cycloalkyl, 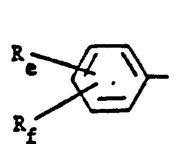 wherein Rₑ and R_{f} each may be H, CH₃ or n-C₁₆H₃₃SO₂-; R_{b} is hydrogen, C₁-C₃₀ alkyl straight chain or branched [optionally mono- or disubstituted with HO- or 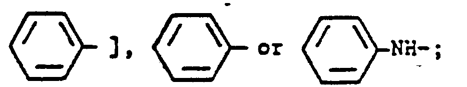 Rc is hydrogen, C₁-C₃₀ alkyl straight chain or branched [optionally mono- or disbustituted with HO- or 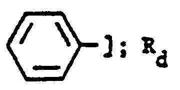 is hydrogen, C₁-C₃₀ alkyl straight chain or branched [optionally substituted with HO-, 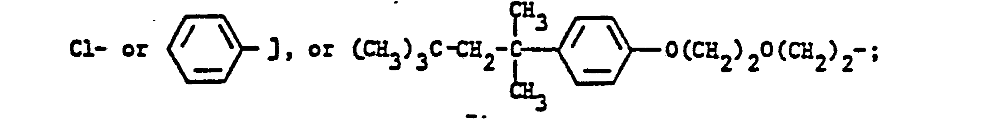 and when Rₐ and R_{b} are taken together vith the nitrogen they are attached to they represent a moiety of 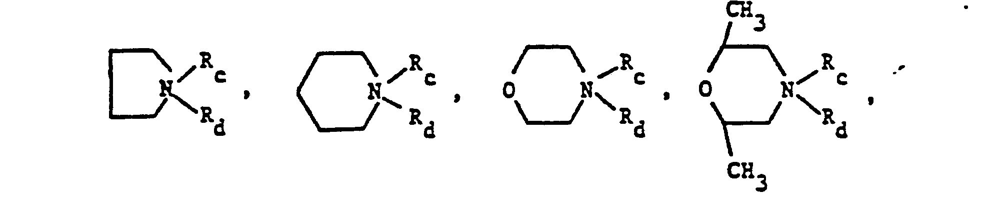 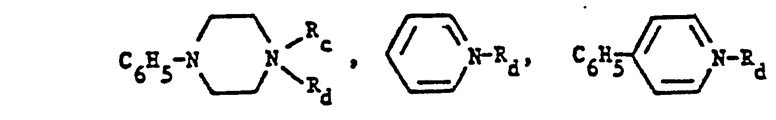 or 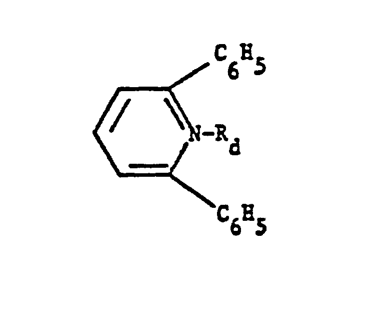 wherein R and R_{d} are defined as above organic cation X⁺ may also be imidazolium, pyridinium, quinolinium, dithiolium, tetrazolium or be represented by formula IIIb 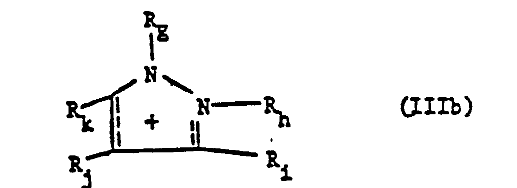 wherein R_{g} and Rₕ each are C₁-C₃ alkyl; Rⱼ is hydrogen, halogen, C.-C. alkyl or C₁-C₃ alkoxy; Rᵢ and Rₖ each are hydrogen C₁-C₆ alkyl straight chain or branched, C₃-C₆ cycloalkyl, 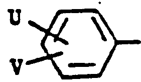 or 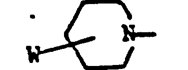 ; wherein U and V each are hydrogen, halogen, Cₗ-C₃ alkyl, C₁-C₃ alkoxy, CN or CF₃; W is hydrogen or C₁-C₃ alkyl; and each 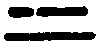 symbol represents a single or double bond, wherein if both are single bonds then the cations are pyrazolidinium cations represented by formula (IIIc) 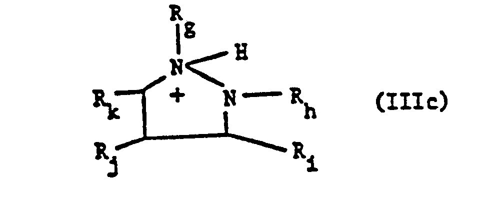 wherein R_{g} to Rₖ are as defined above; and when one of the symbols represents a double bond then the cations are pyrazolinium cations represented by formula (III_{d}) 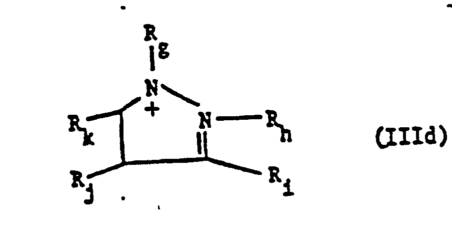 wherein R_{g} to Rₖ are as defined above; and when both 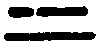 symbols represent double bonds, then the cations are pyrazolium cations represented by formula (IIIe) 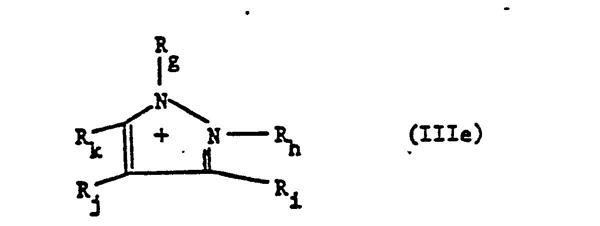 wherein R_{g} to Rₖ are as defined above; X⁺ may also be a structure represented by formulae IIIf, IIIg or IIIh; wherein formula IIIf is 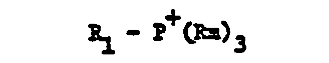 and R₁ is Cₗ-C₆ alkyl, 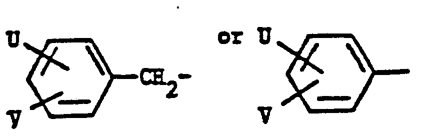 U and V are as hereinabove defined; Rₘ is C₁-C₆ alkyl or 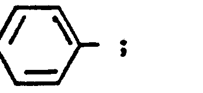 formula IIg is S RₐR_{b}R_{c}; and formula IIh is I RₐR_{b} and Rₐ, R_{b} and ^{R}_{c} are as hereinabove defined; with the additional provisos that when said compounds are of formulae I and Ia and R₁, R₅ and R₇ all are hydrogen and n is 0 then:
a if only R₂ and R₃ are substituted, they cannot be both CH₃ or Cl or combinations thereof;
b if only R₂ and R₆ are substituted they cannot be both CH₃, C₂H₅, C₃H₇, Br or Cl or combinations thereof;
c if only R₂, R₄ and R₆ are substituted, then R₂ cannot be halogen, C₁-C₄ alkyl, SO₂CH₃, C₁-C₃ haloalkyl or Cₗ₋C₃ haloalkoxy; R₄ cannot be halogen or methyl; R₆ cannot be halogen, CF₃, or C₁-C₄ alkyl;
d if _{R3} is NO₂ then R₂, _{R4} and R₆ cannot be all Br; and with the additional provisos that When said compounds are the formula II salts, then:
e when R₂ is halogen, C₁-C₄ alkyl, SO₂CH₃, C₁-C₃ haloalkyl or C₁-C₃ haloalkoxy; R₄ is halogen or methyl; and R₆ is halogen, CF₃ or C₁-C₄ alkyl; then X⁺ cannot be alkali metals, ammonium, H₃NRₐ, H₂NRₐR_{c}, HNRₐRₐR_{c} and NRₐRₐR_{c}R_{d}, wherein the R groups: Rₐ to R_{d} each are selected from C₁-C₁₂ alkyl, C₂-C₄ hydroxyalkyl, aryl substituted aryl wherein the substituents are selected from halogen and C₁-C₆ alkyl, or alkyl C₁-C₃, and substituted benzyl wherein the substituents are selected from halogen and C₁-C₉ alkyl , nor can X⁺ be a heterocyclic ring which may be formed when two of the Rₐ to R_{d} groups are taken together with the nitrogen to Which they are attached to;
_{f} when R₂ and R₆ are both Br, then X⁺ cannot be Na K⁺, i⁺, Ba²⁺, Sr²⁺ or NH₄⁺;
g when R₂ and R₃ are both CH₃, then X⁺ cannot be Li⁺.

2. The compound according to Claim 1, 1,2-dimethyl-3,5-diphenylpyrazolium salt with 2,4,6-tribromo-N-aci-nitroaniline.

3. A method for regulating the growth of plants comprising applying to the foliage of said plants or to the soil in which they are planted, a growth regulating amount of a compound of formulae 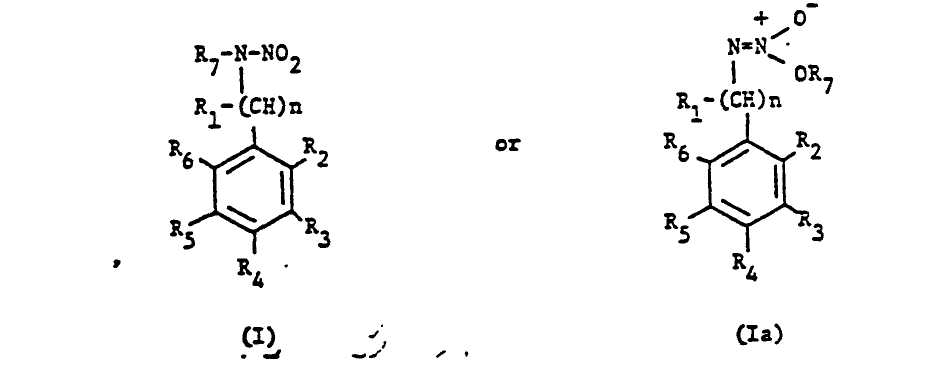 or mixtures thereof, wherein R₁, R₂, R₃, R₄,-R5, R6, R7 and n are as defined above in Claim 1.

4. A method according to Claim 3 wherein said compound is applied to the foliage of said plants at the rate of from 0.06 to 2.0 kg/ha.

5. A method according to Claim 3, for inhibiting lodging of cotton, sunflowers, leguminous crops, graminaceous crops and herbaceous ornamental plants, comprising, applying to the foliage of said plants a lodging inhibiting amount of a compound of formulae

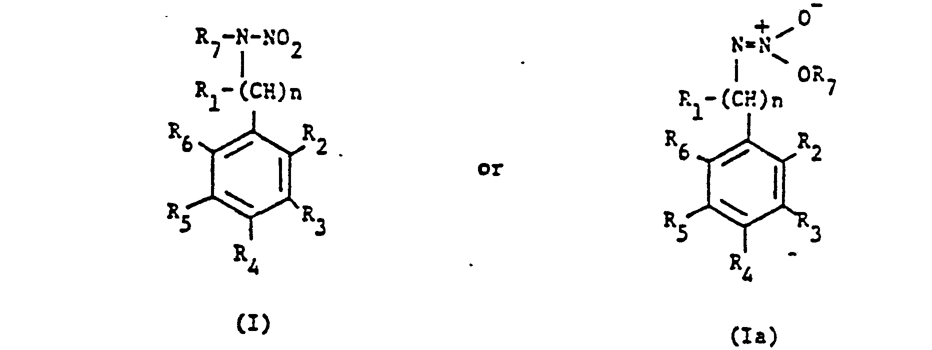

6. A method according to Claim 3, for increasing axillary branching and canopy of plants comprising applying to the foliage of said plants an axillary branch and canopy enhancing amount of a compound having the formulae 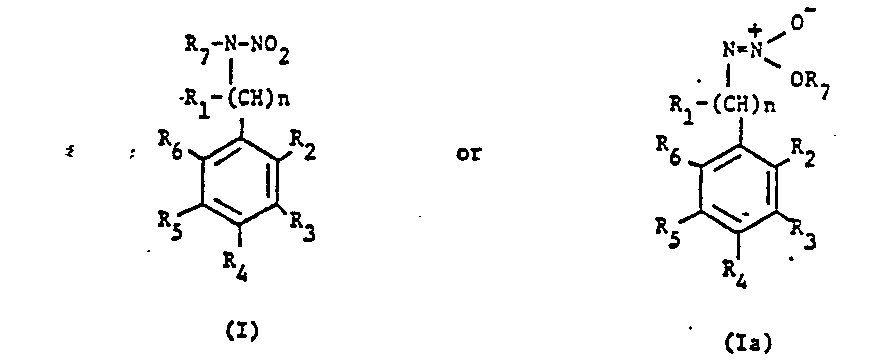 or mixtures thereof, wherein R₁, R₂, R₃, R₄, R₅, R6, R₇ and n are is defined in Claim 1.

7. A method according to Claim 3 for increasing flowering of cotton, sunflowers, leguminous crops, graminaceous crops and herbaceous ornamental plants, comprising applying to the foliage of said plants a compound of formulae 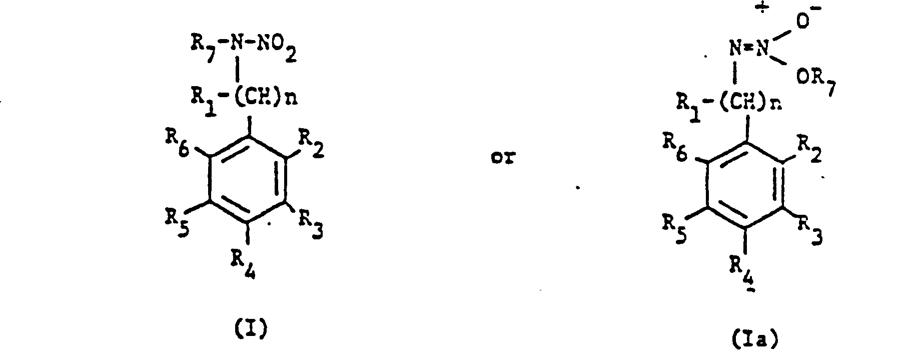 or mixtures thereof, wherein R₁, R₂, R₃, R₄, R₅, R₆, R₇ and n are as defined in Claim 1.

8. A method according to Claim 3 for enhancing the crop yield of cotton, sunflowers, graminacecus crops and leguminous crops comprising, applying to the foliage of crops a yield enhancing amount of a compound of formulae 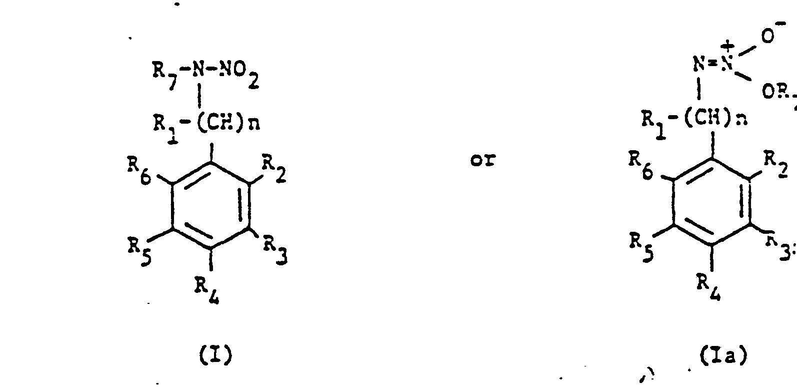 or mixtures thereof, wherein R₁, R₂, R₃, R₄, P₅, R6, R7 and n are as defined in Claim 1.

9. A method according to Claim 3 for reducing the relative stem growth of broadleaf agronomic crops and for increasing the stem stiffness thereof, comprising, applying preemergence to soil in which seeds of said crops are sown, from 0.125 kg/ha to 2.0 kg/ha of a compound of formulae 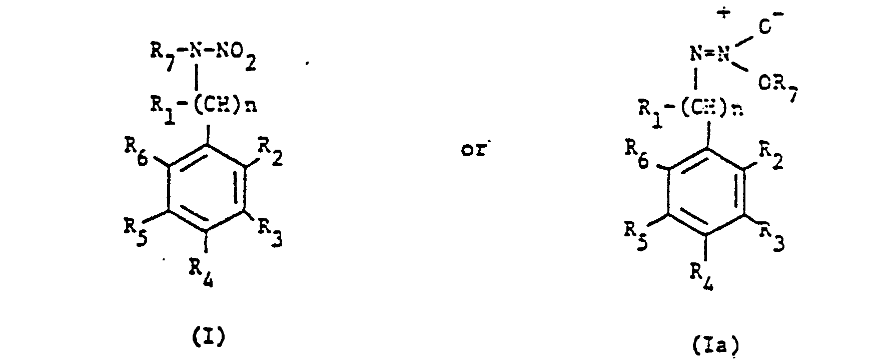 or mixtures thereof, R₁, R₂, R₃, R₄, R₅, R6, R₇ and n are is defined in Claim 1.

10. A method for the preparation of salts of 2,4,6-trisubstituted-N-nitroaniline compounds represented by formula 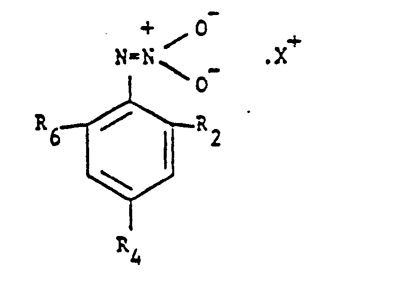 R₂ is Br, Cl, F, I, C₁-C₄ alkyl, OCHF₂ , CF₃, SR₈ , SO₂R₈ or COR₉; R₄ is Br, Cl, F, I, CH₃, OCF₃, NHCOCH₃, CN, SR₈, SO₂R₈, COR₉, phenoxy or substituted phenoxy and the substituents are CH₃, CF₃, OCH₃, Br, Cl, F, I or C₁-C₄ alkyl; R₆ is Br, Cl, F, I or C₁-C₄ alkyl; R₈ is Cₗ₋C₆ alkyl, phenyl or benzyl; R₉ is hydrogen, hydroxy C₁-C₃ alkyl, C₁-C₃ alkoxy or phenyl; and cation X⁺ is inorganic or organic;
when X is inorganic, it is alkali metals, alkaline earth metals, Co, Zn, or Ag;
when the cation X is organic, X⁺ is represented by formula IIIa 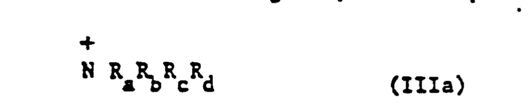 wherein Rₐ is hydrogen, C₁-C₃₀ alkyl straight chain or branched [optionally mono - or disubstituted with HO-, CH₃0₂C-, CH₃S-, C₃-C₁₈alkenylaminoalkylene (C₁-C₃), H₂N^{,} (^{CH}₃)₂^{N-,} 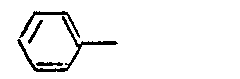 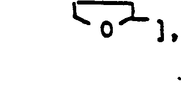 C₃-C₆ alkenyl, C₃-C₆ alkynyl, C₃-C₆ cycloalkyl, 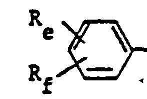 wherein Rₑ and R_{f} each may be H, C^{H}₃ or n-C₁₆H₃₃SO₂-; R_{b} is hydrogen, C₁-C₃₀ alkyl straight chain or branched [optionally mono- or disubstituted with HO- or 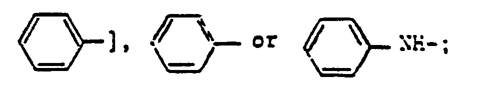 R is hydrogen, C₁-C₃₀ alkyl straight chain or branched [optionally mopo- or disubstituted with HO- or 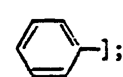 R_{d} is hydrogen, C₁-C₃₀ alkvl straight chain or branched [optionally substituted with HO-, Cl- or 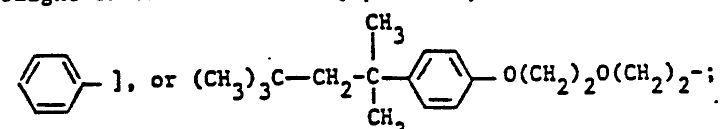 and when R ₐ and R_{b} are taken together with the nitrogen they are attached to they represent a moiety of 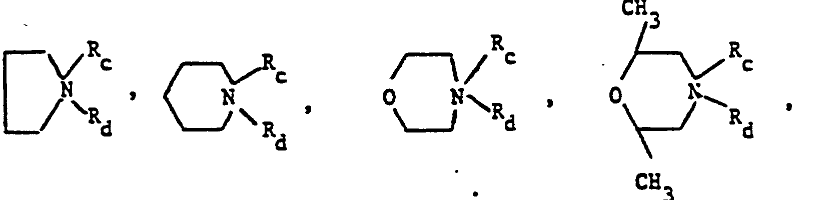 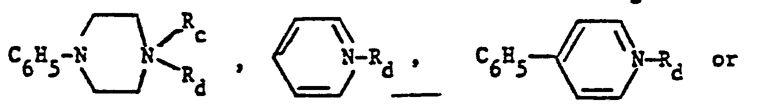 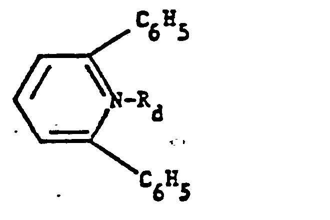 wherein R_{c} and R_{d} are defined as above ;
organic cation X⁺ may also be imidazolium, pyridinium quinolinium, dithiolium, tetrazolium or be represented by formula IIIb 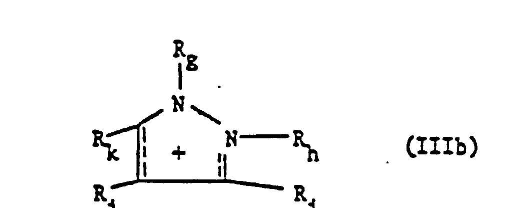 wherein R_{g} and Rₕ each are C₁-C₃ alkyl; R₀ⱼ is hydrogen, halogen, C₁-C₃ alkyl or C₁-C₃ alkoxy; Rⱼ and Rₖ each are hydrogen C₁-C₆ alkyl straight chain or branched, C₃-C₆ cycloalkyl, 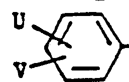 or 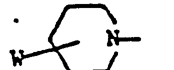 ; wherein U and V each are hydrogen, halogen, C₁-C₃ alkyl, C₁-C₃ alkoxy, CN or CF₃; W is hydrogen or C₁-C₃ alkyl; and each 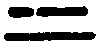 symbol represents a single or double bond, wherein if both are single bonds then the cations are pyrazolidinium cations represented by formula (IIIc) 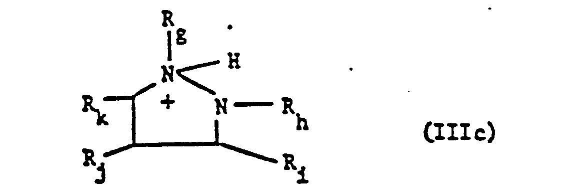 wherein R to Rₖ are as defined above; and when one of the . symbols represents a double bond then the cations are pyrazolinium cations represented by formula (IIId) 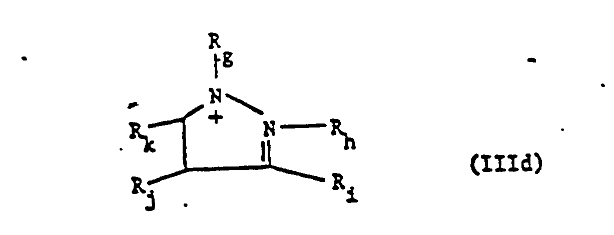 wherein R_{g} to Rₖ are as defined above; and when both 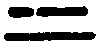 symbols represent double bonds, then the cations are pyrazolium cations represented by formula (IIIe) I 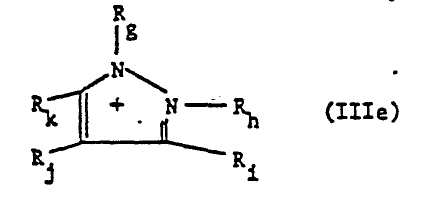 wherein R_{g} to Rₖ are as defined above; X may also be a structure represented by formulae IIIf, IIIg or IIIh: wherein formula IIIf is R₁-R(Rₘ)₃ and R₁ is C₁-C₆ alkyl, 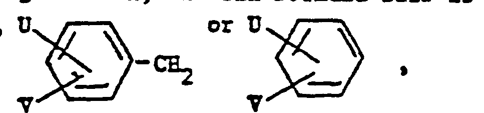 U and V are as hereinabove defined; Rₘ, is C₁-C₆ alkyl or 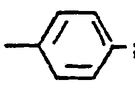 formula IIIgis S RₐR_{b}R_{c}; and formula IIIh is I RₐR_{b} and R , R_{b} and R are as hereinabove defined; with the proviso that when R₂ is halogen C₁-C₄ alkyl, SO₂CH₃, C₁-C₃ haloalkyl or C₁-C₃ haloalkoxy; R₄ is halogen or methyl; R₆ is halogen, CF₃ or C₁-C₄ alkyl; then X⁺ cannot be alkali metals, ammonium, H₃NRₐ, H₂NRₐR_{b}, HNRₐR_{b}R_{c} and NR₈R_{b}R_{c}R_{d}, wherein the R groups: Rₐ to R_{d} each are selected from C₁-C₁₂ alkyl, C₂-C₄ hydroxyalkyl, aryl, substituted aryl wherein the substituents are selected from halogen and C₁-C₆ alkyl, aralkyl C₁-C₃, and substituted benzyl wherein the substituents are selected from halogen and C₁-C₆ alkyl, nor can X⁺ be a heterocyclic ring which may be formed when two of the Rₐ to R_{d} groups are taken together with the nitrogen to which they are attached to;
comprising: treating an alkaline solution of the anion 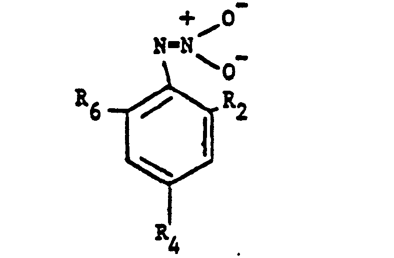 wherein R₂, R₄ and R₆ are as hereinabove defined, with an equimolar or equivalent amount of a salt of X⁺ wherein X is as hereinabove defined, in the presence of an inert solvent of water, lower alcohols or ether, for a period of time sufficient to essentially complete the reaction.

11. A method for the preparation of substituted N-nitroaniline compounds represented by formula 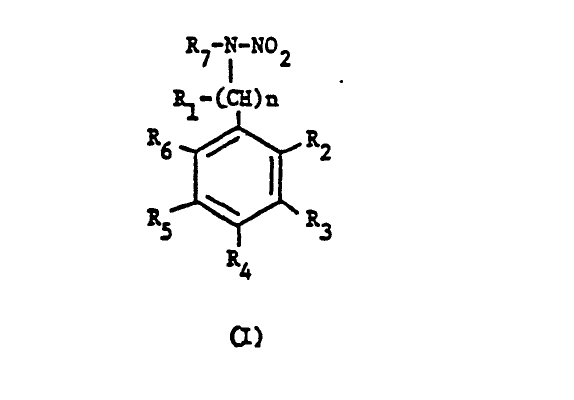 wherein n is an integer of 0 or 1; R₁ is hydrogen, C₁-C₃ alkyl, C₁-C₃ haloalkyl, CN, halogen, NO₂, SO₂R₈, COR9, phenyl or substituted phenyl and the substituents are CH₃, CF₃, CH₃0, halogen, NO₂ or OCH₂ CO₂C₂R₅; R₂ and R₆ are hydrogen, halogen, C₁-C₄ alkyl, C₁-C₄ haloalkyl, C₁-C₄ haloalkoxy, SO₂R₈, SR₈ or COR₁₀; R₃ is hydrogen, C₁-C₄ alkyl, C₁-C₄ alkoxy, NH₂, NH-COCH₃, halogen, NO₂, SO₂P₈, CN or CO₂CH₃; R₄ is hydrogen, halogen, Cₗ-C₂ alkyl, SO₂R₈, SR₈, OCF₃, COR₉, CN, NH-COCH₃, SO₂NR₁₀R₁₁, 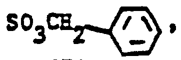 phenoxy or substituted phenoxy and the substituents are CH₃, CF₃, CH₃0, halogen, N0₂ or OCH₂CO₂C₂H₅; R₅ is hydrogen, C₁-C₃ alkyl or halogen; and with the proviso that when R₃, R₅ and R₇ are all hydrogen, and n is an integer of 0, then R₂ cannot be halogen, C₁-C₄ alkyl, SO₂CH₃, C₁-C₃ haloalkyl or C₁-C₃ haloalkoxy; R₄ cannot be halogen or methyl; R₆ cannot be halogen, CF₃ or C₁-C₄alkyl; R₇ is hydrogen, C₁-C₁₂ alkyl optionally substituted with halogen or CN, C₃-C₄ alkenyl, C₃-C₄ alkynyl, the methyl esters of lower alkanoic acids or the moiety 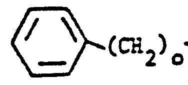 - wherein o is an integer of 0, 1,2 or 3, and the moiety may optionally be substituted with halogen, C₁-C₂ alkyl, C₁-C₂ alkoxy, (C₁-C₃ alkyl)₃ SiCH₂ - or N0₂; R₈ is C₁-C₆ alkyl, phenyl or benzyl; R₉ is hydrogen, hydroxyl, C₁-C₃ alkyl, C₁-C₃ alkoxy or phenyl; R₁₀ and R₁₁ each are hydrogen, C₁-C₄ alkyl or phenyl; with the proviso that not less than two and not more than four of R₂ to R₆ are to be substituted with groups other than hydrogen in any one of the compounds represented by formula (I); comprising: reacting a compound of formula 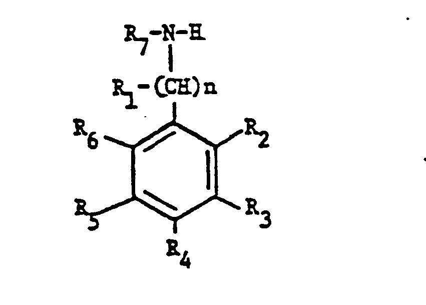 wherein R₁ to R₇ are as hereinabove defined and n=o with an equimolar or slight excess amount of nitric acid in the presence of glacial acetic acid and acetic anhydride in a temperature range of from 5°C to 30°C for a period of time sufficient to essentially complete the reaction.

12. A method for the preparation of substituted N-nitroaniline compounds represented by formula 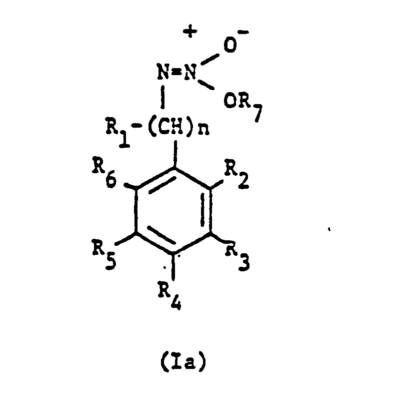 wherein n is an integer of 0 or 1; R₁ is hydrogen, C₁-C₃ alkyl, C₁-C₃ haloalkyl, CN, halogen, N0₂, SO₂R₈, COR₉, phenyl or substituted phenyl and the substituents are CH₃, CF₃, CH₃0, halogen, NO₂ or OCH, CO₂C₂H₅; R₂ and R₆ are hydrogen, halogen, C₁₋C₄alkyl, C₁-C₄ haloalkyl, C₁-C₄ haloalkoxy, SO₂R₈, SR₈ or COR₁₀; R₃ is hydrogen, C₁-C₄ alkyl, C₁-C₄ alkoxy, NH2, NH-COCH₃, halogen, NO₂, SO₂R₈, CN or CO₂CH₃; R₄ is hydrogen, halogen, Cₗ-C₂ alkyl, SO₂R₈, SR₈, COR₉, CN, NH-COCH₃, SO₂NR₁₀R₁₁, 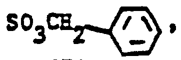 phenoxy or substituted phenoxy and the substituents are CH₃, CF₃, CH30, halogen, NO2 or OCH₂CO₂C₂H₅; R₅ is hydrogen, C₁-C₃ alkyl or halogen; R₇ is hydrogen, C₁-C₁₂ alkyl optionally substituted with halogen or CN, C₃-C₄ alkenyl, C₃-C₄ alkynyl, the methyl esters of lower alkanoic acids or the moiety 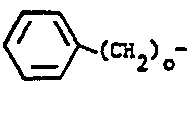 wherein o is an integer of 0, 1,2 or 3, and the moiety may optionally be substituted with halogen C₁-C₂ alkyl, C₁-C₂ alkoxy, (C₁-C₃ alkyl)₃ SiCH₂ - or N0₂; R₈ is C₁-C₆ alkyl, phenyl or benzyl; R₉ is hydrogen, hydroxyl, C₁-C₃ alkyl, C₁-C₃ alkoxy or phenyl; R₁₀ and R₁₁ each are hydrogen, C₁-C₄ alkyl or phenyl; with the proviso that not less than two and not more than four of R₂ to R₆ are to be substituted with groups other than hydrogen in any one of the compounds represented by formula (Ia) comprising: reacting a compound of formula 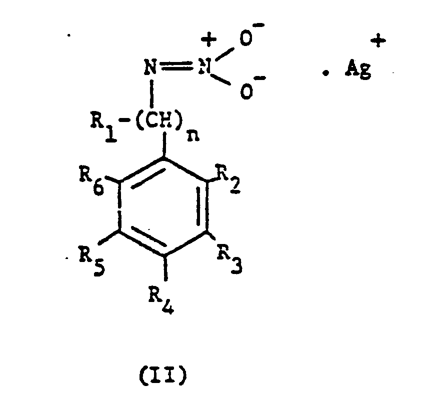 with an equimolar or excess amount of a compound of formula

R₇ - Q wherein R₇ is a hereinabove defined except that it cannot be hydrogen; Q is Br, or chlorine; in the presence of an inert solvent and under a dry, inert atmosphere at 10°C to 30°C for a period of time sufficient to complete the reaction.

13. A composition comprising: 5X to 25X by weight of a substituted N-nitroaniline represented by formulas (I) or (Ia) below: 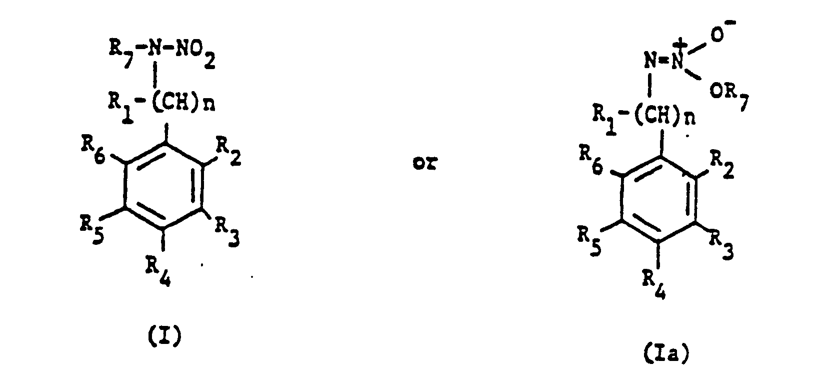 wherein n is an integer of 0 or 1; R₁ is hydrogen, Cₗ-C₃ alkyl, C₁-C₃ haloalkyl, CN, halogen, N0₂₁ SO₂R₈, COR₉, phenyl or substituted phenyl and the substituents are CH₃, CF₃, CH₃0, halogen, NO₂ or OCH₂ CO₂C₂H₅; R₂ and R₆ are hydrogen, halogen, C₁₋C₄ alkyl, C₁-C₄ haloalkyl, C₁-C₄ haloalkoxy, SO₂R₈, SR₈ or COR₁₀; R₃ is hydrogen, C₁-C₄ alkyl, Cl-C4 alkoxy, NH₂, NH-COCH₃, halogen, NO₂, SO₂R₈, CN or CO₂CH₃; R₄ is hydrogen, halogen, C₁-C₂alkyl, SO₂R₈, SR₈, OCF₃, COR₉, CN, NH-COCH₃, SO₂NR₁₀R₁₁, 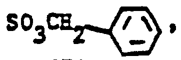 phenoxy or substituted phenoxy and the substituents are CH₃, CF₃, CH₃0, halogen, NO₂ or OCH₂CO₂C₂H₅; R5 is hydrogen, Cl-C3 alkyl or halogen; and with the proviso that when R₃, R₅ and R₇ are all hydrogen, and n is an integer of 0, and the compounds are of formula I, then R₂ cannot be halogen, Cₗ-C₄ alkyl, SO₂CH₃, C₁-C₄ alkyl, SO₂CH₃, C₁-C₃ haloalkyl or C₁-C₃ haloalkoxy; R4 cannot be halogen or methyl; _{R6} cannot be halogen, CF₃ or C₁-C₄ alkyl; R₇ is hydrogen, C₁-C₁₂ alkyl optionally substituted with halogen or CN, C₃-C₄ alkenyl, C₃-C₄ alkynyl, the methyl esters of lover alkanoic acids or the moiety 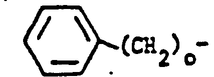 wherein o is an integer of 0, 1,2 or 3, and the moiety may optionally be substituted with halogen, C₁-C₂ alkyl, C₁-C₂ alkoxy, (C₁-C₃ alkyl)3 SiCH₂ - or N0₂; R₈ is C₁-C₆ alkyl, phenyl or benzyl; R₉ is hydrogen, hydroxyl, C₁-C₃ alkyl, C₁-C₃ alkoxy or phenyl; R₁₀ and R₁₁ each are hydrogen, C₁-C₄ alkyl or phenyl; with the proviso that not less than two and not more than four of R₂ to R₆ are to be substituted with groups other than hydrogen in any one of the compounds represented by formulae (I) or (Ia) or mixtures thereof; and when R₇ is hydrogen, the above compounds of formulae (I) or (Ia) may form inorganic or organic salts, the salt being represented by formula (II) below: 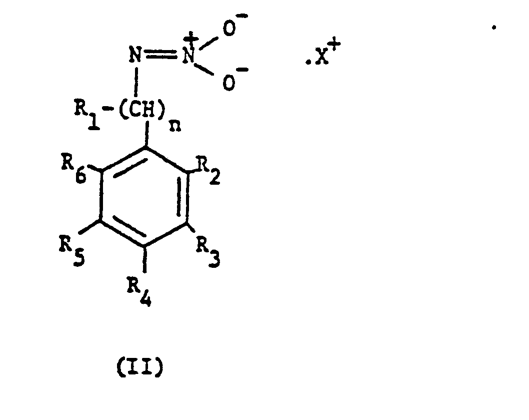 when X is inorganic, it is alkali metals, alkaline earth metals, Co, Cu, Zn or Ag; when the cation X^{÷} is organic, X⁺ is represented by formula IIIa 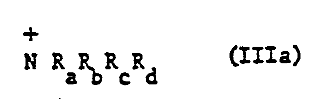 wherein Rₐ is hydrogen, C₁-C₃₀ alkyl straight chain or branched [optionally mono - or disubstituted with HO-, CH₃0₂C-, CH₃S-, C₃-C₁₈ alkenylaminoalkylene (C₁-C₃), H₂N-, (CH₃)₂N-, 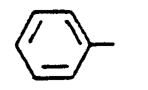 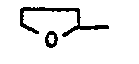 ], C₃-C₆ alkenyl, C₃-C₆ alkynyl, C₃-C₆ cycloalkyl, 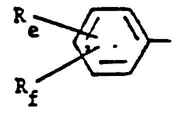 wherein Rₑ and R_{f} each may be H, CH₃ or n-C₁₆H₃₃SO₂-; R_{b} is hydrogen, Cₗ-C₃₀ alkyl straight chain or branched [optionally mono- or disubstituted with HO- or 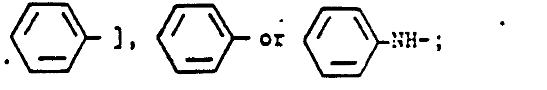 Rₒ is hydrogen, C₁-C₃₀ alkyl straight chain or branched [optionally mono- or disbustituted with HO- or 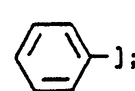 ; R_{d} is hydrogen, C₁-C₃₀ alkyl straight chain or branched [optionally substituted with HO-, Cl- or 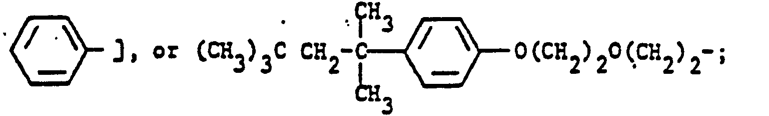 and when Rₐ and R_{b} are taken together with the nitrogen they are attached to they represent a moiety of 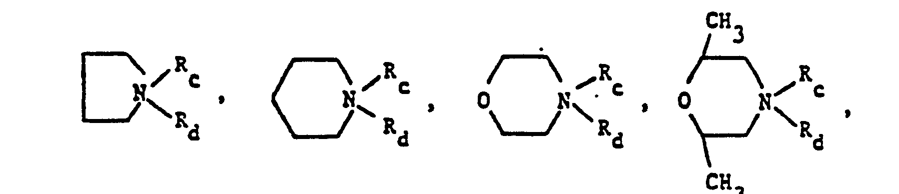 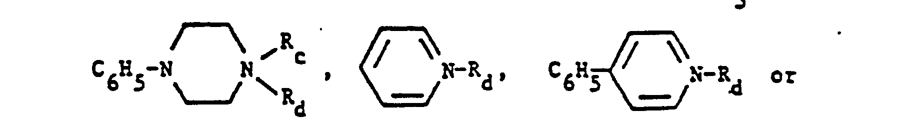 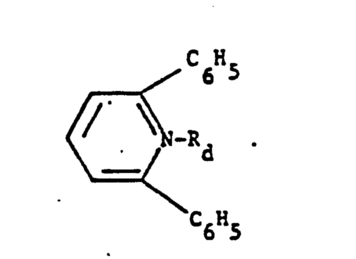 wherein R_{c} and R_{d} are defined as above; organic cation X⁺ may also be imidazolin, pyridinium, quinolinium, dithiolium, tetrazolium or be represented by formula IIIb 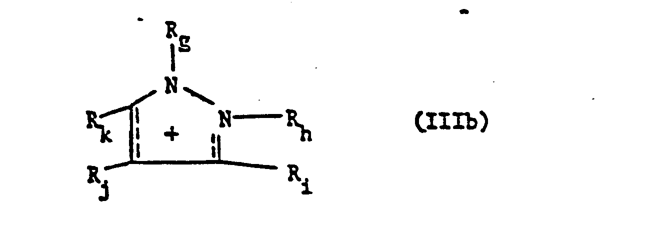 wherein R₈ and Rₕ each are C₁-C₃ alkyl; Rⱼ is hydrogen, halogen, C₁-C₃ alkyl or C₁-C₃ alkoxy; Rᵢ and Rₖ each are hydrogen C₁-C₆ alkyl straight chain or branched, C₃-C₆ cycloalkyl, 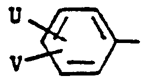 or 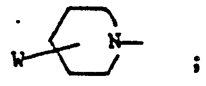 wherein U and V each are hydrogen, halogen, C₁-C₃ alkyl, C₁-C₃ alkoxy, CN or CF₃; W is hydrogen or C₁-C₃ alkyl; and each ―― symbol represents a single or double bond, wherein if both are single bonds then the cations are pyrazolidinium cations represented by formula (IIIc) 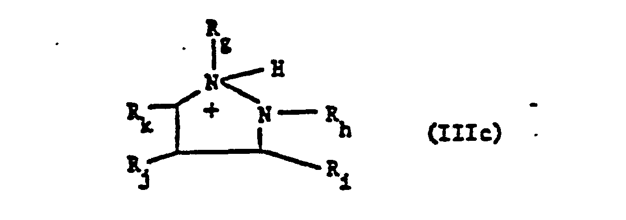 wherein R_{g} to Rₖ are as defined above; and when one of the 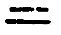 symbols represents a double bond then the cations are pyrazolinium cations represented by formula (IIId) 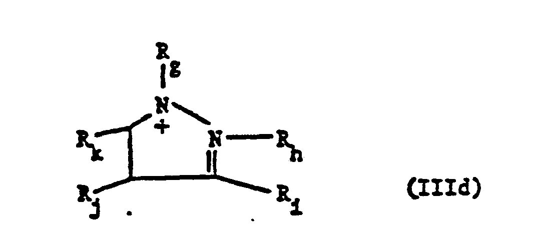 wherein R_{g} to Rₖ are as defined above; and when both 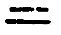 symbols represent double bonds, then the cations are pyrazolium cations represented by formula (IIIe) 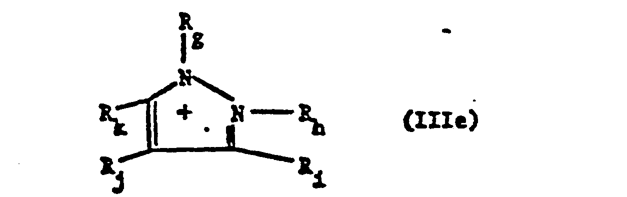 wherein R_{g} and Rₖ are as defined above; X⁺ may also be represented by formulae IIIf and IIIg 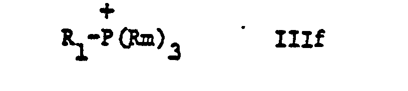 wherein R₁ is C₁-C₆ alkyl, 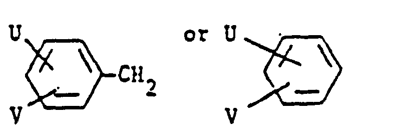 wherein U and V are as hereinabove defined; Rₘ is C₁-C₆ alkyl or 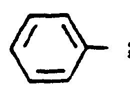 ÷ + ; S RₐR_{b}R_{c} (III^{g}) and I RₐR_{b} (IIIh), wherein Rₐ, R_{b} and R_{c} are as hereinabove defined; and with the proviso that when n is an integer of 0; R₃ and R₄ are both hydrogen; R₂ is halogen, C₁-C₄ alkyl, SO₂CH₃, C₁-C₃ haloalkyl or C₁-C₃ haloalkoxy; R₄ is halogen or methyl; R₆ is halogen, CF₃ or C₁-C₄ alkyl, and the salts are of formula II; then X cannot be alkali metals, ammonium, H₃NRₐ, ⁺ + + a H₂NRₐR_{b}, HNRₐR_{b}R_{c} and NRₐR_{b}R_{c}R_{d}, wherein the R groups: Rₐ to R_{d} each are selected from C₁-C₁₂ alkyl, C₂-C₄ hydroxyalkyl, aryl, substituted aryl wherein the substituents are selected from halogen and C₁-C₆ alkyl, aralkyl C₁-C₃, and substituted benzyl wherein the substituents are selected from halogen and C₁-C₆ alkyl, nor can X⁺ be a heterocyclic ring which may be formed when tw of the Rₐ to R_{d} groups are taken together with the nitrogen to which they are attached to; 0.5% to 5% of a surfactant and the composition is totaled to 100X with an inert carrier.
